# EUROPEAN PATENT APPLICATION

(11) **EP 1 460 069 A1**
(43) Date of publication of application: **22.09.2004**
(21) Application number: 03006255.8
(22) Date of filing: 20.03.2003
(51) Int. Cl.: C07D 401/10, C07D 401/12, C07D 401/14, C07D 405/12, C07D 405/14, C07D 217/26, C07D 211/70, A61K 31/4725, A61P 3/04, A61P 15/00, A61P 25/22, A61P 25/24

(54) **Substituted cyclohexyl and piperidinyl derivatives as melanocortin-4 receptor modulators**

(71) Applicant: MyoContract Ltd., 4410 Liestal (CH)
(72) Inventor: Soeberdt, Michael, 79618 Rheinfelden (DE); Weyermann, Philipp, 4450 Sissach (CH); Sprecher von, Andreas, 4104 Oberwill (CH)
(74) Representative: Grünecker, Kinkeldey, Stockmair & Schwanhäusser Anwaltssozietät

(57) **Abstract**

The present invention relates to novel substituted cyclohexyl and piperidinyl derivatives as melanocortin-4 receptor (MC-4R) modulators. MC-4R agonists of the invention can be used for the treatment of disorders and diseases such as obesity, diabetes and sexual dysfunction, whereas the MC-4R antagonists are useful for the treatment of disorders and diseases such as cancer cachexia, muscle wasting, anorexia, anxiety and depression. All diseases and disorders where the regulation of the MC-4R is involved can be treated with the compounds of the invention.

## Description

### Field of the Invention

The present invention relates to novel substituted cyclohexyl and piperidinyl derivatives as melanocortin-4 receptor modulators. Depending on the structure and the stereochemistry, the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression Generally, all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G- protein-coupled receptors, termed melanocortin receptors (MC-Rs). Activation of any of these MC-Rs results in stimulation of CAMP formation.

To date, five distinct types of receptor subtypes for MC (MC-1R to MC-5R) have been identified and these are expressed in different tissues.

MC-1R was first found in melanocytes. Naturally occurring inactive variants of MC-1R in animals were shown to lead to alterations in pigmentation, and a subsequent lighter coat color, by controlling the conversion of phaeomelanin to eumelanin through the control of tyrosinase.

From these and other studies, it is evident that MC-1R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat and placenta, and a low level of expression is also observed in the brain. However its expression is greatest in exocrine glands. Genetic knock-out of this receptor in mice results in altered regulation of exocnne gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators, to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-kB. NF-kB is a pivotal component of the pro-inflammatory cascade and its activation is a central event in initiating many inflammatory diseases. Additionally, anti-inflammatory actions of α-MSH may be, in part, mediated by agonism of MC-3R and/or MC-5R.

A specific single MC-R, that may be targeted for the control of obesity, has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-R's in obesity includes: 1) the agouti (A^{vy}) mouse, which ectopically expresses an antagonist of the MC-1R, MC-3R and MC-4R, is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R and -5R agonist) injected intracerebroventricularly (ICV) in rodents, reduces food intake in several animal feeding models (NPY, ob/ob, agouti, fasted), while ICV injected SHU-9119 (MC-3R and -4R antagonist; MC-1R and -5R agonist) reverses this effect and can induce hyperphagia; 4) chronic intrapentoneal treatment of Zucker fatty rats with an α-NDP-MSH derivative (HP-228) has been reported to activate MC1R, -3R, -4R and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study", J. Urol., 160: 389-393, 1998). Activation of melanocortin receptors of the brain appears to cause normal stimulation of sexual arousal. Evidence for the involvement of MC-R in male and/or female sexual dysfunction is detailed in WO/0074679.

Diabetes is a disease in which a mammal's ability to regulate glucose levels in the blood is impaired because the mammal has a reduced ability to convert glucose to glycogen for storage in muscle and liver cells. In "Type I diabetes", this reduced ability to store glucose is caused by reduced insulin production. "Type II diabetes", or "Non-Insulin Dependent Diabetes Mellitus" (NIDDM), is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue, and of glucose production and secretion in liver. When these cells become desensitized to insulin, the body tries to compensate by producing abnormally high levels of insulin, and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL, which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF), (Owen MJ and Nemeroff CB (1991), (Physiology and pharmacology of corticotrophin releasing factor. *Pharmacol Rev* 43:425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003)304(2), 818-26).

Chronic diseases, such as malignant tumors or infections, are frequently associated with cachexia, resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus, by α-MSH, reduces appetite and increases energy expenditure. Experimental evidence, in tumor bearing mice, suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. *et al.* Role of the central melanocortin system in cachexia. Cancer Res. (2001) 61: 1432-1438).

In view of the unresolved deficiencies in the treatment of vanous diseases and disorders as discussed above, it is an object of the present invention to provide novel substituted cyclohexyl and piperidinyl derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

Also provided are processes for producing the MC-4R modulators and pharmaceutical compositions containing the same.

### Summary of the Invention

The present invention relates to novel-substituted piperidyl and cyclohexyl derivatives of structural formula (I).

The present invention relates to compounds of formula (I) 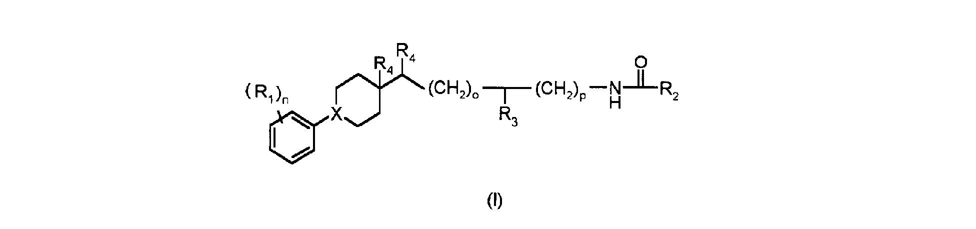 wherein the variables R₁, R₂, R₃, R₄, X, n, o and p have the meaning as defined below.

The present invention also relates to novel-substituted piperidyl and cyclohexyl derivatives of structural formula (II) 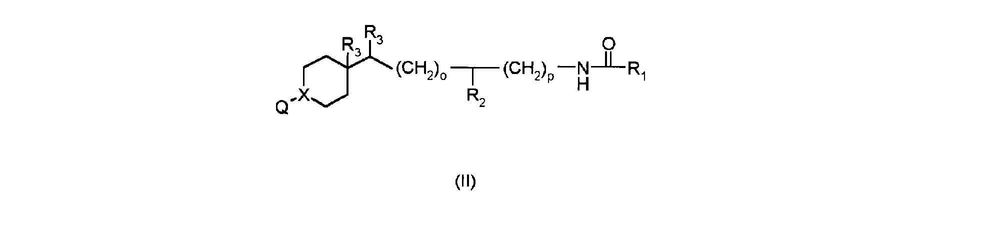 wherein the variables R₁, R₂, R₃, X, Q, o and p have the meaning also defined below.

The piperidyl and cyclohexyl derivatives of structural formulas (I) and (II) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to the intermediate compounds of structural formulas (III) and (IV) 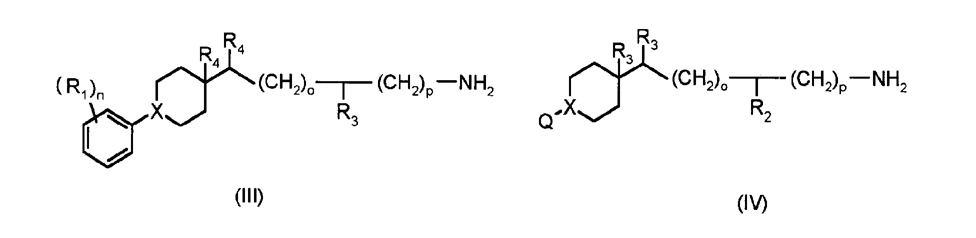 wherein the variables R₁, R₂, R₃, R₄, Q, X, n, o and p have the meaning as defined below.

Moreover, the present invention also relates to pharmaceutical compositions comprising the compounds of the present invention and a pharmaceutically acceptable carrier.

### Detailed Description of the Invention

The present invention relates to novel-substituted piperidinyl and cyclohexyl derivatives useful as melanocortin receptor modulators, in particular, selective MC-4R agonists and MC-4R antagonists.

Compounds of the present invention are represented by structural formula (I) 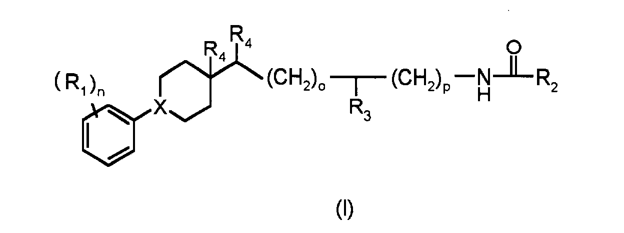 or a pharmaceutically acceptable salt or a solvate thereof, wherein R₁ is independently:
hydrogen,
hydroxy,
cyano,
nitro,
halo,
alkyl,
alkoxy,
haloalkyl,
(D)-C(O)R₁₅,
(D)-C(O)OR₁₅,
(D)-C(O)SR₁₅,
(D)-C(O)-heteroaryl,
(D)-C(O)-heterocyclyl,
(D)-C(O)N(R₁₅)₂,
(D)-N(R₁₅)₂,
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅,
(D)-NR₁₅SO₂N(R₁₅)₂,
(D)-NR₁₅(D)-heterocyclyl,
(D)-NR₁₅(D)-heteroaryl,
(D)-OR₁₅,
OSO₂R₁₅,
(D)-[O]ᵥ(C₃ - C₇ cycloalkyl),
(D)-[O]ᵥ(D)aryl,
(D)-[O]ᵥ(D)-heteroaryl or
(D)-[O]ᵥ(D)-heterocyclyl,
   wherein heterocyclyl excludes a heterocyclyl containing a single nitrogen when v=1,
(D)-SR₁₅,
(D)-SOR₁₅,
(D)-SO₂R₁₅ or
(D)-SO₂N(R₁₅)₂,
wherein alkyl, alkoxy, cycloalkyl, aryl, heterocyclyl and heteroaryl are unsubstituted or substituted
- R₂: is: 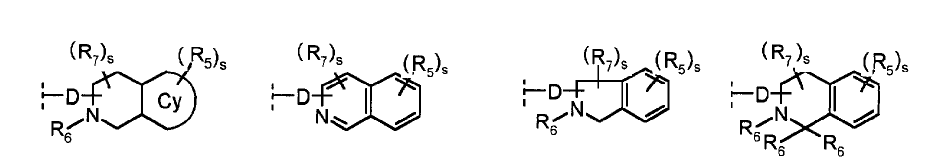 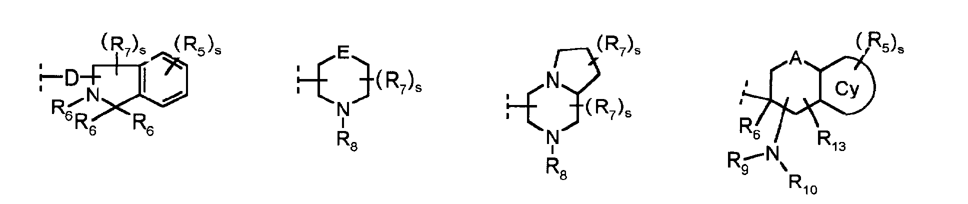 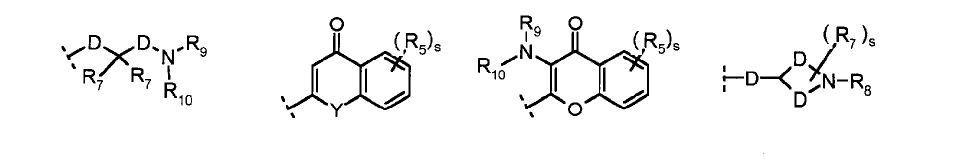
- R₃: is independently:
(D)-aryl or
(D)-heteroaryl,
wherein aryl and heteroaryl are unsubstituted or substituted;
- R₄: is H or a bond;
- each R₅: is independently.
hydrogen,
halo,
alkyl,
haloalkyl,
(D)-cycloalkyl,
(D)-aryl (wherein aryl is phenyl or naphthyl),
(D)-heteroaryl,
(D)-heterocyclyl (wherein heterocyclyl excludes a heterocyclyl containing a single nitrogen), and
wherein aryl, heteroaryl, heterocyclyl, alkyl or cycloalkyl is unsubstituted or substituted
- each R₆: is independently.
hydrogen,
alkyl,
C(O)-alkyl,
(D)-aryl or
cycloalkyl;
- each R₇: is independently:
hydrogen,
alkyl,
(D)-aryl,
(D)-heteroaryl,
(D)-N(R₉)₂,
(D)-N R₉C(O)alkyl,
(D)-NR₉SO₂ alkyl,
(D)-SO₂N(R₉)₂,
(D)-(O)ᵣ alkyl,
(D)-(O)ᵣ(D)-NR₉COR₉,
(D)-(O)ᵣ(D)-NR₉SO₂R₉,
(D)-(O)ᵣ-heterocyclyl or
(D)-(O)ᵣ(alkyl)-heterocyclyl,
- each R₈: is independently:
hydrogen,
alkyl,
(D)-aryl,
C(O)alkyl,
C(O)-aryl,
SO₂-alkyl or
SO₂-aryl;
- R₉ and R₁₀: are each independently:
hydrogen,
alkyl or
cycloalkyl, or
R₉ and R₁₀ together with the nitrogen to which they are attached form a 5- to 8-membered ring optionally containing an additional heteroatom selected from O, S and NR₆,
wherein alkyl and cycloalkyl are unsubstituted or substituted;
- R₁₃: is:
hydrogen or
alkyl;
- each R₁₅: is independently:
hydrogen,
alkyl,
haloalkyl,
(D)-cycloalkyl,
(D)-aryl (wherein aryl is phenyl or naphthyl),
(D)-heteroaryl or
(D)-heterocyclyl,
   wherein heterocyclyl excludes a heterocyclyl containing a single nitrogen, and
   wherein aryl, heteroaryl, heterocyclyl, alkyl and cycloalkyl is unsubstituted or substituted;
- Cy: is:
aryl,
5- or 6-membered heteroaryl,
5- or 6-membered heterocyclyl or
5- or 7-membered carbocyclyl;
A is a bond, O, S(O)ᵤ, NR₈ or CH₂;
D is a bond or alkylene;
E is O, S or NR₈;
X is N or CH;
Y is O or NR₉;
n is 1 - 4;
o is 0 - 2;
p is 0 - 2;
r is 0 or 1;
s is 0 - 5;
u is 0 - 2;
v is 0 to 1.

In preferred embodiments, the variants have the following meanings:

R₁ is as defined above wherein heterocyclyl includes azetidin-2-one-1-yl, pyrrolidin-2-one-1-yl, piperid-2-one-1-yl and azepan-2-one-1-yl. Moreover, alkyl, alkoxy, cycloalkyl, aryl, heterocyclyl and heteroaryl are preferably substituted or unsubstituted alkyl with one to five, preferably 1 to 3, more preferably 1 or 2, substituents independently selected from R₁₄.

Preferably, R₁ is hydrogen, hydroxy, cyano, nitro, halo, alkyl, alkoxy, haloalkyl, (D)-N(R₁₅)₂, (D)-NR₁₅COR₁₅, (D)-NR₁₅CON(R₁₅)₂, (D)-NR₁₅C(O)OR₁₅, (D)-NR₁₅C(R₁₅)=N(R₁₅), (D)-NR₁₅C(=NR₁₅)N(R₁₅)₂, (D)-NR₁₅SO₂R₁₅, (D)-NR₁₅SO₂N(R₁₅)₂ or (D)-heterocyclyl, wherein alkyl or alkoxy are substituted or unsubstituted with one to five, preferably one to three, substituents selected from R₁₄. More preferably, R₁ is cyano, nitro, halo, alkyl, (D)-heterocyclyl, (D)-N(R₁₅)₂, (D)-NR₁₅COR₁₅, (D)-NR₁₅CON(R₁₅)₂, (D)-NR₁₅C(O)OR₁₅ or (D)-NR₁₅SO₂R₁₅. Most preferably, R₁ is halo, (D)-heterocyclyl, (D)-NR₁₅SO₂R₁₅, (D)-NR₁₅CON(R₁₅)₂ or (D)-NR₁₅COR₁₅, in particular (D)-heterocyclyl. Halo is preferably F, Cl or Br. R₁ can be on any position of the ring, preferably in the 1-position.

R₂ is each of the rings as defined above, preferably 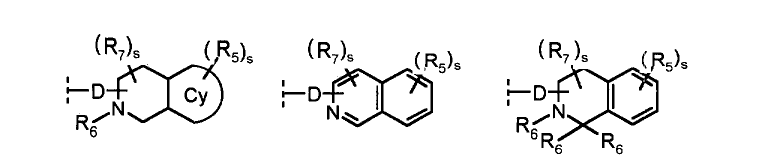 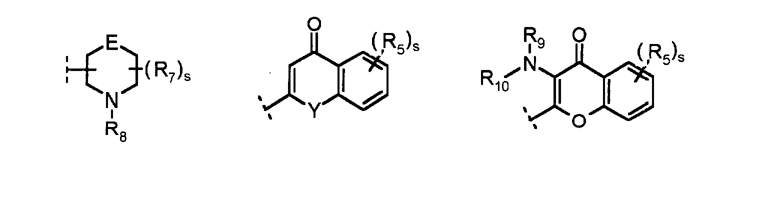 more preferably 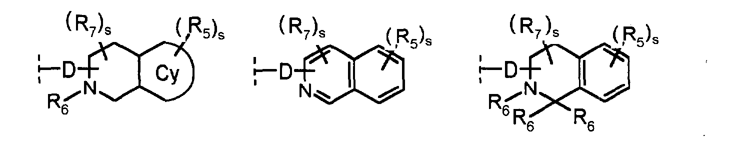 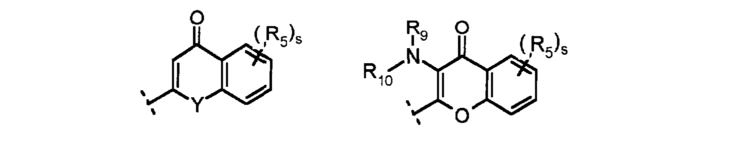 In one embodiment, R₂ is

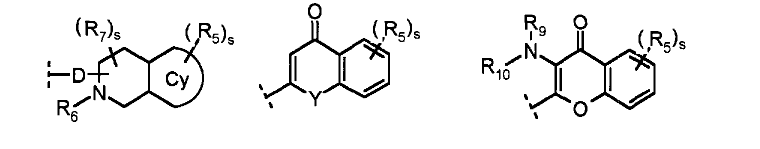

R₃ is as defined above and is preferably (CH₂)-aryl, more preferably (CH₂)-phenyl or (CH₂)-naphthyl. If aryl or heteroaryl are substituted, it is preferably substituted with one to three, more preferably 1 or 2, most preferably 1, substituents. The substituents are preferably independently selected from the group consisting of cyano, nitro, perfluoroalkoxy, halo, alkyl, (D)-cycloalkyl, alkoxy and haloalkyl, more preferably selected from perfluoroalkoxy, halo, alkyl, alkoxy or haloalkyl, most preferably selected from halo, alkyl, alkoxy and haloalkyl, in particular halo.

Most preferably, R₃ is (CH₂)-phenyl or (CH₂)-naphthyl which both, preferably (CH₂)-phenyl, may be substituted with one to three, in particular one, halo, e.g. Cl. The substitution can be in any position, preferably in the 4-position.

R₄ is as described above. In one embodiment R₄ is a bond.

R₅ is as defined above wherein aryl, heteroaryl, heterocyclyl, alkyl or cycloalkyl is preferably unsubstituted or substituted with one to three substituents selected from the group consisting of oxo, alkyl, N(R₆)₂, OR₆, SR₆ and CO₂R₆. Preferably, R₅ is hydrogen, halo, alkyl, haloalkyl, alkoxy or (D)-cycloalkyl, more preferably hydrogen, halo or alkyl, e.g. methyl, ethyl, n-propyl, iso-propyl, most preferably hydrogen.

R₆ is as defined above, preferably hydrogen or alkyl, e.g. methyl, ethyl, n-propyl, iso-propyl, more preferably hydrogen.

R₇ is as defined above, preferably hydrogen, (D)-aryl, (D)-heteroaryl, (D)-N(R₉)₂, (D)-NR₉C(O)alkyl, (D)-NR₉SO₂alkyl or alkyl, e.g. methyl, ethyl, n-propyl, iso-propyl, more preferably hydrogen.

R₈ is as defined above, preferably hydrogen or alkyl, e.g. methyl, ethyl, n-propyl, iso-propyl, more preferably hydrogen.

R₉ and R₁₀ are each independently as defined above. When R₉ and R₁₀ form a ring, the ring preferably contains an additional heteroatom selected from O, S and NR₆. R₉ and R₁₀ are each independently preferably selected from the group consisting of hydrogen, alkyl or cycloalkyl, or R₉ and R₁₀ together with the nitrogen to which they are attached form a 5- to 7-membered ring optionally containing an additional heteroatom selected from O, S and NR₆. Moreover, alkyl and cycloalkyl are preferably unsubstituted or substituted with one to three, preferably one or two, groups independently selected from R₁₁ and oxo.

More preferably R₉ and R₁₀ are each independently selected from the group consisting of hydrogen or alkyl, or R₉ and R₁₀ together with the nitrogen to which they are attached form a 5-to 6-membered ring optionally containing an additional oxygen atom.

The above mentioned R₁₁ is alkyl, (D)-aryl, (D)-cycloalkyl, (D)-heteroaryl, halo, OR₁₂, NHSO₂R₁₂, N(R₁₂)₂, C=N, CO₂R₉, C(R₁₂)(R₁₂)N(R₁₂)₂, nitro, SO₂N(R₁₂)₂, S(O)ᵤR₁₂, CF₃ or OCF₃; preferably selected from the group consisting of alkyl, OR₁₂,(D)-aryl, (D)-cycloalkyl, (D)-heteroaryl and halo.

R₁₂ is independently hydrogen, alkyl, (D)-aryl or cycloalkyl, preferably hydrogen, (D)-aryl or alkyl, e.g. methyl, ethyl, n-propyl, iso-propyl, more preferably hydrogen or (D)-aryl.

R₁₃ is as defined above, preferably hydrogen or C₁ - C₄ alkyl as defined below, more preferaby hydrogen, methyl or ethyl, most preferably hydrogen or methyl.

The above mentioned R₁₄ is independently hydrogen, halo, oxo, N(R₆)₂, alkyl, (D)-cycloalkyl, haloalkyl, alkoxy, heteroaryl, hydroxy or heterocyclyl, wherein heterocyclyl excludes a heterocyclyl containing a single nitrogen, aryl, (D)-COR₁₅, (D)-C(O)OR₁₅, (D)-OR₁₅, (D)-OCOR₁₅, (D)-OCO₂R₁₅, (D)-SR₁₅, (D)-SOR₁₅ or (D)-SO₂R₁₅, wherein aryl, heteroaryl, heterocyclyl, alkyl or cycloalkyl are preferably substituted or unsubstituted with one to three, preferably one or two, substituents selected from the group consisting of oxo, alkyl, N(R₁₅)₂, OR₁₅, SR₁₅ and CO₂R₁₅. Preferably, R₁₄ is hydrogen, halo, alkyl, (D)-cycloalkyl, alkoxy or phenyl, more preferably R₁₄ is hydrogen, halo, alkyl, alkoxy or phenyl.

R₁₅ is as defined above wherein aryl, heteroaryl, heterocyclyl, alkyl or cycloalkyl are preferably substituted or unsubstituted with one to three, preferably one or two, substituents selected from the group consisting of oxo, alkyl, N(R₆)₂, OR₆, SR₆ and CO₂R₆. Preferably, R₁₅ is hydrogen, halo, alkyl, (D)-cycloalkyl, alkoxy or phenyl, more preferably R₁₅ is hydrogen, halo, alkyl, alkoxy or phenyl.

Cy is as defined above and preferably selected from aryl, 5- or 6-membered heteroaryl, 5- or 6-membered heterocyclyl and 5- to 7-membered carbocyclyl. More preferably, Cy is aryl and heteroaryl. In one embodiment, Cy may be aryl, such as phenyl or naphthyl.

A is as described above, preferably a bond or CH₂.
D is as defined above, preferably a bond or CH₂, more preferably a bond.
E is as described above, preferably NR₈.
X is as defined above. In one embodiment, X is CH.
Y is as defined above, preferably NR₉, more preferably N-alkyl. In one embodiment, Y is N-n-propyl.

Alkyl is as defined below, preferably C₁ - C4-alkyl
n is 1 - 4, preferably 0, 1 or 2, more preferably 1 or 2.
o is 0 - 2, preferably 0 or 1.
p is 0 - 2, preferably 0 or 1.
r is 0 or 1.
s is 0 - 5, preferably 0 - 3, more preferably 0 or 1, most preferably 0.
u is 0 - 2.
v is 0 or 1.

In the above, any of the preferred definitions for each variant can be combined with the preferred definition of the other variants.

Compounds of the present invention are also represented by structural formula (II), 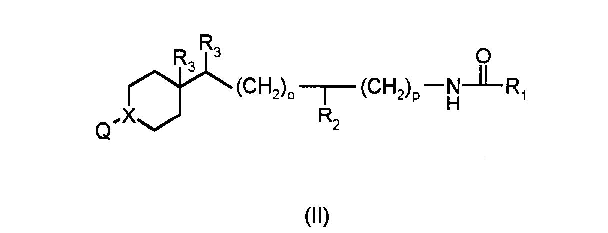 or a pharmaceutically acceptable salt or a solvate thereof, wherein
- R₁: is: 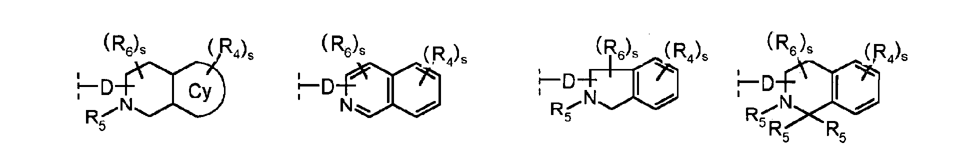 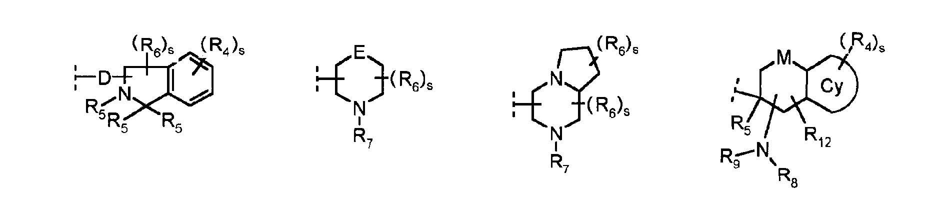 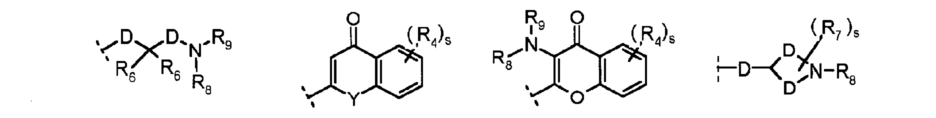
- R₂: is independently:
(D)-aryl or
(D)-heteroaryl,
wherein aryl and heteroaryl are unsubstituted or substituted;
- R₃: is H or a bond;
- each R₄: is independently:
hydrogen,
halo,
alkyl,
haloalkyl,
(D)-cycloalkyl,
(D)-aryl (wherein aryl is phenyl or naphthyl),
(D)-heteroaryl or
(D)-heterocyclyl,
wherein heterocyclyl excludes a heterocyclyl. containing a single nitrogen, and
wherein aryl, heteroaryl, heterocyclyl, alkyl and cycloalkyl is unsubstituted or substituted;
- each R₅: is independently:
hydrogen,
alkyl,
C(O)-alkyl,
(D)-aryl or
cycloalkyl;
- each R₆: is independently:
hydrogen,
alkyl,
(D)-aryl,
(D)-heteroaryl,
(D)-N(R₈)₂,
(D)-NR₈C(O)-alkyl,
(D)-NR₈SO₂-alkyl,
(D)-SO₂N(R₈)₂,
(D)-(O)ᵣ-alkyl,
(D)-(O)ᵣ(D)-NR₈COR₈,
(D)-(O)ᵣ(D)-NR₈SO₂R₈,
(D)-(O)ᵣ-heterocyclyl or
(D)-(O)ᵣ(alkyl)-heterocyclyl;
- each R₇: is independently:
hydrogen,
alkyl,
(D)-aryl,
C(O)-alkyl,
C(O)-aryl,
SO₂-alkyl or
SO₂-aryl;
- R₈ and R₉: are each independently:
hydrogen,
alkyl or
cycloalkyl, or
R₈ and R₉ together with the nitrogen to which they are attached form a 5- to 8-membered ring optionally containing an additional heteroatom selected from O, S and NR₅,
wherein alkyl and cycloalkyl are unsubstituted or substituted;
- R₁₂: is:
hydrogen or
alkyl;
- Cy: is:
aryl,
5- or 6-membered heteroaryl,
5- or 6-membered heterocyclyl or
5- or 7-membered carbocyclyl;
- Q: is:
-C(Rₐ₁)(Rₐ₂)(Rₐ₃),
wherein Rₐ₁ is alkyl, alkenyl, alkynyl, alkoxy, (D)-cycloalkyl, (D)-heterocyclyl, (D)-aryl, 5-to 7- membered benzofused bicyclic ring or (D)-heteroaryl,
wherein alkyl, alkenyl, alkynyl, (D)-cycloalkyl, (D)-heterocyclyl, (D)-aryl, 5- or 7-membered benzofused bicyclic ring and (D)-heteroaryl are each unsubstituted or substituted;
- Rₐ₂: is:
alkyl,
alkenyl,
alkynyl,
(D)-cycloalkyl,
aryl,
(D)-N(R₁₃R₁₃),
(D)-NR₁₃C(O)-alkyl,
(D)-NR₁₃C(O)0-alkyl,
(D)-NR₁₃SO₂(alkyl),
(D)-O-alkyl,
(D)-OC(O)-alkyl or
CON(R₁₃R₁₃),
wherein for the group or subgroup -N(R₁₃R₁₃), each R₁₃ may combine with the other to form a 5-, 6- or 7-membered saturated or unsaturated, unsubstituted or substituted, nitrogen containing heterocycle;
- Rₐ₃: is:
hydrogen,
methyl,
ethyl and
propyl;
- each R₁₃: is independently:
hydrogen,
oxo,
alkyl,
alkenyl,
(D)-cycloalkyl,
aryl or
heteroaryl,
wherein alkyl, alkenyl, cycloalkyl, aryl and heteroaryl are unsubstituted or substituted,
M is a bond, O, S(O)ᵤ, NR₇ or CH₂;
D is a bond or alkylene;
E is O, S or NR₇;
X is N or CH;
Y is O or NR₈;
o is 0 - 2;
p is 0 - 2;
r is 0 or 1;
s is 0 - 5;
u is 0 - 2.

In preferred embodiments, the variants have the following meanings:

R₁ is each of the rings as defined above, preferably 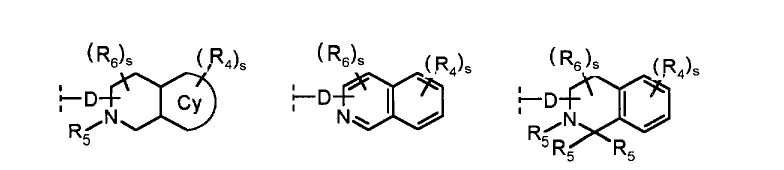 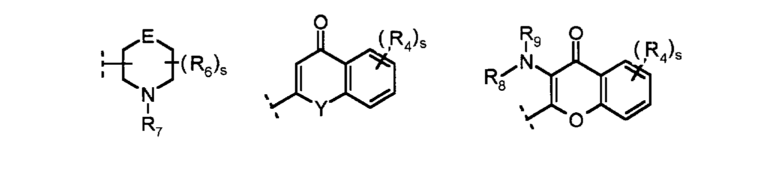 more preferably

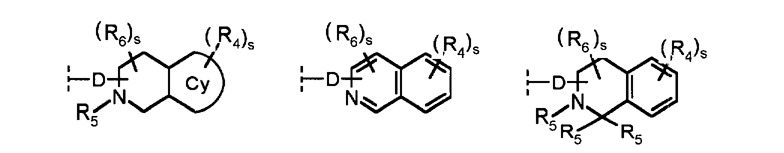

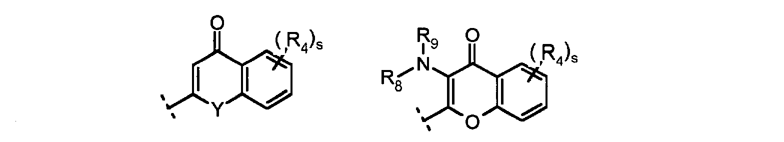

In one embodiment, R₁ is

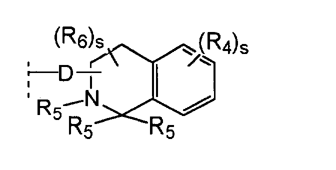

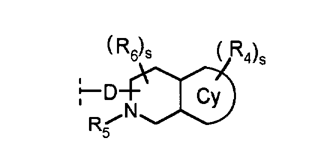

R₂ is as defined above and is preferably (CH₂)-aryl, more preferably (CH₂)-phenyl or (CH₂)-naphthyl. If aryl or heteroaryl are substituted, it is preferably substituted with one to three, more preferably 1 or 2, most preferably 1, substituents. The substituents are preferably independently selected from the group consisting of cyano, nitro, perfluoroalkoxy, halo, alkyl, (D)-cycloalkyl, alkoxy and haloalkyl, more preferably selected from perfluoroalkoxy, halo, alkyl, alkoxy or haloalkyl, most preferably selected from halo, alkyl, alkoxy and haloalkyl, in particular halo.

Most preferably, R₂ is (CH₂)-phenyl or (CH₂)-naphthyl which both, preferably (CH₂)-phenyl, may be substituted with one to three, in particular one, halo, e.g. Cl. The substitution can be in any position, preferably in the 4-position.

R₃ is as described above. In one embodiment R₃ is a bond.

R₄ is as defined above wherein aryl, heteroaryl, heterocyclyl, alkyl or cycloalkyl is preferably unsubstituted or substituted with one to three substituents selected from the group consisting of oxo, alkyl, N(R₅)₂, OR₅, SR₅ and CO₂R₅. Preferably, R₄ is hydrogen, halo, alkyl, haloalkyl, alkoxy or (D)-cycloalkyl, more preferably hydrogen, halo or alkyl, e.g. methyl, ethyl, n-propyl, iso-propyl, most preferably hydrogen.

R₅ is as defined above, preferably hydrogen or alkyl, e.g. methyl, ethyl, n-propyl, iso-propyl, more preferably hydrogen.

R₆ is as defined above, preferably hydrogen, (D)-aryl, (D)-heteroaryl, (D)-N(R₈)₂, (D)-NR₈C(O)alkyl, (D)-NR₈SO₂alkyl or alkyl, e.g. methyl, ethyl, n-propyl, iso-propyl, more preferably hydrogen.

R₇ is is as defined above, preferably hydrogen or alkyl, e.g. methyl, ethyl, n-propyl, iso-propyl, more preferably hydrogen.

R₈ and R₉ are each independently as defined above. When R₈ and R₉ form a ring, the ring preferably contains an additional heteroatom selected from O, S and NR₅. R₈ and R₉ are each independently preferably selected from the group consisting of hydrogen, alkyl and cycloalkyl, or R₈ and R₉ together with the nitrogen to which they are attached form a 5- to 7-membered ring optionally containing an additional heteroatom selected from O, S and NR₅. Moreover, alkyl and cycloalkyl are preferably unsubstituted or substituted with one to three, preferably one or two, groups independently selected from R₁₀ and oxo. More preferably R₈ and R₉ are each independently selected from the group consisting of hydrogen and alkyl, or R₈ and R₉ together with the nitrogen to which they are attached form a 5- to 6-membered nng optionally containing an additional oxygen atom.

R₁₀ is alkyl, (D)-aryl, (D)-cycloalkyl, (D)-heteroaryl, halo, OR₁₁, NHSO₂R₁₁, N(R₁₁)₂, C=N, CO₂R₈, C(R₁₁)(R₁₁)N(R₁₁)₂, nitro, SO₂N(R₁₁)₂, S(O)ᵤR₁₁, CF₃ or OCF₃ and preferably selected from the group consisting of alkyl, OR₁₁, (D)-aryl, (D)-cycloalkyl, (D)-heteroaryl and halo.

R₁₁ is hydrogen, alkyl, (D)-aryl or cycloalkyl, preferably hydrogen, (D)-aryl or alkyl, e.g. methyl, ethyl, n-propyl, iso-propyl, more preferably hydrogen or (D)-aryl.

R₁₂ is as defined above, preferably hydrogen or C₁ - C₄ alkyl as defined below, more preferaby hydrogen, methyl or ethyl, most preferably hydrogen or methyl.

Cy is as defined above and preferably selected from aryl, 5- or 6-membered heteroaryl, 5- or 6-membered heterocyclyl and 5- to 7-membered carbocyclyl, more preferably Cy is aryl or heteroaryl. In one embodiment, Cy may be aryl, such as phenyl or naphthyl.

Q is as described above;
wherein Rₐ₁ is as described above, preferably alkyl, alkoxy, (D)-cycloalkyl, (D)-heteroaryl, (D)-heterocyclyl or (D)-aryl, more preferably (D)-cycloalkyl, (D)-heterocyclyl, (D)-heteroaryl and (D)-aryl, most preferably (D)-heteroaryl and (D)-heterocyclyl,
wherein alkyl, alkenyl, alkynyl, (D)-cycloalkyl, (D)-heterocyclyl, (D)-aryl, 5- or 7-membered benzofused bicyclic ring and (D)-heteroaryl, are each unsubstituted or substituted with one to five, preferably one to three substituents, more preferably one substituent independently selected from R_{b}.

R_{b} is hydroxy, halo, alkyl, alkenyl, alkoxy, haloalkyl, (D)-cycloalkyl, (D)-aryl, (D)-heteroaryl, (D)-C(O)-alkyl, (D)-C(O)O-alkyl, (D)-C(O)heteroaryl, (D)-N(R₁₃R₁₃), (D)-NR₁₃C(O)-alkyl, (D)-NR₁₃SO₂(alkyl), (D)-O-alkyl, (D)-OC(O)-alkyl, (D)-heterocyclyl, (D)-S-alkyl or (D)-SO₂N(R₁₃R₁₃), wherein alkyl, alkoxy, cycloalkyl, aryl, heterocyclyl and heteroaryl are unsubstituted or substituted with one to five substituents independently selected from R₁₃, and provided that when R_{b} is halo or hydroxy, it is not substituted on a carbon adjacent to a heteroatom. R_{b} is preferably hydroxy, halo, alkyl, alkoxy, haloalkyl, (D)-cycloalkyl, (D)-aryl, (D)-N(R₁₃R₁₃), (D)-NR₁₃C(O)-alkyl, (D)-NR₁₃SO₂(alkyl) and CON(R₁₃R₁₃), more preferably halo, alkyl, alkoxy, haloalkyl, (D)-N(R₁₃R₁₃), (D)-NR₁₃C(O)-alkyl, (D)-NR₁₃SO₂(alkyl) and CON(R₁₃R₁₃₎, wherein alkyl, alkoxy, cycloalkyl, aryl, heterocyclyl and heteroaryl are unsubstituted or substituted with one to five, preferably one to three substitutents, more preferably one substitutent independently selected from R₁₃.

Rₐ₂ is as described above, preferably alkyl, aryl, (D)-cycloalkyl, (D)-N(R₁₃R₁₃), (D)-NR₁₃C(O)-alkyl, (D)-NR₁₃SO₂(alkyl) or CON(R₁₃R₁₃), more preferably aryl, (D)-cycloalkyl, (D)-N(R₁₃R₁₃), (D)-NR₁₃C(O)-alkyl, (D)-NR₁₃SO₂(alkyl) or CON(R₁₃R₁₃), most preferably aryl, (D)-NR₁₃SO₂(alkyl) or CON(R₁₃R₁₃).

Rₐ₃ is as described above, preferably hydrogen.

R₁₃ is as described above, preferably hydrogen, alkyl, alkenyl, (D)-cycloalkyl and aryl, more preferably hydrogen, alkyl and (D)-cycloalkyl, most preferably hydrogen,
wherein alkenyl, (D)-cycloalkyl, aryl and heteroaryl are unsubstituted or substituted with one to three preferably one substituent selected from the group consisting of alkyl, halo and hydroxyl, preferably halo.

M is as described above, preferably a bond or CH₂.
D is as defined above, preferably a bond or CH₂, more preferably a bond.
E is as described above, preferably NR₇.
X is as defined above. In one embodiment, X is CH.
Y is as defined above, preferably NR₈, more preferably N-alkyl. In one embodiment, Y is N-n-propyl.
Alkyl is as defined below, preferably C₁ - C₄-alkyl.
o is 0 - 2, preferably 0 or 1.
p is 0 - 2, preferably 0 or 1.
r is 0 or 1.
s is 0 - 5, preferably 0 - 3, more preferably 0 or 1, most preferably 0.
u is 0 - 2.

In the above, any of the preferred definitions for each variant can be combined with the preferred definition of the other variants.

In the above and the following, the employed terms have the meaning as described below:

Aryl is an aromatic mono- or polycyclic moiety with 6 to 20 carbon atoms which is preferably selected from phenyl, biphenyl, naphthyl, tetrahydronaphthyl, fluorenyl, indenyl or phenanthrenyl, more preferably phenyl or naphthyl.

Heteroaryl is an aromatic moiety having 6 to 20 carbon atoms with at least one heterocycle and is preferably selected from thienyl, benzothienyl, naphthothienyl, furanyl, benzofuranyl, chromenyl, indolyl, isoindolyl, indazolyl, quinolyl, isoquinolyl, phthalazinyl, quinoxalinyl, cinnolinyl or quinazolinyl, more preferably thienyl, furanyl, benzothienyl, benzofuranyl or indolyl.

Heterocyclyl is a saturated, unsaturated or aromatic ring containing at least one heteroatom selected from O, N and/or S and 1 to 6 carbon atoms and is preferably selected from azetidin-2-one-1-yl, pyrrolidin-2-one-1-yl, piperid-2-one-1-yl, azepan-2-one-1-yl, thienyl, furyl, piperidinyl, pyranyl, pyrrolyl, imidazolyl, pyrazolyl, pyridyl, pyrazinyl, pyrimidinyl, pyridazinyl, triazolyl, isothiazolyl or isoxazyl, more preferably pyridyl, piperidinyl, triazolyl, imidazolyl or pyrazinyl.

Carbocyclyl is a monocyclic or polycyclic ring system of 3 to 20 carbon atoms which may be saturated, unsaturated or aromatic.

Alkyl is straight chain or branched alkyl having preferably 1 to 8 carbon atoms, such as methyl, ethyl, n-propyl, isopropyl, n-butyl, isobutyl, tert-butyl, n-pentyl, isopentyl, neopentyl, hexyl or heptyl, more preferably 1 to 4 carbon atoms.

Cycloalkyl is an alkyl ring having preferably 3 to 8 carbon atoms, such as cyclopropyl, cyclobutyl, cyclopentyl, cyclohexyl, cycloheptyl or cyclooctyl, more preferably 3 to 6 carbon atoms.

Alkoxy is O-alkyl wherein alkyl is as defined above and has preferably 1 to 4 carbon atoms, more preferably 1 or 2 carbon atoms.

Halo or halogen is a halogen atom preferably selected from F, Cl, Br and I, preferably F, Cl and Br.

Haloalkyl is an alkyl moiety as defined above having preferably 1 to 4 carbon atoms, more preferably 1 or 2 carbon atoms, wherein at least one, preferably 1 to 3 hydrogen atoms have been replaced by a halogen atom. Preferred examples are -CF₃, -CH₂CF₃ and -CF₂CF₃.

Therein, the alkylene moiety may be a straight chain or branched chain group. Said alkylene moiety preferably has 1 to 6 carbon atoms. Examples thereof include methylene, ethylene, n-propylene, n-butylene, n-pentylene, n-hexylene, iso-propylene, sec-butylene, tert.-butylene, 1,1-dimethyl propylene, 1,2-dimethyl propylene, 2,2-dimethyl propylene, 1,1-dimethyl butylene, 1,2-dimethyl butylene, 1,3-dimethyl butylene, 2,2-dimethyl butylene, 2,3-dimethyl butylene, 3,3-dimethyl butylene, 1-ethyl butylene, 2-ethyl butylene, 3-ethyl butylene, 1-n-propyl propylene, 2-n-propyl propylene, 1-iso-propyl propylene, 2-iso-propyl propylene, 1-methyl pentylene, 2-methyl pentylene, 3-methyl pentylene and 4-methyl pentylene. More preferably, said alkylene moiety has 1 to 3 carbon atoms, such as methylene, ethylene, n-propylene and iso-propylene. Most preferred is methylene.

The compounds of structural formulas (I) and (II) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

### Optical Isomers - Diastereomers - Geometric Isomers - Tautomers

Compounds of structural formulas (I) and (II) contain one or more asymmetric centers and can occur as racemates and racemic mixtures, single enantiomers, diastereomeric mixtures and individual diastereomers. The present invention is meant to comprehend all such isomeric forms of the compounds of structural formulas (I) and (II).

Some of the compounds described herein may exist as tautomers such as keto-enol tautomers. The individual tautomers, as well as mixtures thereof, are encompassed within the compounds of structural formulas (I) and (II).

Compounds of structural formulas (I) and (II) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the structural formulas (I) and (II) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids, including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines and cyclic amines, and basic ion exchange resins, such as arginine, betaine, caffeine, choline, N,N'-dibenzylethylenedliamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formulas (I) and (II) are meant to also include the pharmaceutically acceptable salts.

### Utility

Compounds of formulas (I) and (II) are melanocortin receptor modulators and as such are useful in the treatment, control or prevention of diseases, disorders or conditions responsive to the activation or inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance), hypertension, hyperlipidemia, osteoarthritis, cancer, gall bladder disease, sleep apnea, depression, anxiety, compulsion, neuroses, insomnia/sleep disorder, substance abuse, pain, male and female sexual dysfunction (including impotence, loss of libido and erectile dysfunction), fever, inflammation, immune-modulation, rheumatoid arthritis, skin tanning, acne and other skin disorders, neuroprotective and cognitive and memory enhancement, including the treatment of Alzheimer's disease.

Some compounds encompassed by formulas (I) and (II) show highly selective affinity for the melanocortin-4 receptor relative to MC-1R, MC-2R, MC-3R and MC-5R, which makes them especially useful in the prevention and treatment of cancer cachexia, muscle wasting, anorexia, anxiety, depression, obesity, as well as male and/or female sexual dysfunction, including erectile dysfunction. "Male sexual dysfunction" includes impotence, loss of libido and erectile dysfunction. "Female sexual dysfunction" can be seen as resulting from multiple components, including dysfunction in desire, sexual arousal, sexual receptivity and orgasm.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like, may be employed. Dosage forms include tablets, troches, dispersions, suspensions, solutions, capsules, creams, ointments, aerosols and the like. Preferably compounds of formulas (I) and (II) are administered orally or topically.

The effective dosage of an active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formulas (I) and (II) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compound of formulas (I) and (II) is preferably formulated into a dosage form prior to administration. Accordingly, the present invention also includes a pharmaceutical composition comprising a compound of formulas (I) and (II) and a suitable pharmaceutical carrier.

The present pharmaceutical compositions are prepared by known procedures using well-known and readily available ingredients. In making the formulations of the present invention, the active ingredient (a compound of formulas (I) and (II)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl, propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formulas (I) and (II), the terms "A moiety", "B moiety" and "C moiety" are used below. This moiety concept is illustrated below:

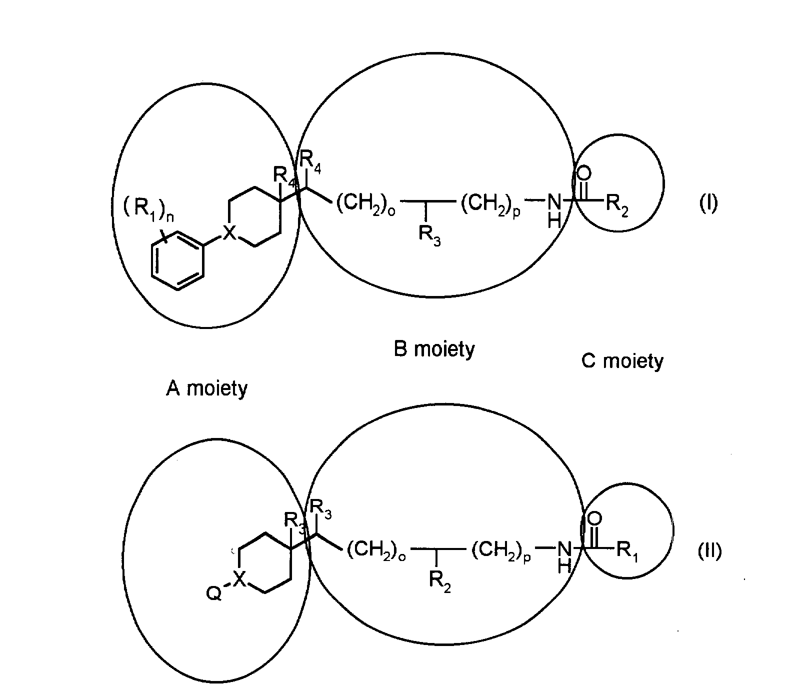

Preparation of the compounds of the present invention may be carried out via sequential or convergent synthetic routes. The skilled artisan will recognize that, in general, the "B" and "C moieties" of a compound of formulas (I) and (II) are connected via amide bonds. The skilled artisan can, therefore, readily envision numerous routes and methods of connecting these two moieties via standard peptide coupling reaction conditions.

The phrase "standard peptide coupling reaction conditions" means coupling a carboxylic acid with an amine using an acid activating agent such as EDC, dicyclohexylcarbodiimide and benzotriazol-1-yloxytris(dimethylamino)-phosphonium hexafluorophosphate in an inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene, et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The compounds of formulas (I) and (II), when existing as a diastereomeric mixture, may be separated into diastereomeric pairs of enantiomers by fractional crystallization from a suitable solvent, such as methanol, ethyl acetate or a mixture thereof. The pair of enantiomers thus obtained may be separated into individual stereoisomers, by conventional means using an optically active acid as a resolving agent. Alternatively, any enantiomer of a compound of the formulas (I) and (II) may be obtained by stereospecific synthesis using optically pure starting materials or reagents of known configuration.

The compounds of formulas (I) and (II) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described above. The amine-free bases corresponding to the isolated salts can be generated by neutralization with a suitable base, such as aqueous sodium hydrogencarbonate, sodium carbonate, sodium hydroxide and potassium hydroxide, and extraction of the liberated amine-free base into an organic solvent, followed by evaporation. The amine-free base, isolated in this manner, can be further converted into another pharmaceutically acceptable salt by dissolution in an organic solvent, followed by addition of the appropriate acid and subsequent evaporation, precipitation or crystallization All temperatures are degrees Celsius. Mass spectra (MS) were measured by electron-spray ion-mass spectroscopy.

In the schemes, preparations and examples below, various reagent symbols and abbreviations have the following meanings:
- BINAP: 2,2'-Bis(diphenylphosphino)-1,1'-binaphtyl
- Boc: t-butoxycarbonyl
- Bu: butyl
- Bz₂O₂: dibenzoylperoxide
- DCM: dichloromethane
- DIEA: diisopropylethylamine
- DMAP: 4-dimethylaminopyridine
- DME: dimethoxyethane
- DMF: N,N-dimethylformamide
- EDC: 1-(3-dimethylaminopropyl)-3-ethylcarbodiimide hydrochloride
- Et: ethyl
- EtOAc: ethyl acetate
- Fmoc: 9-fluorenylmethyl-carbamate
- HOAc: acetic acid
- HOAt: 1 -hydroxy-7-azabenzotriazole
- HOBt: 1-hydroxybenzotriazole
- h: hour(s)
- NBS: N-bromosuccinimide
- NMM: N-methylmorpholine
- Me: methyl
- Ms: methanesulfonyl
- Pd₂(dba)₃: tris(dibenzylideneacetone) dipalladium(0)
- Phe: phenylalanine
- TEA: triethylamine
- TFA: trifluoroacetic acid
- THF: tetrahydrofuran
- Tic: 1,2,3,4-tetrahydroisoquinoline-3-carboxylic acid
- TLC: thin layer chromatography
- TMOF: trimethylorthoformate
- Z: benzyloxycarbonyl

### Coupling of the three moieties:

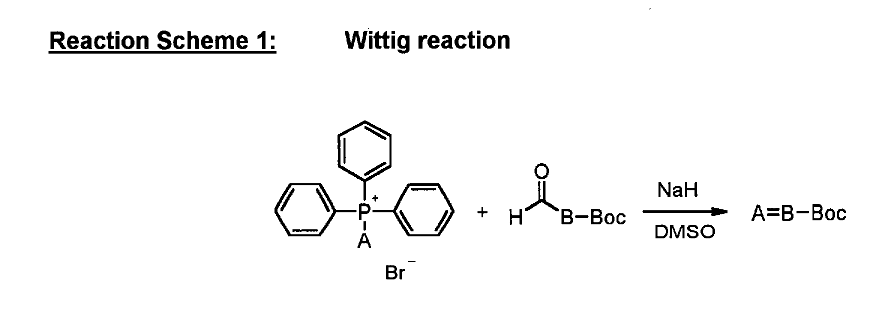

The "A" and the "B moiety" of the compounds of the present invention are coupled using the Wittig reaction.

Generally the Wittig ylid is formed from the "A moiety", and the "B moiety" is introduced as amino acid derived aldehyde. A Wittig ylid can be reacted with an amino aldehyde in the presence of a base such as NaH, in a appropriate solvent such as DMSO, at a suitable temperature *(Synth. Commun.* 1999, 29, 7, 1143-1155; *Tetrahedron Lett.* 1992, 33, 30, 4257-4260) to yield the corresponding alkenes.

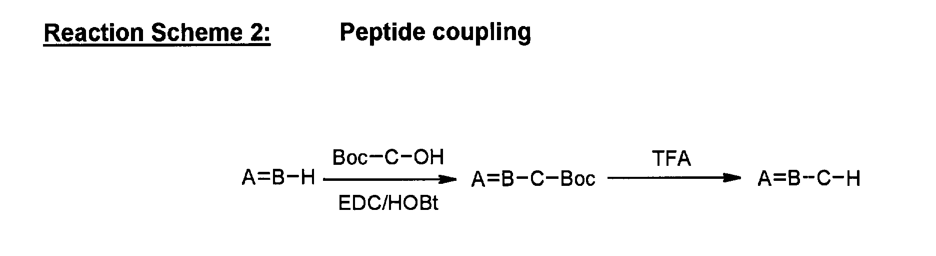

For coupling of H-BA with Boc-C-OH, EDC/HOAt, EDC/HOBt or DCC/HOBt can be used.

Generally, the starting material of Boc-protected amine (Boc-BA) can be deprotected in the presence of TFA/CH₂Cl₂, HCl/EtOAc, HCl/dioxane or HCl in MeOH/Et₂O, with or without a cation scavenger, such as dimethyl sulfide (DMS), before being subjected to the coupling procedure. It can be fre-based before being subjected to the coupling procedure or, in some cases, used as the salt

A suitable solvent, such as CH₂Cl₂, DMF, THF or a mixture of the above solvents, can be used for the coupling procedure. A suitable base includes TEA, DIEA, NMM, collidine or 2,6-lutidine. A base may not be needed when EDC/HOBt is used.

Generally after the reaction is completed, the reaction mixture can be diluted with an appropriate organic solvent, such as EtOAc, CH₂Cl₂ or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

Protecting groups, such as Boc, Z, Fmoc and CF₃CO, can be deprotected in the presence of H₂/Pd-C, TFA/DCM, HCl/EtOAc, HCl/dioxane, HCl in MeOH/Et₂O, NH₃/MeOH or TBAF, with or without a cation scavenger, such as thioanisole, ethane thiol and dimethyl sulfide (DMS). The deprotected amines can be used as the resulting salt or are free-based by dissolving in DCM and washed with aqueous bicarbonate or aqueous NaOH. The deprotected amines can also be free-based by ion exchange chromatography.

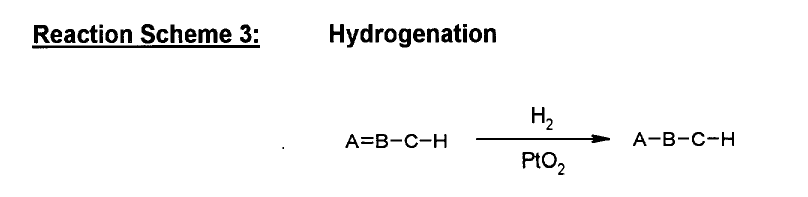

The alkenes A=B-C-H can be hydrogenated using a catalyst such as PtO₂ and an appropriate solvent like methanol at a suitable hydrogen pressure and a capable temperature.

The "A", "B" and "C moieties" of the present invention, in general, may be prepared from commercially available starting materials via known chemical transformations.

### Reaction Schemes for Preparation of "A moiety"

The preparation of "A moiety", of the compound of formula (I), is illustrated in the reaction schemes below.

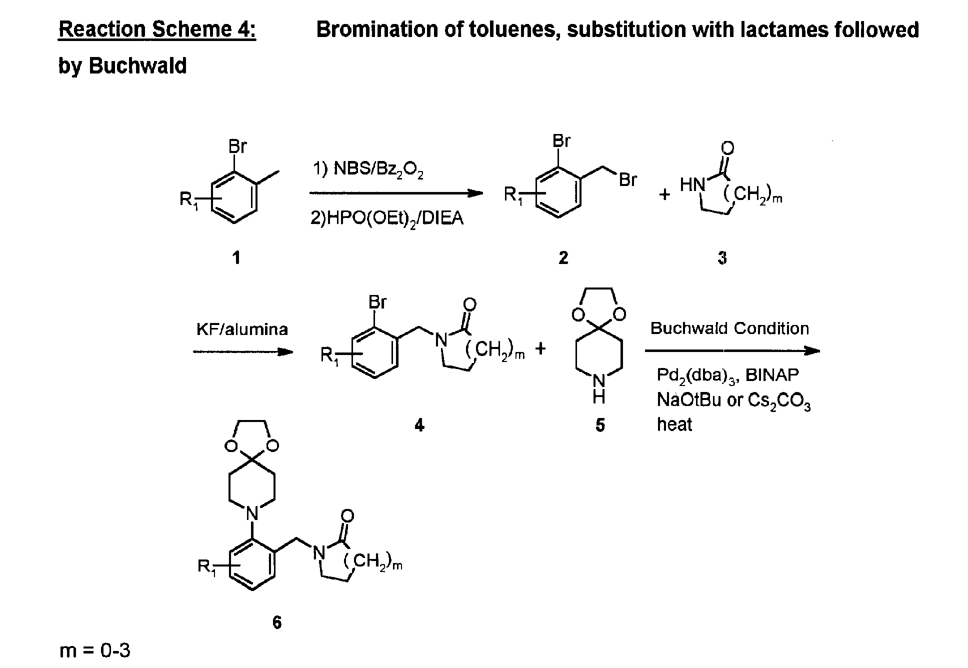

As shown in Reaction Scheme 4, the "A moiety" of the compounds of formula (I) can be prepared by reacting various methyl benzenes **1** with NBS in the presence of a radical starter such as Bz₂O₂, followed by reaction with diethyl phosphite in the presence of a base, such as DIEA, to give benzylbromides **2**, which can then used to alkylate lactames like **3** in the presence of an appropriate base, such as KF/alumina. The substituted bromobenzenes can then be subjected to Buchwald conditions. By coupling bromobenzenes **4** with 1,4-dioxa-8-azaspiro[4.5]decane **5** in the presence of tri(dibenzylideneacetone) dipalladium (Pd₂(dba)₃), 2,2'-Bis(diphenylphosphino)-1,1'-binaphtyl (BINAP) and sodium-tert-butoxide (NaOtBu) or cesium carbonate (Cs₂CO₃) in an organic solvent, such as toluene, at a suitable temperature

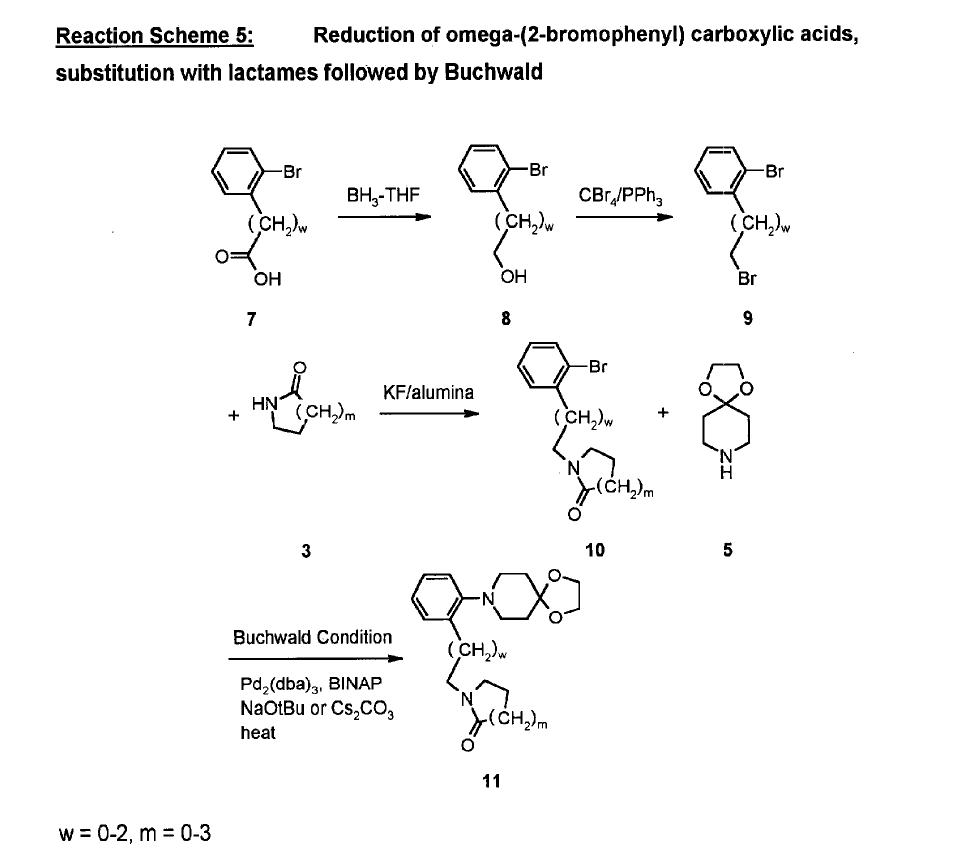

As shown in Reaction Scheme 5, carboxylic acids **7** can be reduced to the corresponding alcohols **8** using an appropriate reagent, such as BH₃-THF, which are subsequently transferred to the corresponding alkyl bromides **9** with reagents such as CBr₄ and PPh₃. The alkyl bromides can then be used to alkylate lactames, like **3**, in the presence of an appropriate base such as KF/alumina The substituted bromobenzenes can then be subjected to Buchwald conditions to yield compounds **11.**

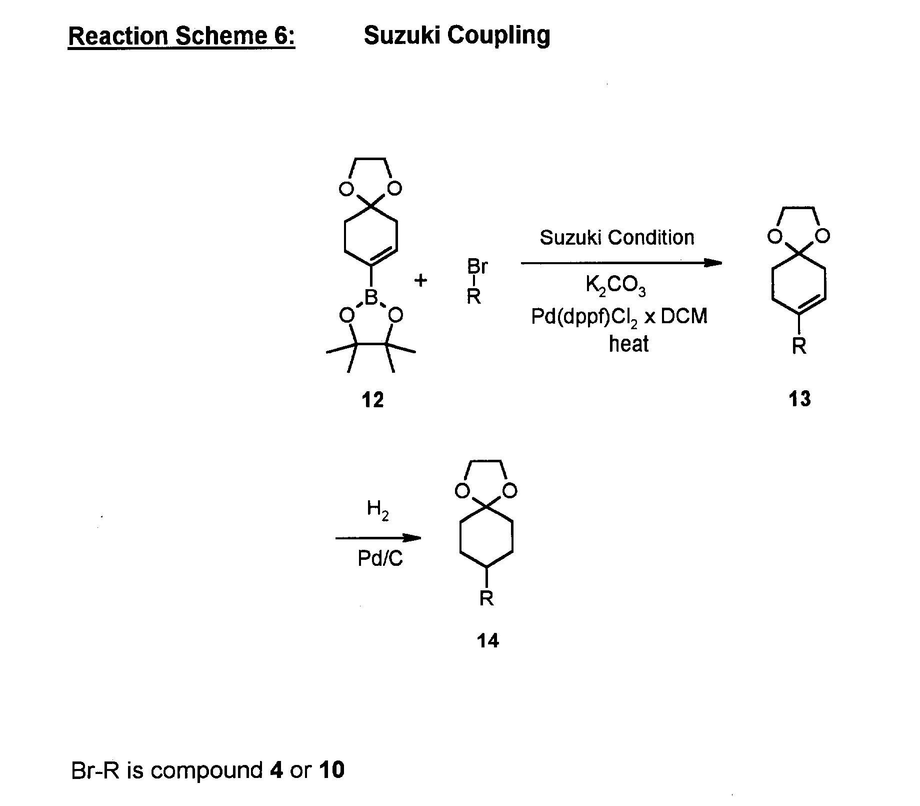

As shown in Reaction Scheme 6, 8-(4,4,5,5-tetramethyl-[1,3,2]dioxaborolan-2-yl)-1,4-dioxa-spiro[4.5]dec-7-ene **12** (analog *Tetrahedron Lett.* 2000, 41, 3705-3708) can be reacted with haloaromates such as **4** or **10,** in the presence of a base such as K₂CO₃ and a catalyst such as dichloro(1,1'-bis(diphenylphosphino)-ferrocene)palladium(II) DCM adduct, in an organic solvent such as DMF, at a suitable temperature. The protected cyclohexenones **13** can be hydrogenated in the presence of a catalyst such as Pd/C, to yield the protected cyclohexanones **14.**

The preparation of "A moiety" of the compounds of formula (II) is illustrated in the reaction schemes below.

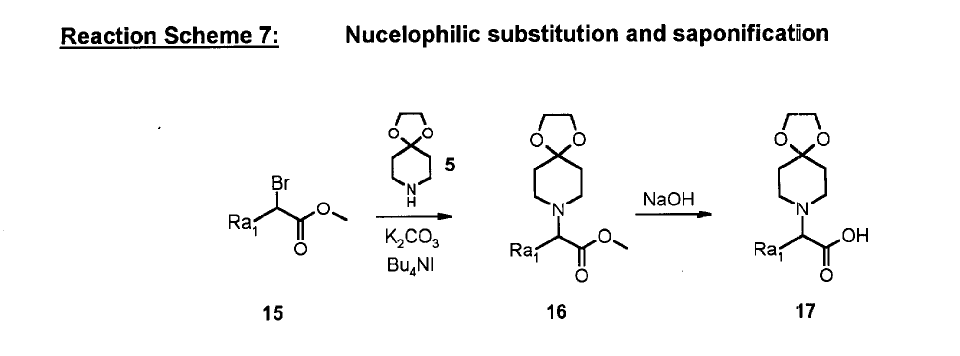

As shown in Reaction Scheme 7, alpha-bromo acetic acids **15** (WO02059095) can be subjected to a nucleophilic substitution reaction with 1,4-dioxa-8-aza-spiro[4.5] decane **5** in the presence of reagents such as K₂CO₃ and Bu₄Nl, in a solvent like MeCN, to give phenylglycine derivatives **16.** The esters can subsequent be saponified with a base such as NaOH, in a solvent like EtOH

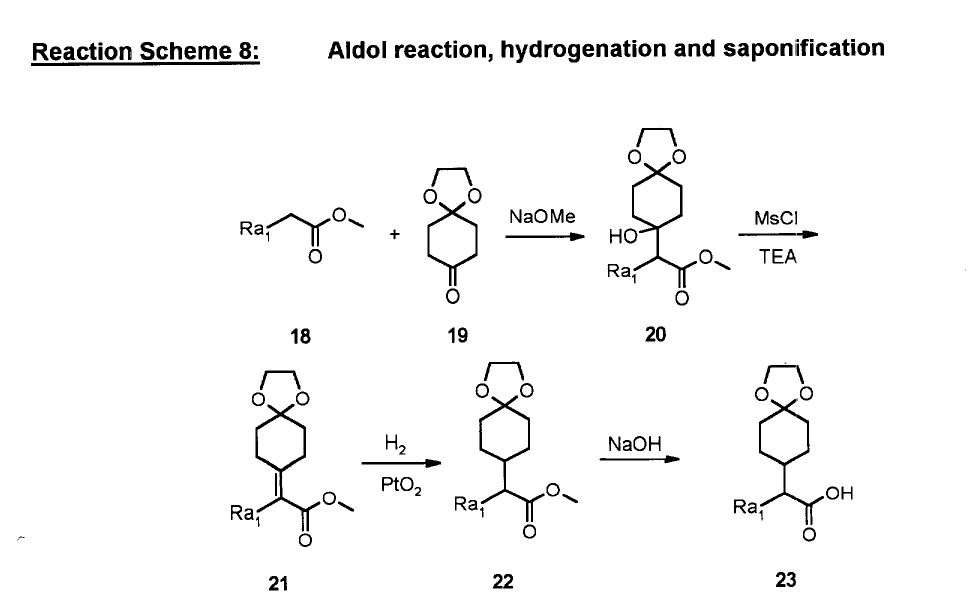

As shown in Reaction Scheme 8, esters 18, which are not branched in the alpha-position, can be deprotonated with a base such as NaOMe, in a suitable solvent such as methanol, and the corresponding carbanion can undergo an aldol reaction with 1,4-dioxaspiro[4.5]decan-8-one to provide compounds **20.** Condensation with a reagent like MsCl, in the presence of a base such as TEA, leads to compound **21.** Hydrogenation of the double bond of compounds **21** can be achieved by using a catalyst, such as PtO₂, and an appropriate solvent like methanol, at a suitable hydrogen pressure and a capable temperature. Compounds **22** can be saponified by treatment with a base like NaOH, in an appropriate solvent such as EtOH, at a suitable temperature.

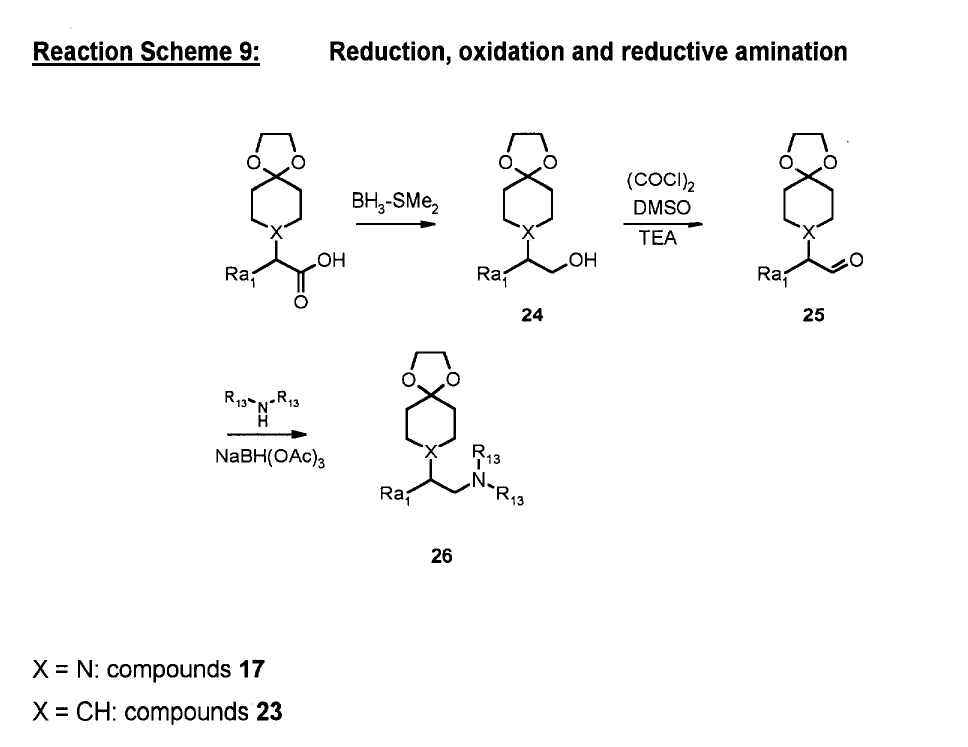

As shown in Reaction Scheme 9, alcohols **24** can be obtained by reduction of the corresponding carboxylic acids **17** and **23** with a reagent such as BH₃-SMe₂, in an appropriate solvent like THF. The alcohols can be transformed to the aldehydes **25** using Swern conditions such as (COCl)₂, DMSO and TEA, in a suitable solvent like DCM. Redutive amination can be achieved by subsequent treatment of the aldehydes with amines in the presence of a reducing reagent such as NaBH(OAc)₃, in a suitable solvent such as DCM.

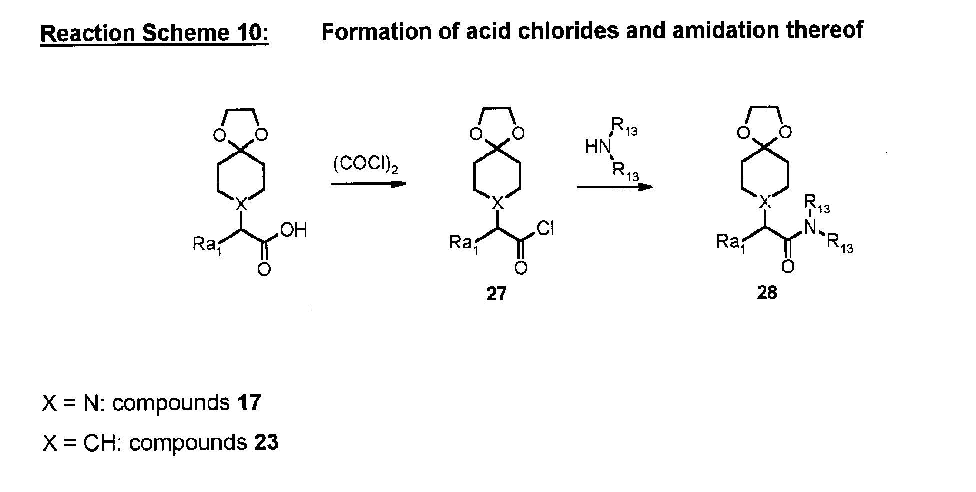

As shown in Reaction Scheme 10, activation of carboxylic acids **17** and **23** with a reagent such as (COCl₂), in an appropriate solvent like DCM, followed by reaction with an amine in a suitable solvent such as DCM, to yield the amides **28.**

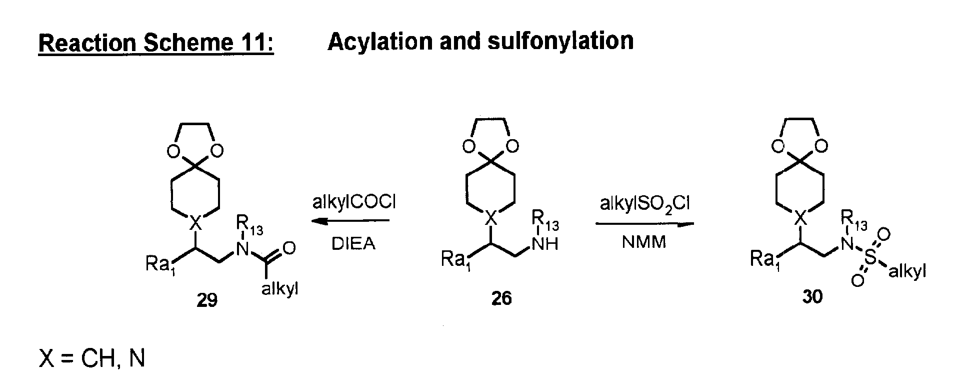

As shown in Reaction Scheme 11, secondary amines **26** can be reacted with acid chlorides and sulfonyl chlorides in the presence of a base like DIEA and NMM, respectively, in an appropriate solvent such as DCM

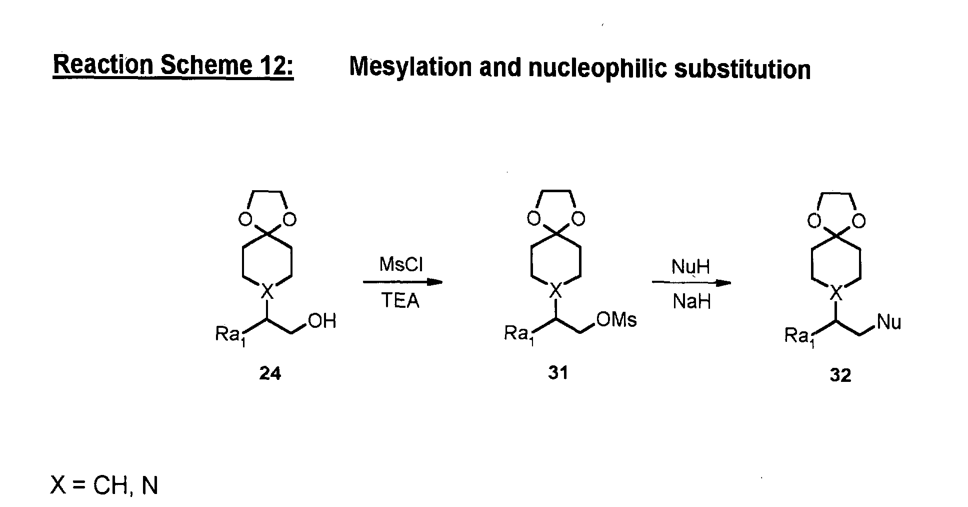

As shown in Reaction Scheme 12, alcohols **24** can be transformed into the corresponding mesylates with reagents such as MsCl, in the presence of a base like TEA. Subsequent reaction of the mesylates with nucleophiles NuH, in the presence of a base such as NaH, leads to compounds **32.**

The "A moieties" of the compounds of formulas (I) and (II) can be further transformed as described in the following Reaction Schemes.

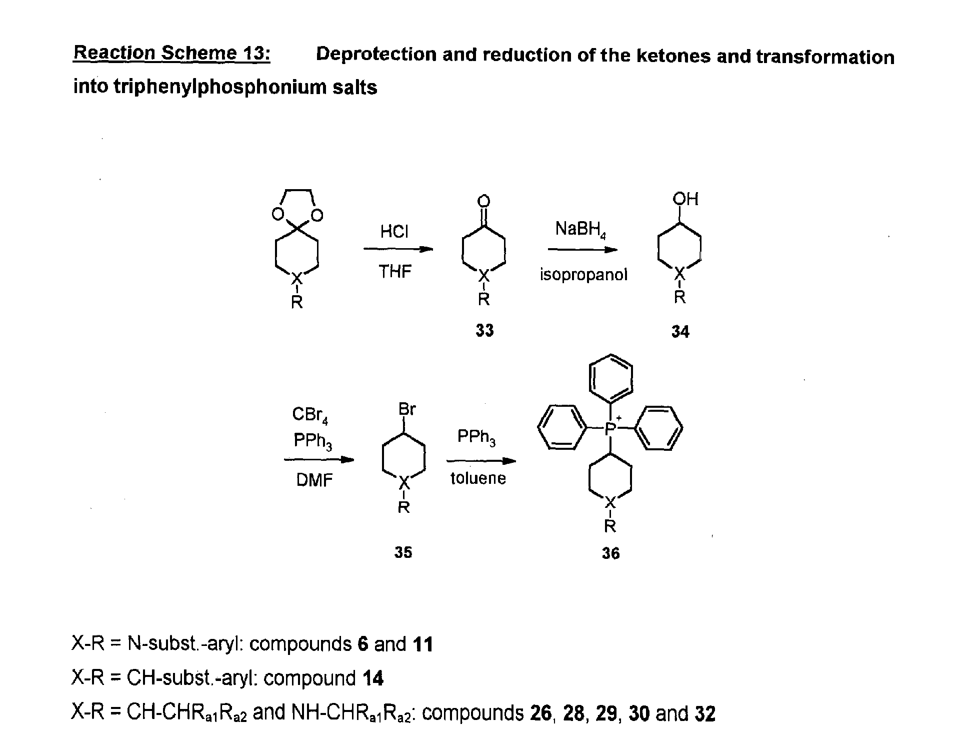

As shown in Reaction Scheme 13, protected ketones **6, 11, 14, 26, 28, 29, 30** and **32** can be deprotected by acid-catalyzed dioxolanation using an acid such as HCl, in an appropriate solvent such as THF, at a suitable temperature. Reduction of the ketones **33** can be performed with complex hydrides such as NaBH₄, in an appropriate solvent such as isopropanol. Alcohols **34** can be transformed to the corresponding bromides **35** with reagents like CBr₄ and PPh₃, in an appropriate solvent such as DMF *(Eur. J. Org. Chem.* 2001, 1335-1347). Reaction of the alkyl bromides with PPh₃ can be performed in a solvent such as toluene, at a suitable temperature to yield phosphonium salts **36.**

### Reaction Schemes for Preparation of "B moiety"

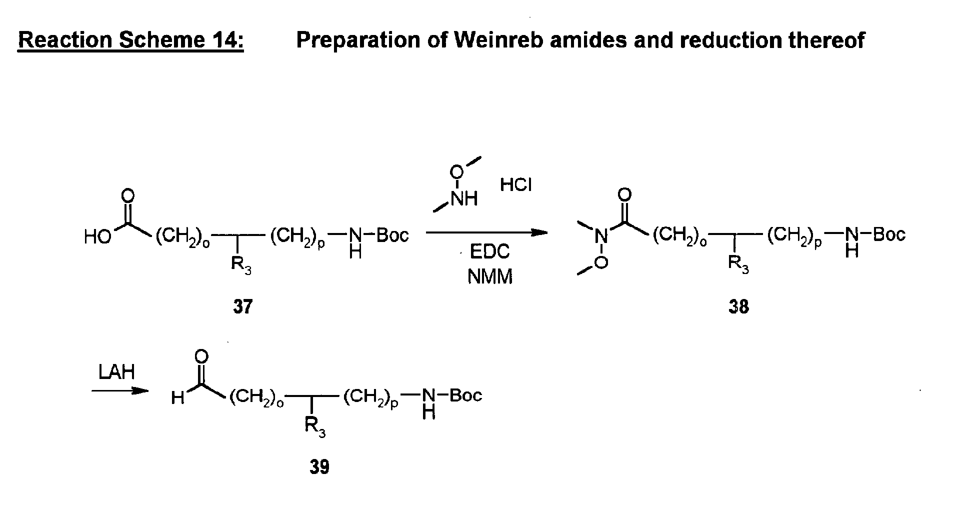

As shown in Reaction Scheme 14, amino acids **37** can be converted into the corresponding Weinreb amides using standard peptide coupling conditions such as EDC/NMM, in an appropriate solvent such as DCM (analog *Synth. Commun.* 1982, 676). Reduction of the Weinreb amides **38** to the aldehydes **39** can be performed with reagents like LAH, in an appropriate solvent such as diethyl ether *(Chirality* 2000, 12, 2).

### Reaction Schemes for Preparation of "C moiety"

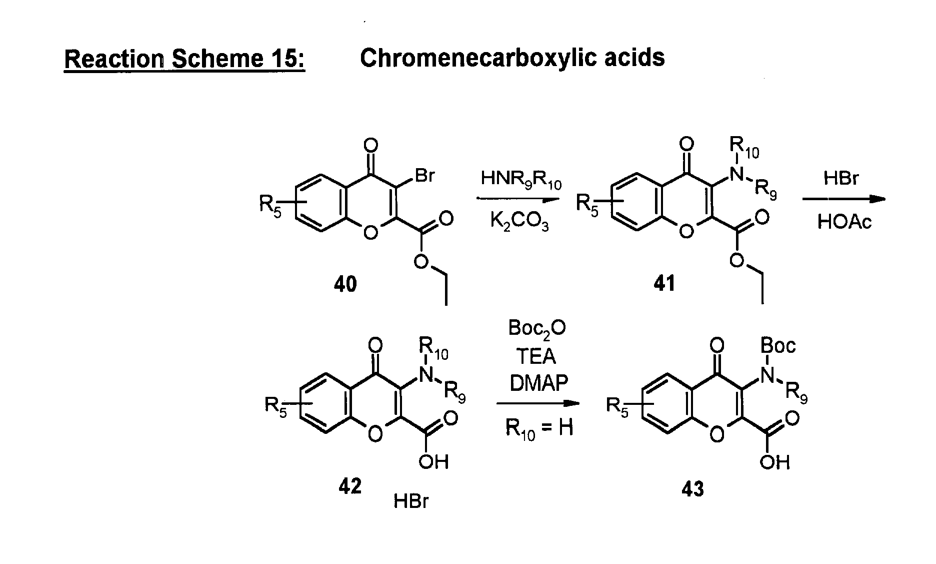

As shown in Reaction Scheme 15, ethyl 3-bromo-4-oxochromene-2-carboxylate **40** *(J. Chem. Soc. Perkin Trans. I* 1986, 1643-1649) can be reacted with amines with or without a base, such as K₂CO₃, in an appropriate solvent such as MeCN, to form products **41** which are subsequently treated with a reagent such as HBr/HOAc to form carboxylic acids **42.** When R₈ is hydrogen, the amine-free can be protected with a reagent such as Boc₂O in the presence of TEA and DMAP, in an appropriate solvent.

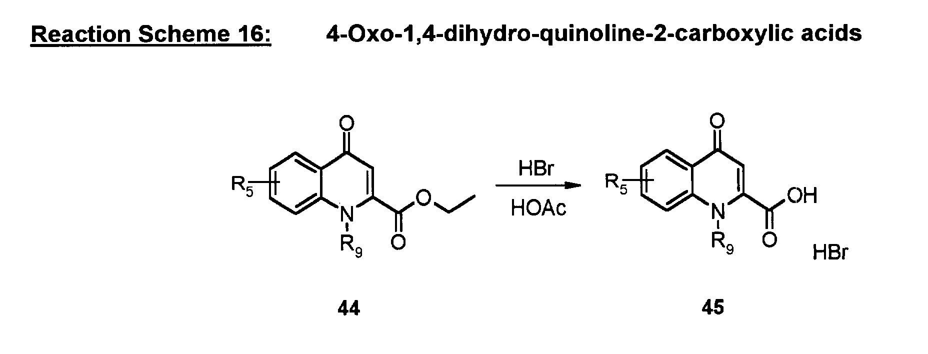

As shown in Reaction Scheme 16, ethyl 4-oxo-1,4-dihydro-quinoline-2-carboxylates **44** *(Bioorg. Med. Chem. Lett.* 2000, 10, 1487-1490) can be converted into the corresponding acids **45** by an appropriate reactant such as HBr/HOAc.

The following describes the detailed examples of the invention.

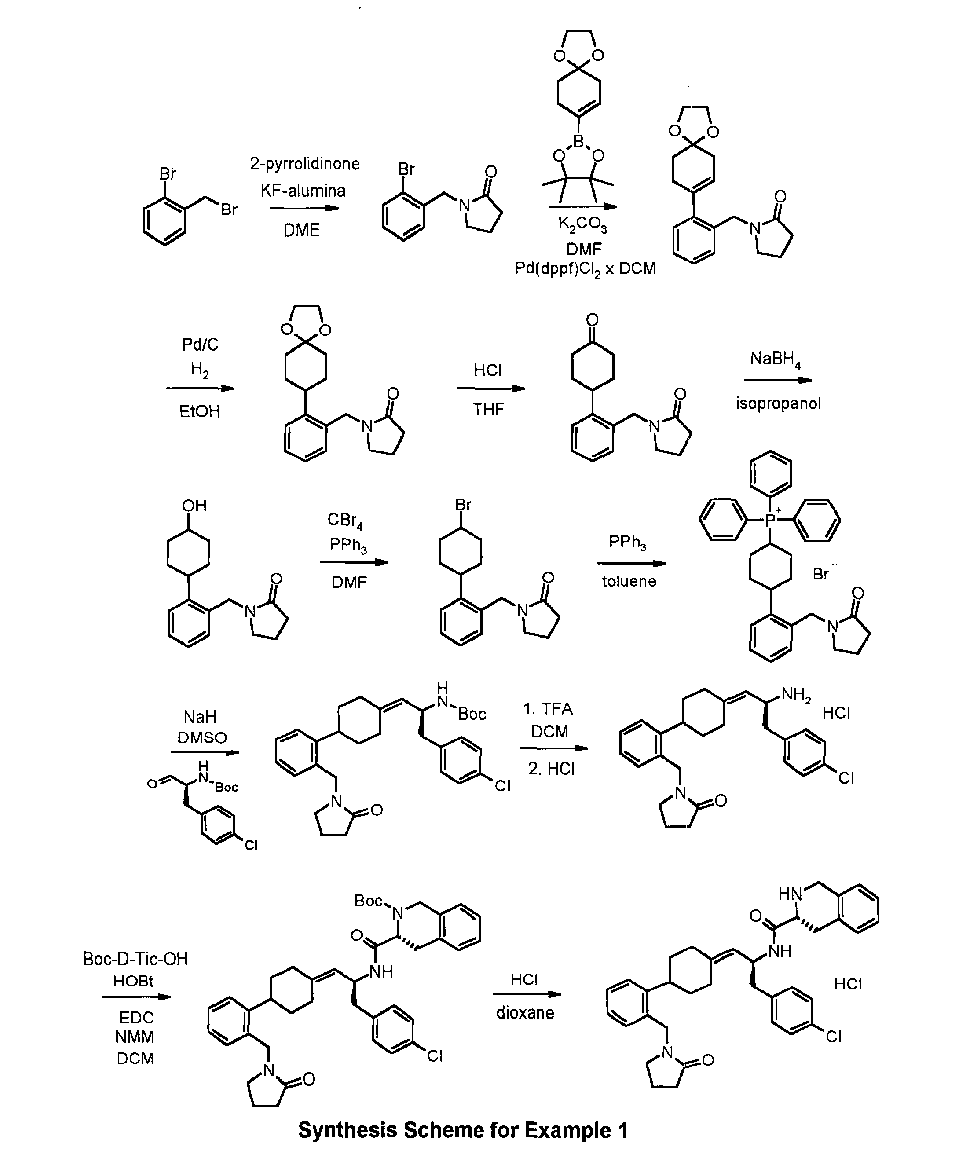

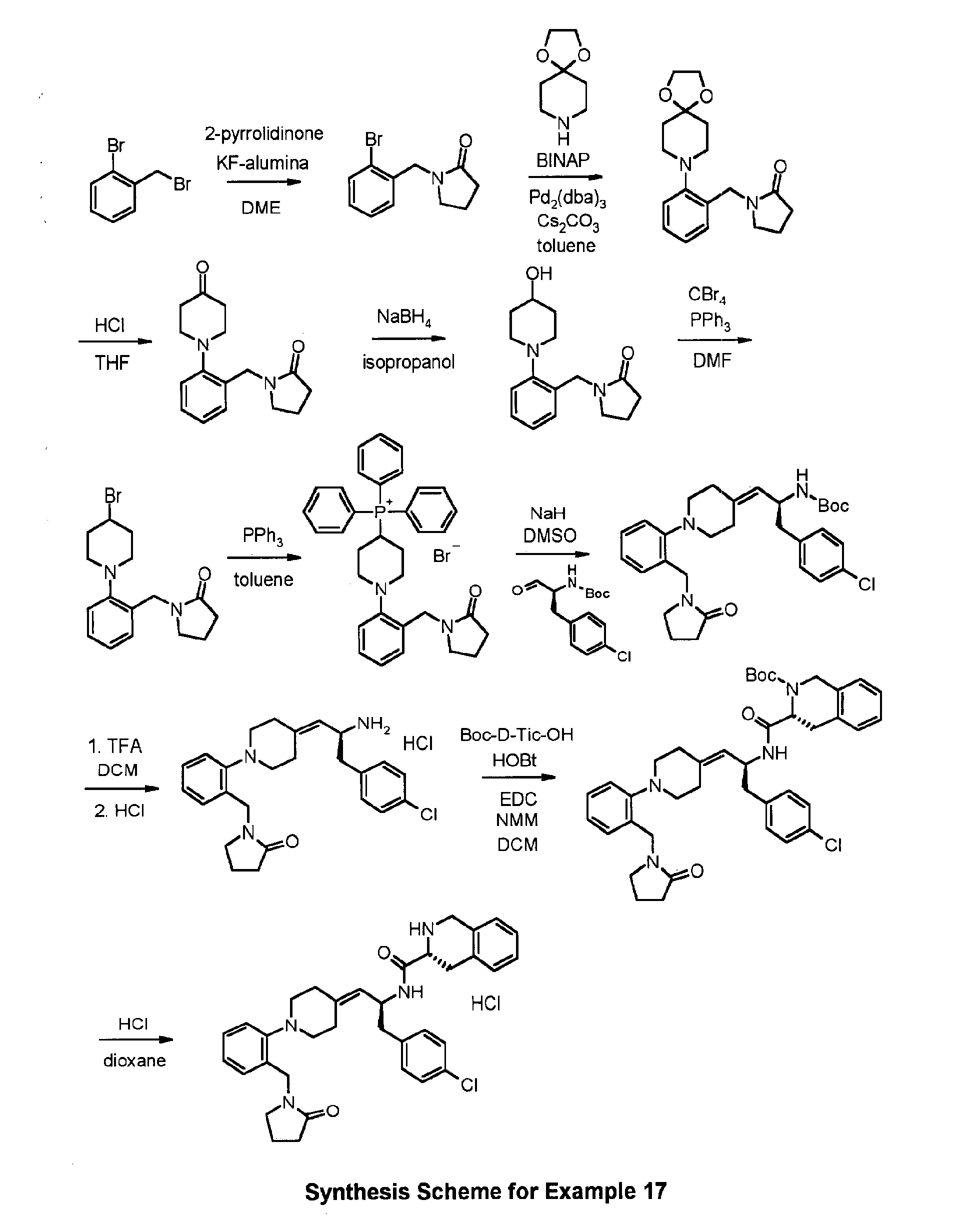

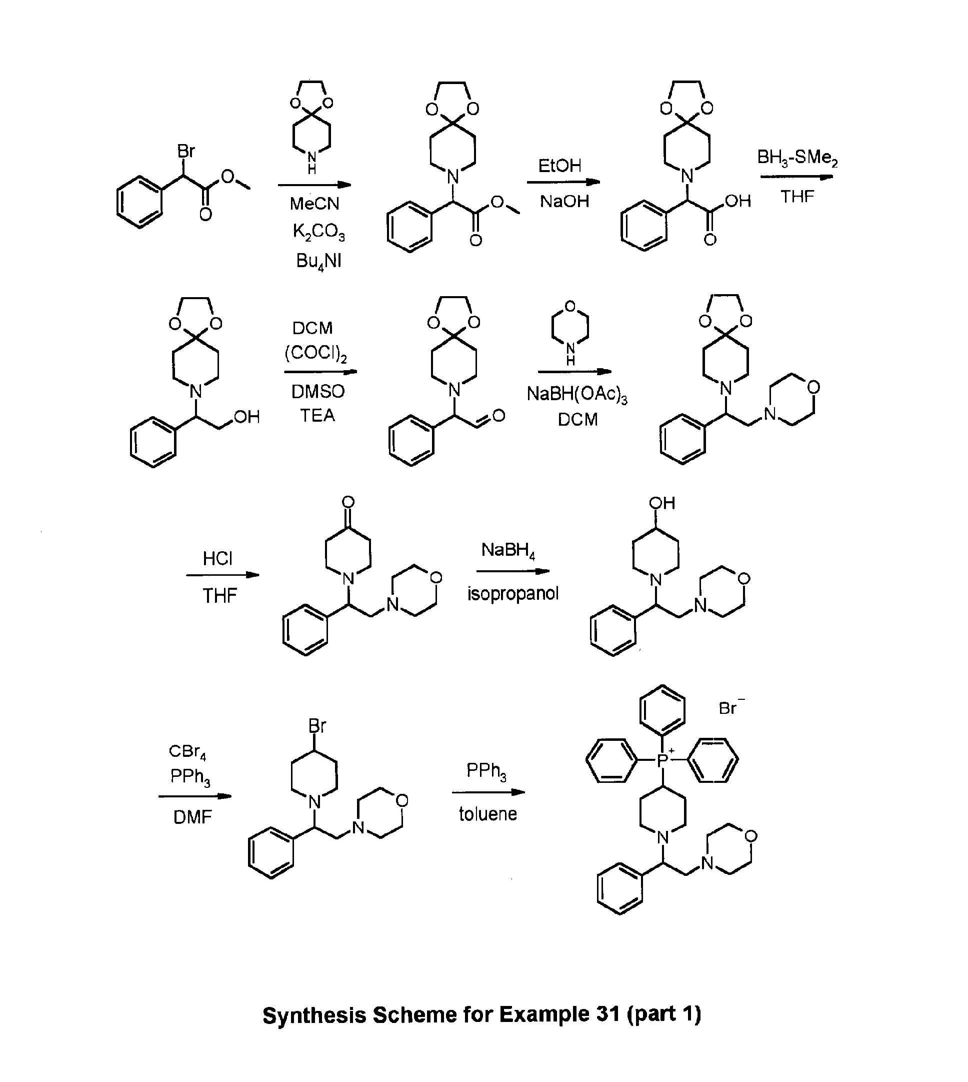

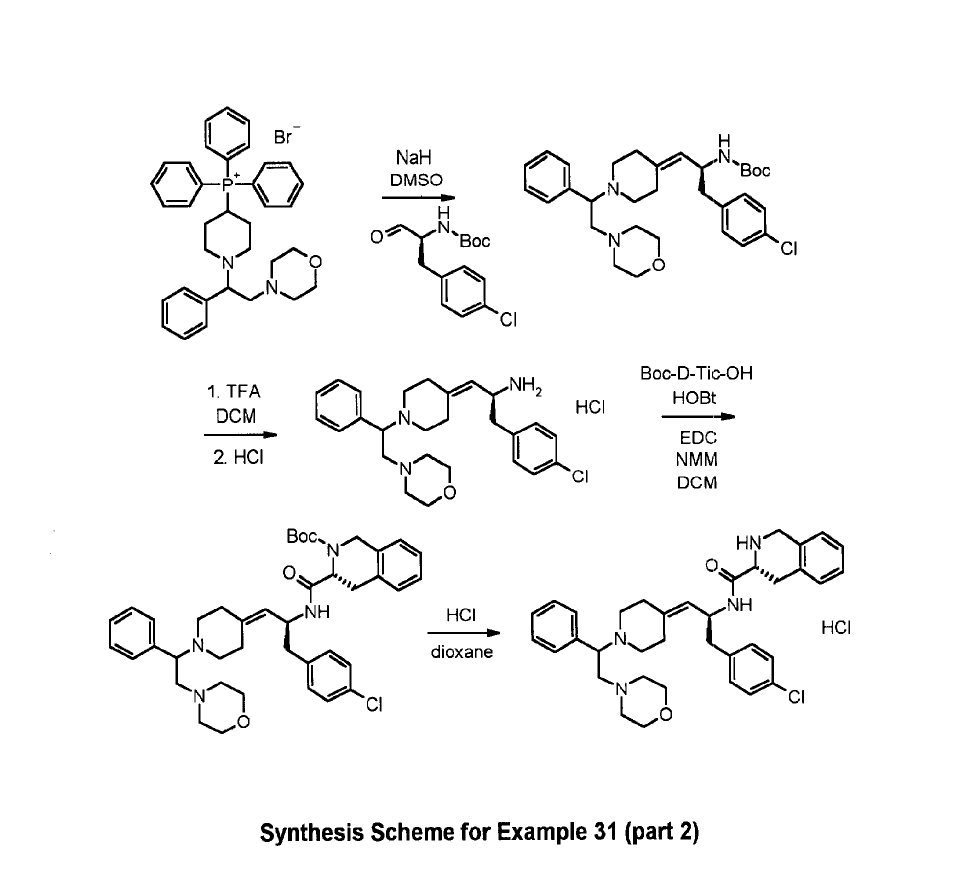

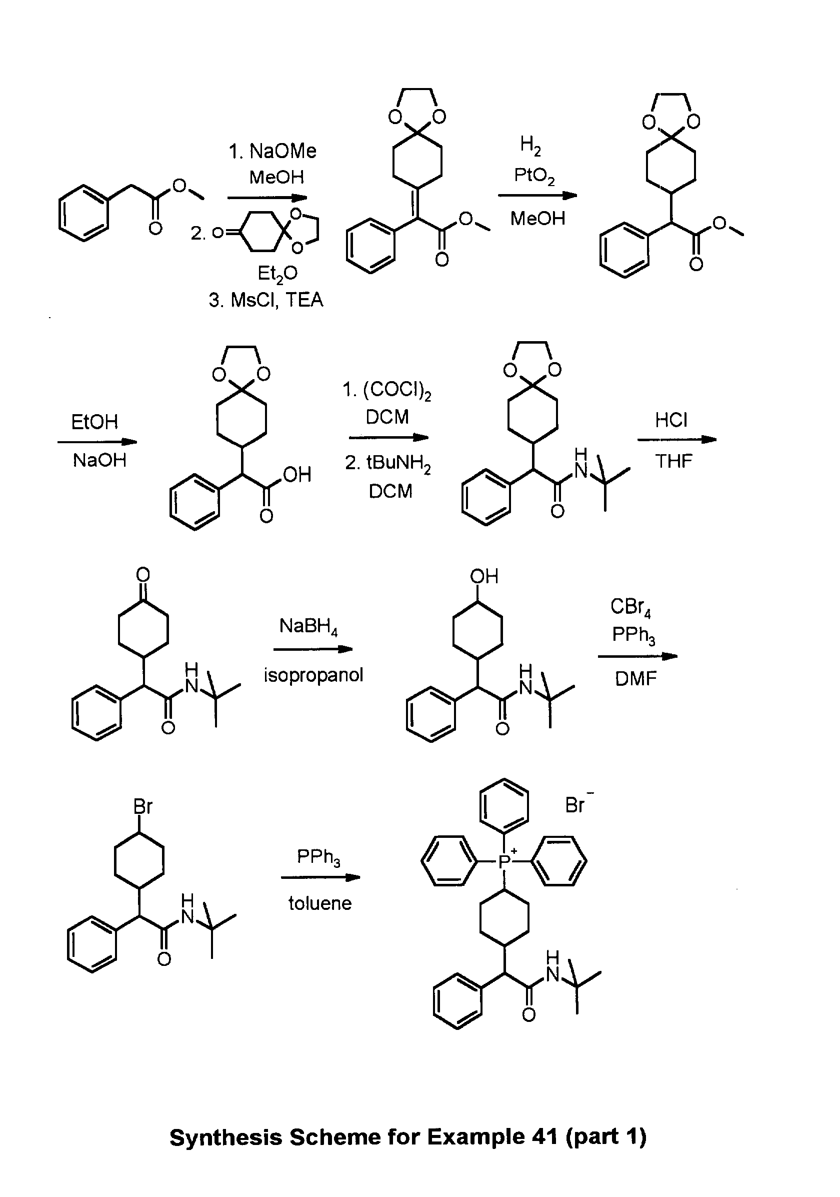

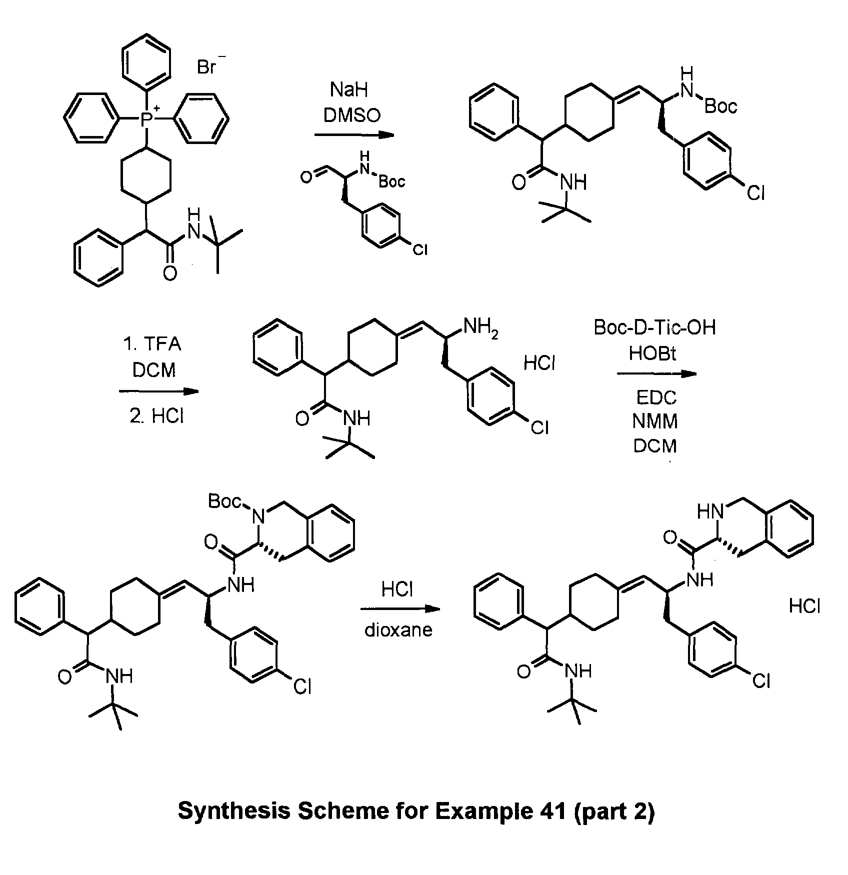

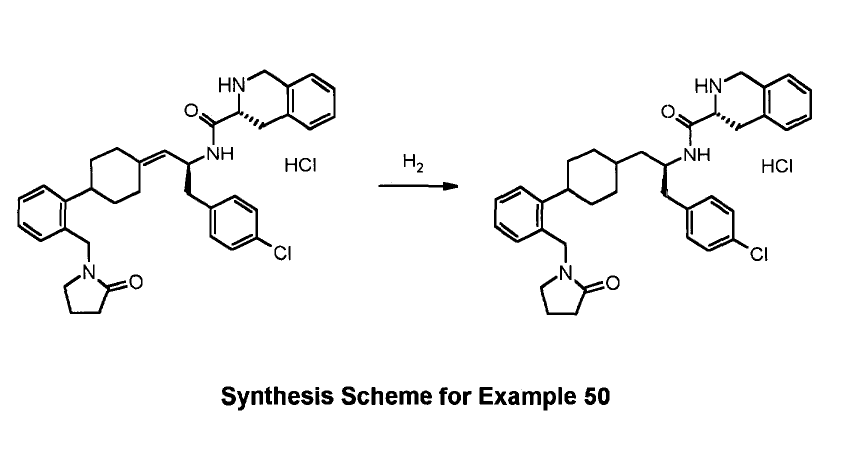

The following examples are provided to illustrate the invention and are not limiting the scope of the invention in any manner.

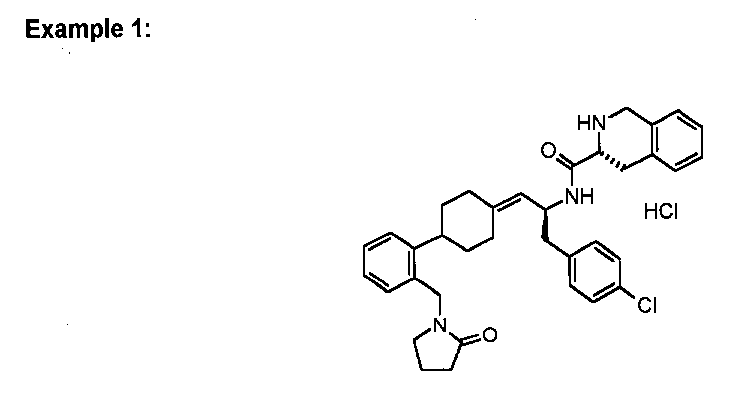

To Boc-protected intermediate 1j) (30 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

The required intermediates can be synthesized in the following way:

### Intermediate 1a):

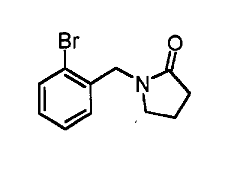

To a solution of 2-bromobenzyl bromide (3.05 g) and 2-pyrrolidinone (0.85 g) in DME (20 ml) was added KF-alumina (0.45 g) and the mixture was stirred for 48 h at room temperature. The inorganics were filtered off and the solvent was removed to afford the desired compound.

### Intermediate 1b):

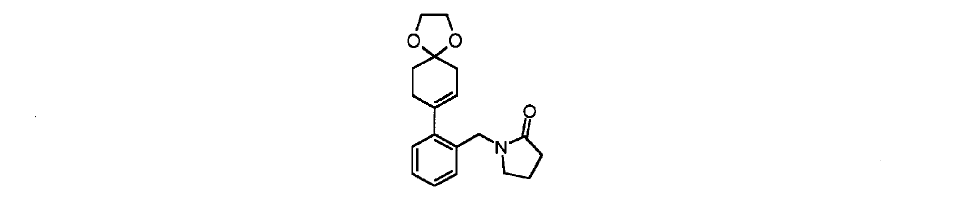

To intermediate 1a) (1246 mg) in DMF (40 ml) was added 8-(4,4,5,5-Tetramethyl[1,3,2]dioxaborolan-2-yl)-1,4-dioxa-spiro[4.5]dec-7-ene (1566 mg), dichloro(1,1'-bis(diphenylphosphino)ferrocene)palladium(II) DCM adduct (216 mg) and K₂CO₃ (2004 mg). The reaction was heated to about 90°C overnight. The mixture was cooled, diluted with DCM and filtered through Celite. The filtrate was concentrated to dryness and the resulting residue was taken up in EtOAc (100 ml). The organics were washed with water, brine and concentrated to dryness. The crude product was purified by flash chromatography

### Intermediate 1c):

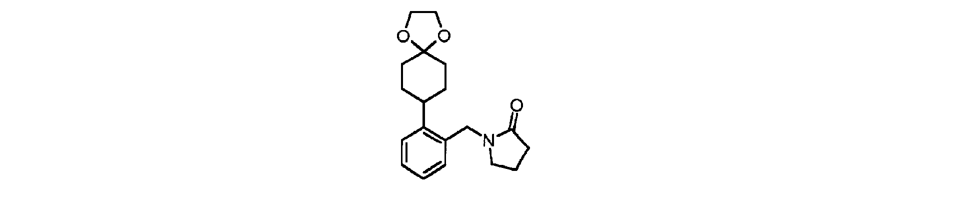

To intermediate 1b) (453 mg) in EtOH (20 ml) was added a slurry of 10% Pd/C in EtOH (20 ml). The mixture was stirred rapidly under H₂ (1 atm) for about 2 h. The reaction mixture was filtered over a pad of Celite and washed with EtOAc (100 ml). The filtrate was concentrated to dryness to yield the final compound.

### Intermediate 1d):

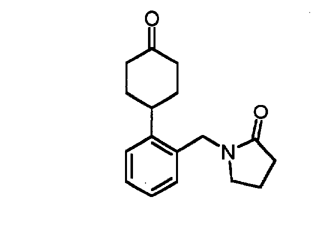

To the protected ketone from 1c) (407 mg) was added 5% HCl in THF (20 ml) and stirred overnight. The volatiles were removed under reduced pressure and the residue was dissolved in EtOAc. The solution was extracted with water and saturated sodium bicarbonate solution, dried over sodium sulfate and the solvent was evaporated to yield the title compound.

### Intermediate 1e):

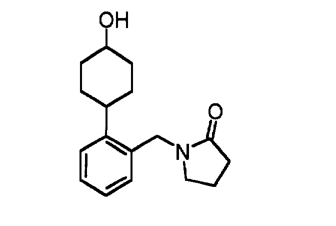

The ketone from 1 d) (302 mg) was added portionwise to a solution of sodium borohydride (17 mg) in isopropanol (10 ml) The reaction mixture was stirred overnight and quenched with 1 M HCl. Volatiles were removed under reduced pressure and the residue was taken up in EtOAc and extracted with 1 M NaOH, water and brine The organic layer was dried over Na₂SO₄ and the solvent was removed in vacuo to yield the desired product

### Intermediate 1f):

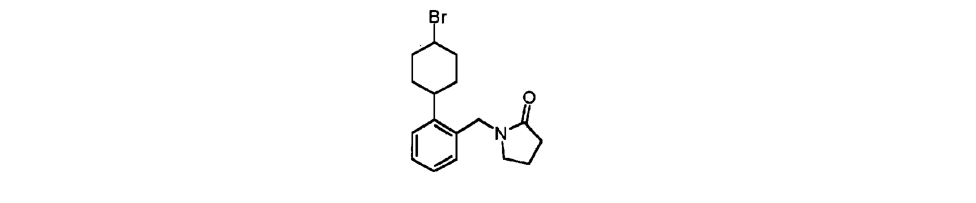

To a stirred solution of intermediate 1e) (253 mg) in DMF (1 ml) under argon at 0°C was added CBr₄ (289 mg). After 15 min PPh₃ (533 mg) was added in small portions over 10 min. The reaction was monitored by TLC. At the end of the reaction the mixture was poured in ice-water and carefully basified with saturated NaHCO₃. The reaction mixture was extracted three times with DCM. The combined organic fractions were dried over Na₂SO₄ and the solvent was evaporated. The title compound was obtained after column chromatography.

### Intermediate 1g):

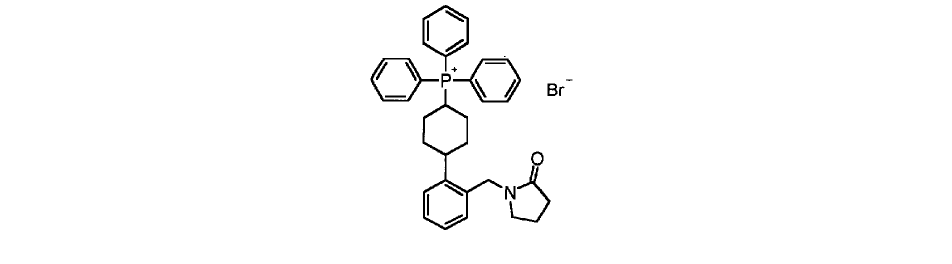

A solution of PPh₃ (499 mg) and intermediate 1f) (280 mg) in toluene (10 ml) was refluxed for 48 h. The precipitate was filtered off, washed with hot toluene and dried under reduced pressure.

### Intermediate 1h):

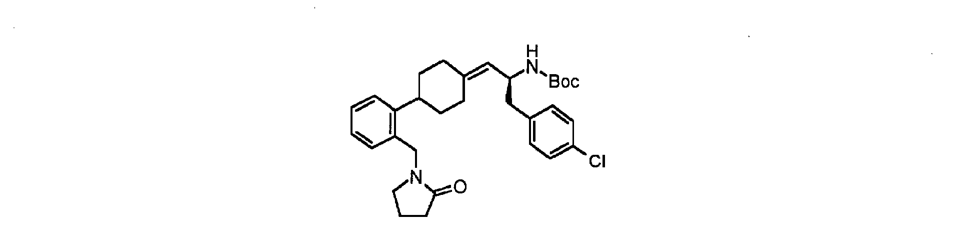

Sodium hydride (40 mg, 60% suspension in mineral oil) was washed three times with hexane and decanted. Dry DMSO (1 ml) was added and heated to 80°C until no more hydrogen was formed. The reaction mixture was cooled in an ice-bath and a solution of intermediate 1 g) (599 mg) in dry DMSO (1 ml) was added. The solution was stirred for 10 min and Boc-4-chlorophenylalaninal (284 mg) was added. The reaction mixture was stirred until complete conversion was observed (monitoring by TLC) and then poured in water (5 ml) and extracted three times with EtOAc. The combined organic layers were washed with water and brine and dried over Na₂SO₄. The solvent was removed under reduced pressure. The crude product was purified by column chromatography.

### Intermediate 1i):

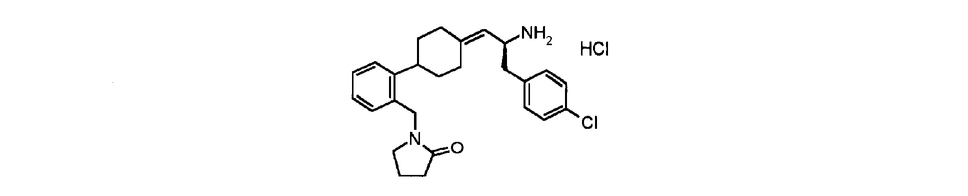

To the Boc-protected amine from 1 h) (143 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄ and concentrated to give a white solid.

For prolonged storage, the free-base was converted into the corresponding hydrochloride. The free-base was dissolved in DCM (5 ml) and app. 1 M HCl in ether (10 ml) was added. The precipitate was filtered and the residue was washed three times with ether and dried under reduced pressure to yield the desired compound.

### Intermediate 1j):

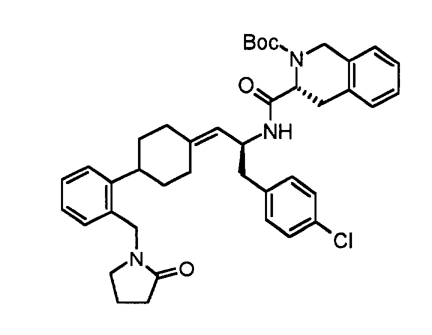

To (R)-Boc-1,2,3,4-tetrahydroisoquinoline-3-carboxylic acid (24 mg) in DCM (2 ml) was added intermediate 1h) (37 mg), N-methylmorpholine (14 µl) and HOBt (14 mg) and it was stirred for 20 min. EDC (23 mg) was added and stirring was continued for 1 h. An additional amount of N-methylmorpholine (8 µl) was added and it was stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was punfied by column chromatography.

The following examples can be prepared in a similar way:

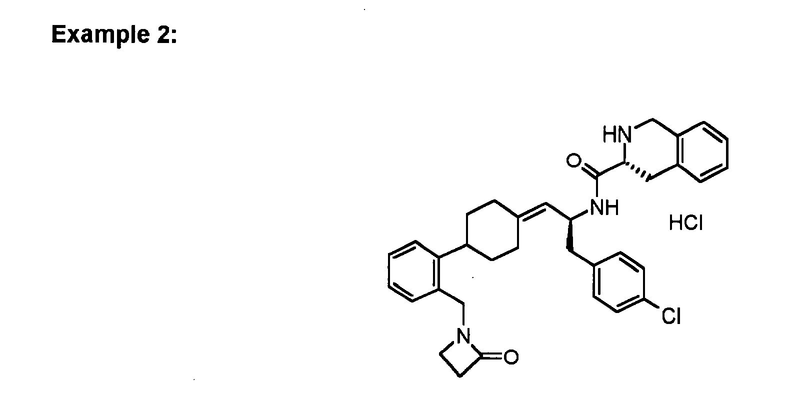

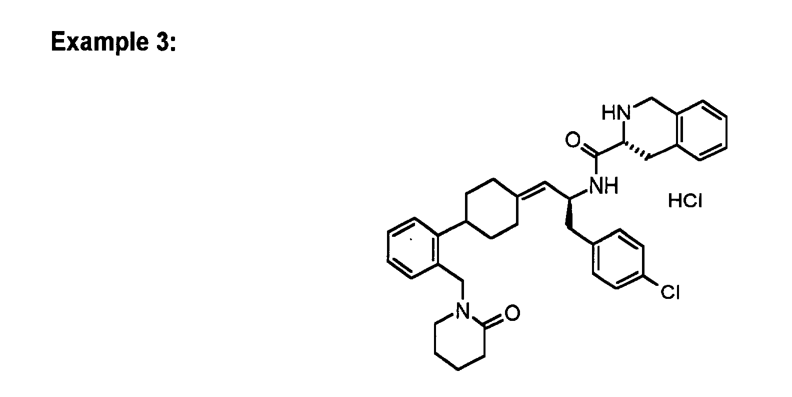

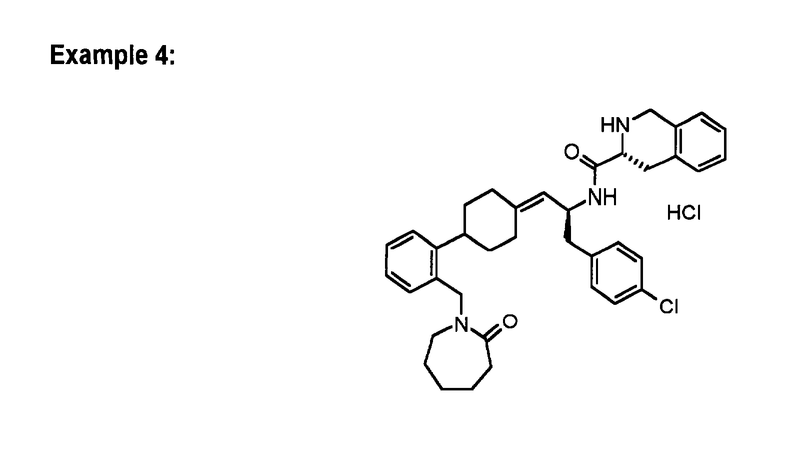

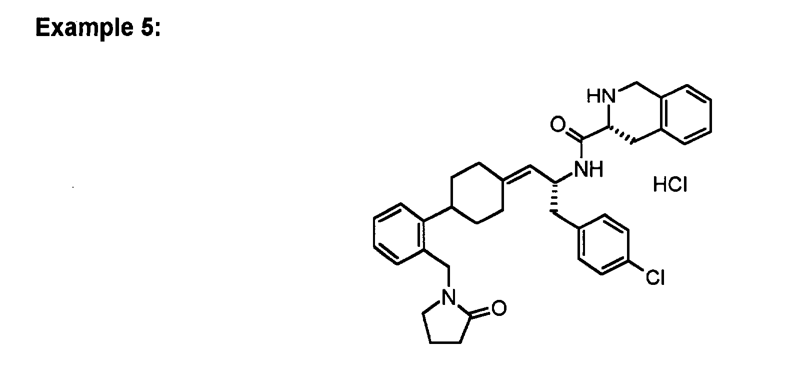

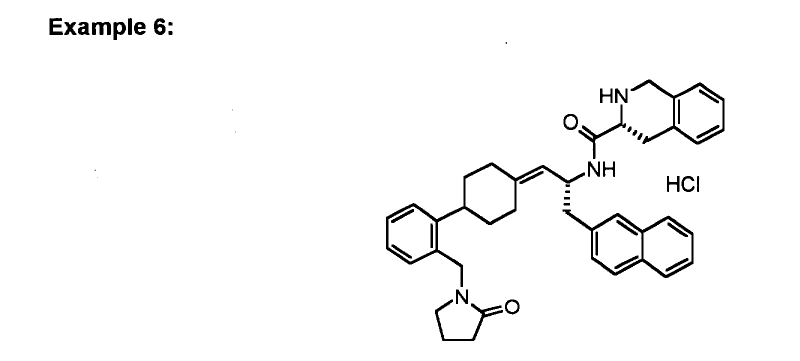

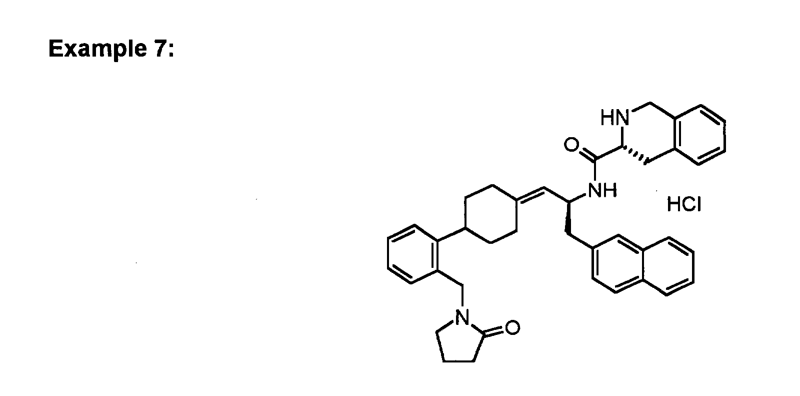

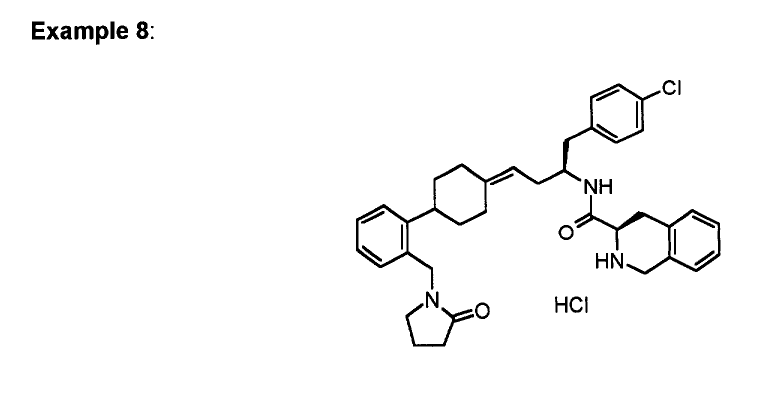

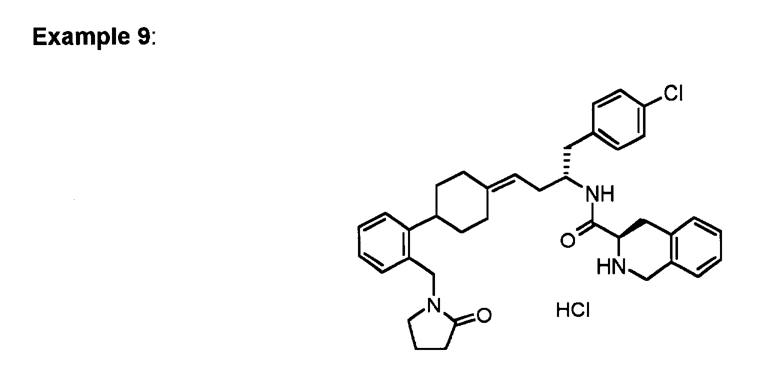

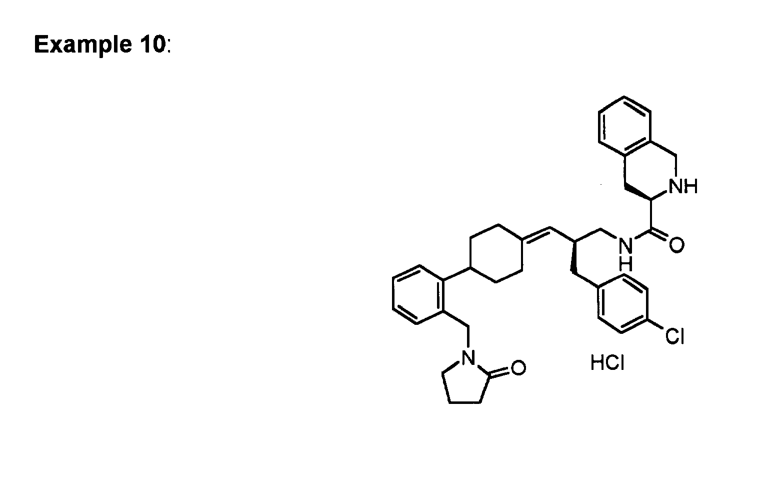

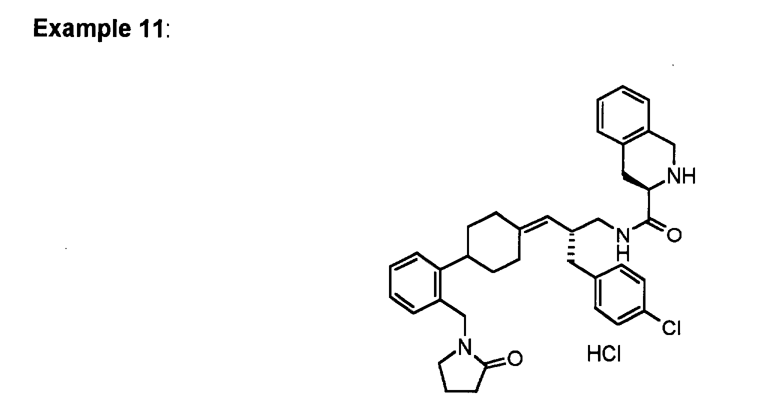

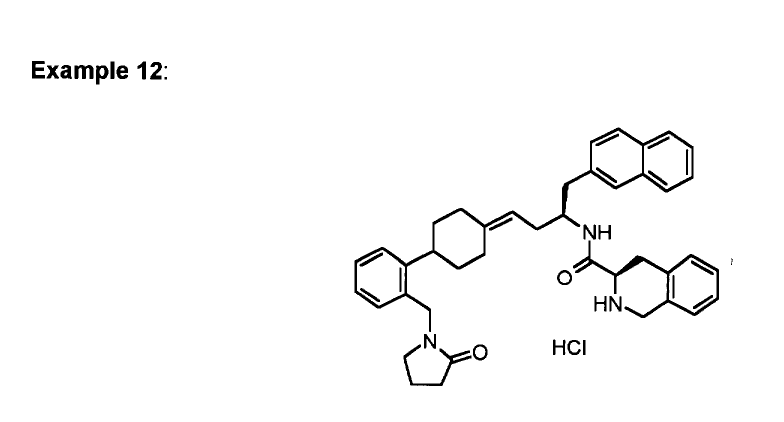

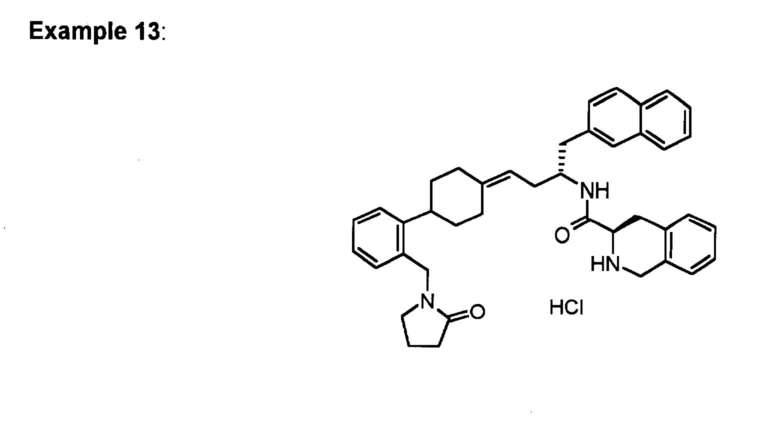

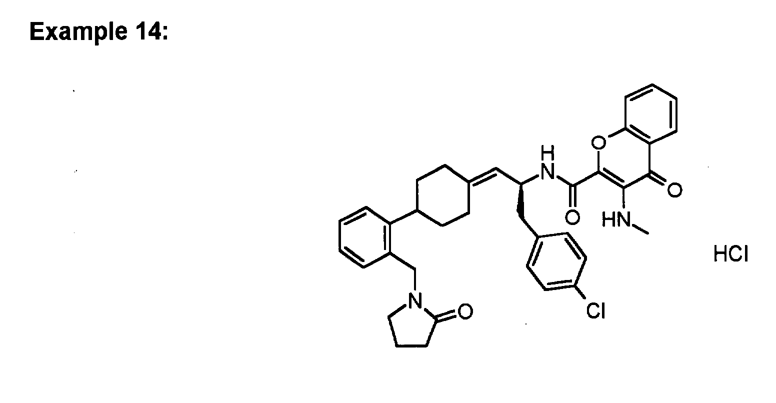

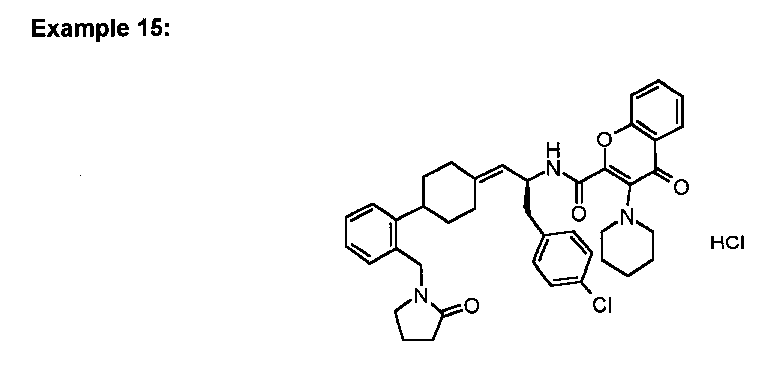

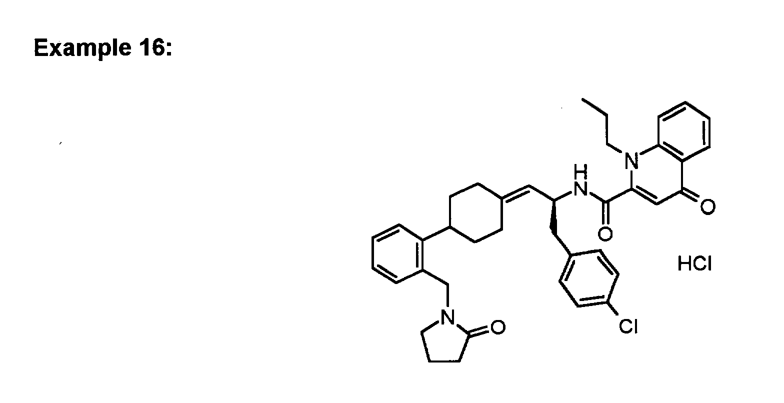

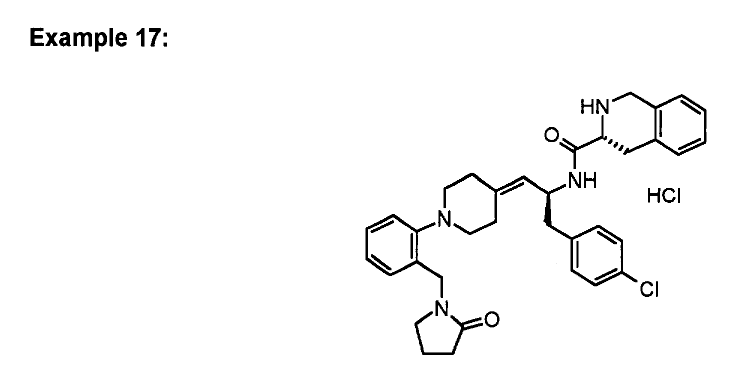

To Boc-protected intermediate 17h) (27 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

### Intermediate 17a):

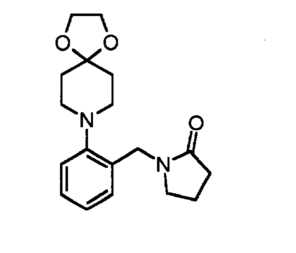

1,4-Dioxa-8-azaspiro[4.5]decane (688 mg), intermediate 1a) (1004 mg), Pd₂(dba)₃ (235 mg), BINAP (442 mg) and cesium carbonate (3 g) were mixed together in toluene (20 ml). The mixture was degassed and heated to 100°C for 3 d. The mixture was diluted with ether (100 ml) and filtered over Celite. The filtrate was concentrated and then subjected to chromatography on silica gel to yield the title compound.

### Intermediate 17b):

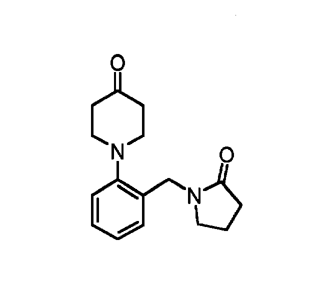

To the protected ketone from 17a) (407 mg) was added 5% HCl in THF (20 ml) and stirred overnight. The volatiles were removed under reduced pressure and the residue was dissolved in EtOAc. The solution was extracted with water and saturated sodium bicarbonate solution, dried over sodium sulfate and the solvent was evaporated to yield the title compound.

### Intermediate 17c):

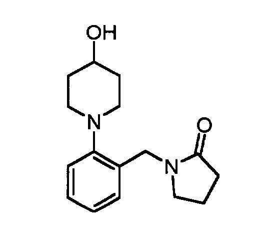

The ketone from 17b) (302 mg) was added portionwise to a solution of sodium borohydride (17 mg) in isopropanol (10 ml). The reaction mixture was stirred overnight and then quenched with 1 M HCl. Volatiles were removed under reduced pressure and the residue was taken up in EtOAc and extracted with 1 M NaOH, water and brine. The organic layer was dried over Na₂SO₄ and the solvent was removed in vacuo to yield the desired product.

### Intermediate 17d):

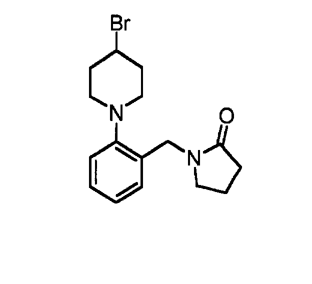

To a stirred solution of intermediate 17c) (253 mg) in DMF (1 ml) under argon at 0°C was added CBr₄ (289 mg). After 15 min PPh₃ (533 mg) was added in small portions over 10 min. The reaction was monitored by TLC. At the end of the reaction the mixture was poured in ice-water and carefully basified with saturated NaHCO₃. The reaction mixture was extracted three times with DCM. The combined organic layers were dried over Na₂SO₄ and the solvent was evaporated. The title compound was obtained after column chromatography.

### Intermediate 17e):

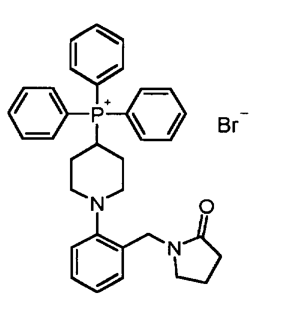

A solution of PPh₃ (499 mg) and intermediate 17d) (280 mg) in toluene (10 ml) was refluxed for 48 h. The precipitate was filtered off, washed with hot toluene and dried under reduced pressure.

### Intermediate 17f):

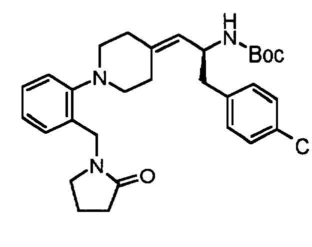

Sodium hydride (40 mg, 60% suspension in mineral oil) was washed three times with hexane and decanted. Dry DMSO (1 ml) was added and heated to 80°C until no more hydrogen was formed. The reaction mixture was cooled in an ice-bath and a solution of intermediate 17e) (600 mg) in dry DMSO (1 ml) was added. The solution was stirred for 10 min and Boc-4-chlorophenylalaninal (284 mg) was added. The reaction mixture was stirred until complete conversion was observed (monitoring by TLC) and then poured in water (5 ml) and extracted three times with EtOAc. The combined organic layers were washed with water and brine and dried over Na₂SO₄. The solvent was removed under reduced pressure. The crude product was purified by column chromatography.

### Intermediate 17g):

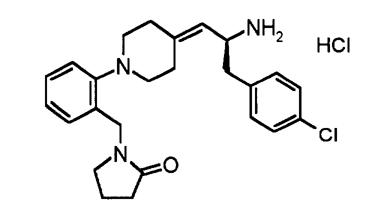

To the Boc-protected amine from 17f) (154 mg) in DCM (5 ml) was added TFA (1 ml) and stirred at room temperature for 90 min Additional TFA (1 ml) was added and stirred for 10 min The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄ and concentrated to give a white solid.

For prolonged storage, the free-base was converted into the corresponding hydrochloride. The free-base was dissolved in DCM (5 ml) and app. 1 M HCl in ether (10 ml) was added. The precipitate was filtered and the residue was washed three times with ether and dried under reduced pressure to yield the desired compound.

### Intermediate 17h):

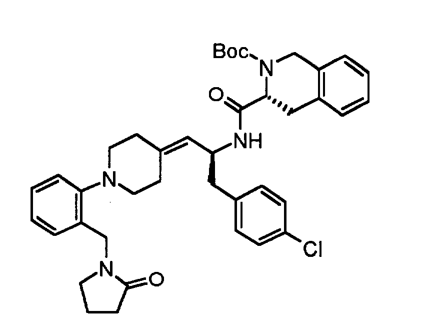

To (R)-Boc-1,2,3,4-tetrahydroisoquinoline-3-carboxylic acid (24 mg) in DCM (2 ml) was added intermediate 17g) (37 mg), N-methylmorpholine (14 µl) and HOBt (14 mg) and it was stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was purified by column chromatography.

The following examples can be prepared in a similar way:

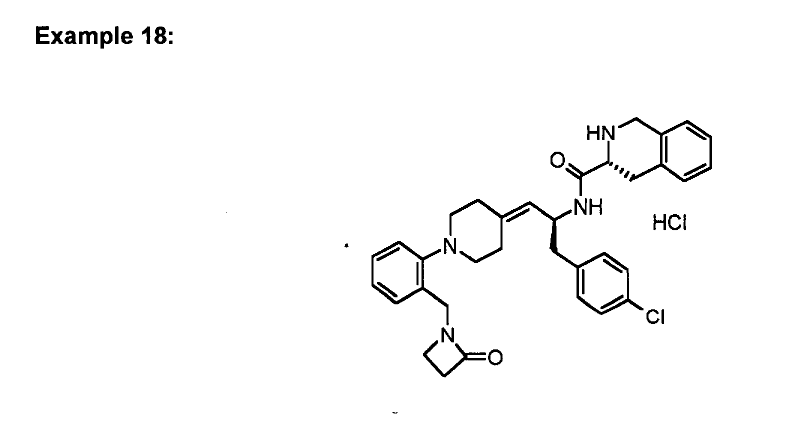

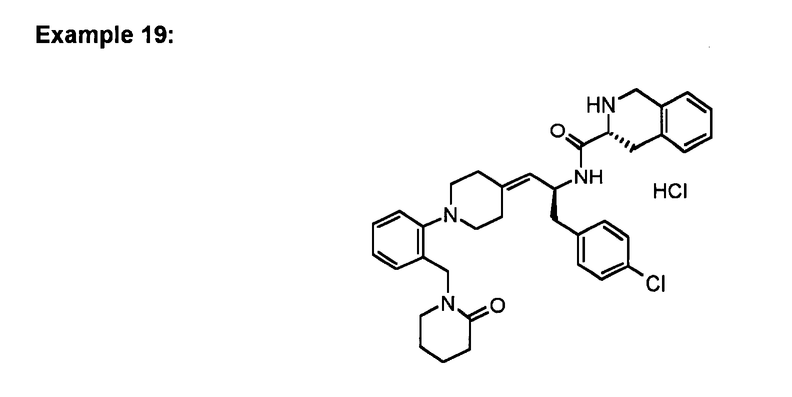

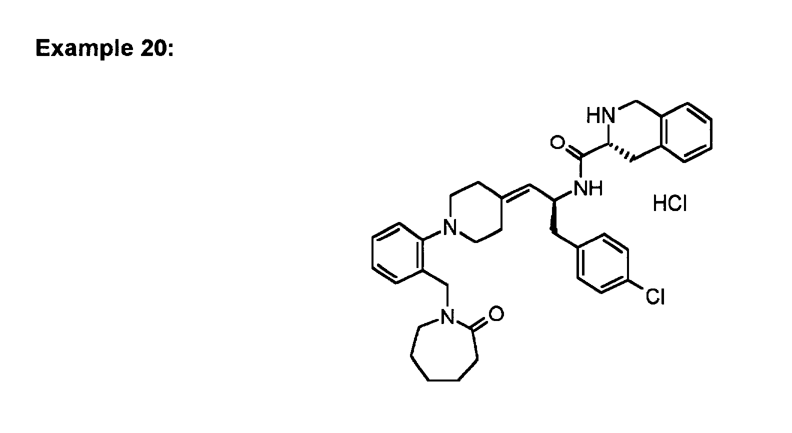

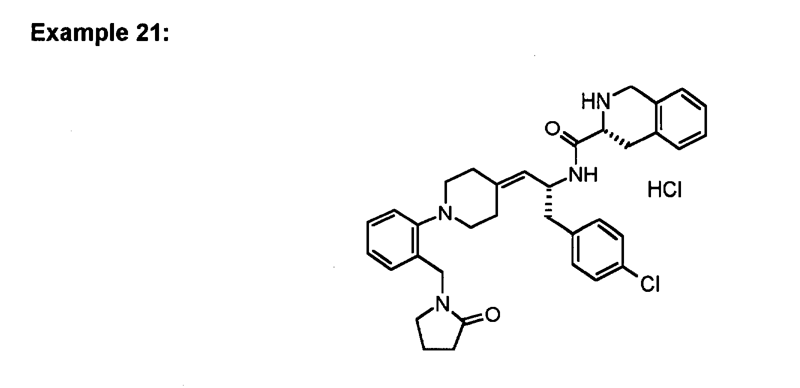

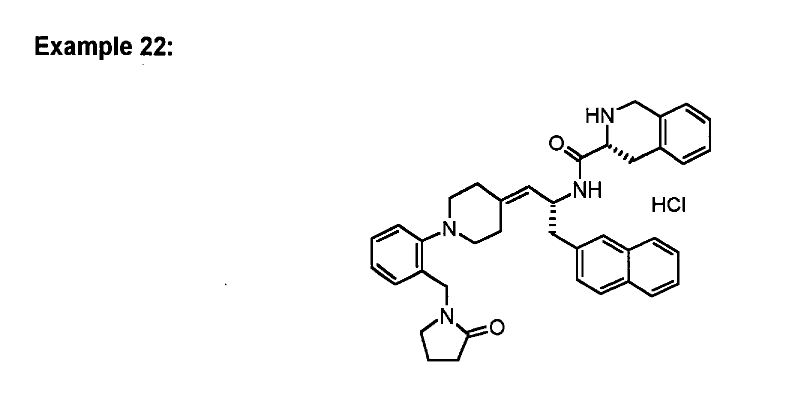

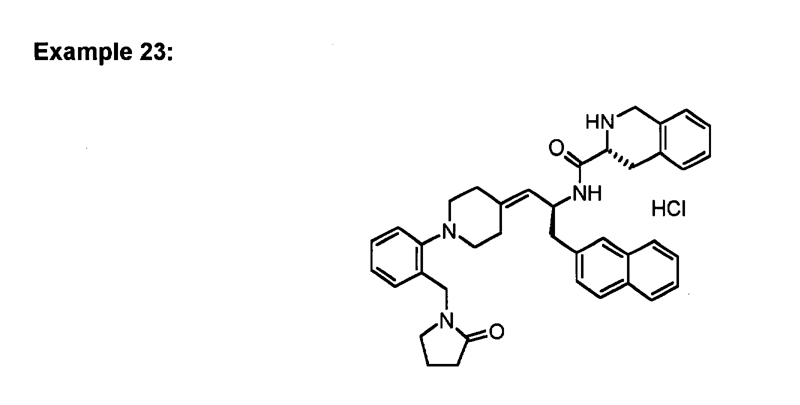

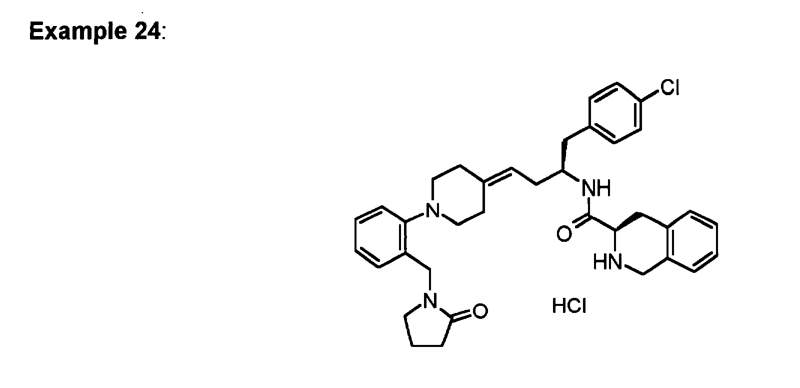

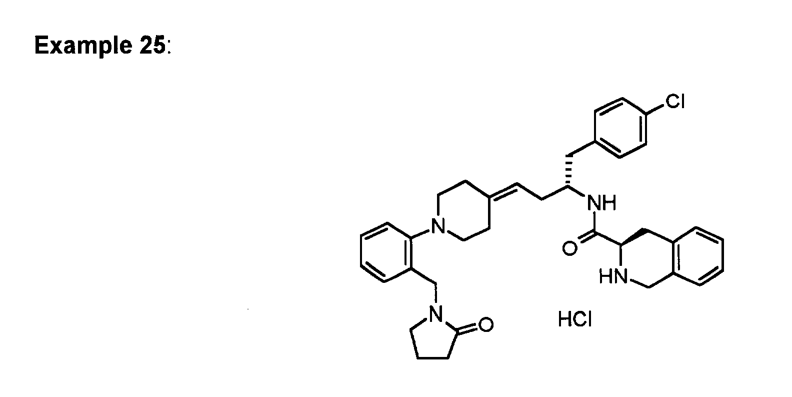

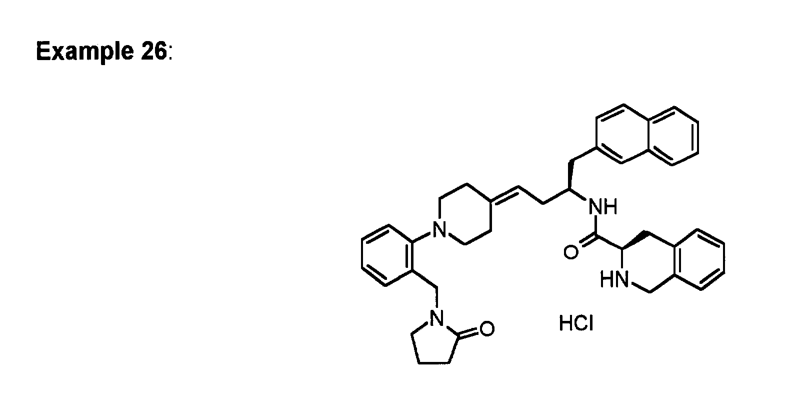

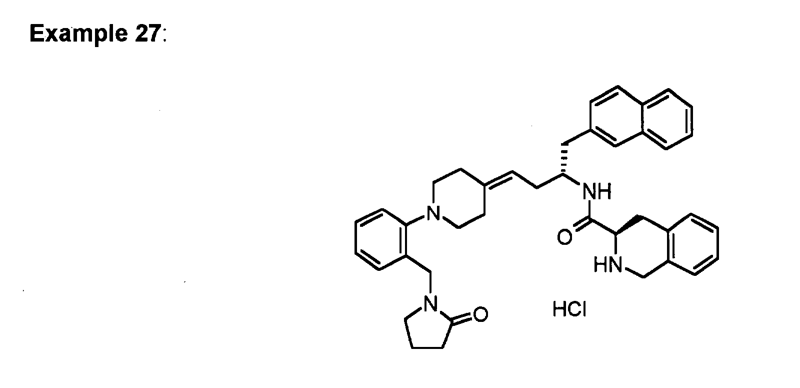

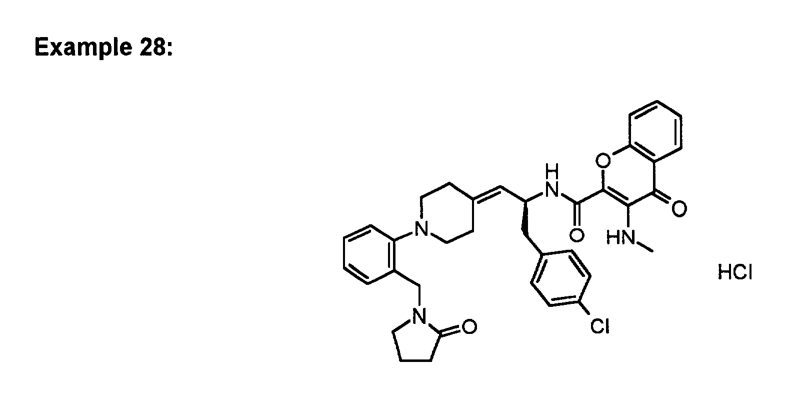

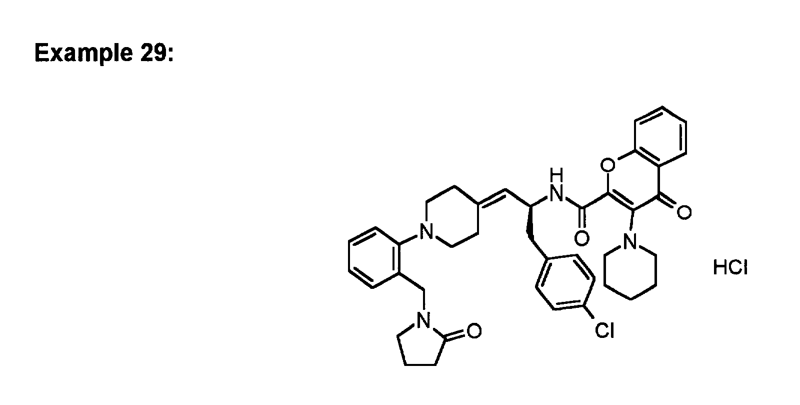

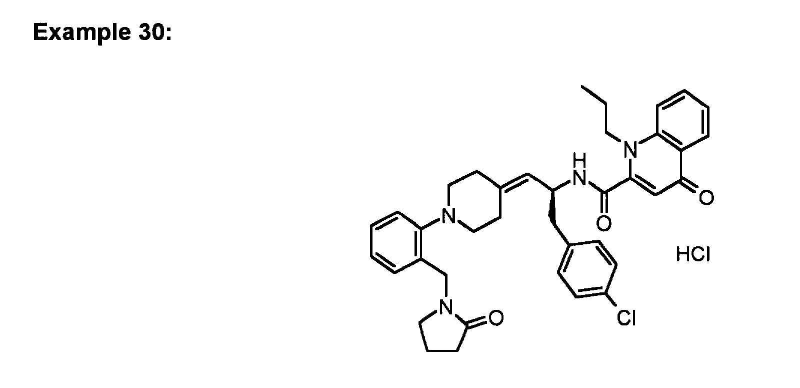

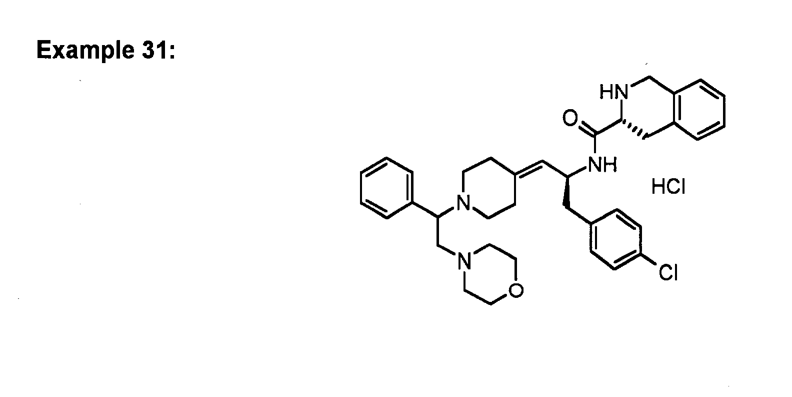

To Boc-protected intermediate 311) (32 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

### Intermediate 31a):

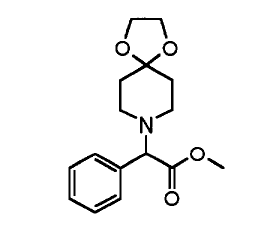

To a solution of methyl α-bromo phenylacetate (14.8 g) in MeCN (250 ml) was added K₂CO₃ (17.8 g), 1,4-dioxa-8-azaspiro[4.5]decane (10.2 g) and tetrabutylammonium iodide (0.2 g). The reaction mixture was allowed to reflux for 24 h. The reaction mixture was then diluted with EtOAc (800 ml) and washed with brine (800 ml). The organic phase was concentrated to dryness. The crude material was purified by column chromatography to yield the desired product.

### Intermediate 31b):

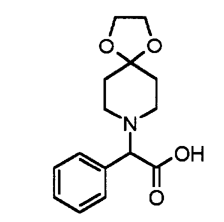

To intermediate 31 a) (15.4 g) in EtOH (100 ml) was added 1 M NaOH (117 ml) and the reaction mixture stirred for 18 h and concentrated to dryness. The residue was taken up in water and acidified slowly at 0°C with 10% NaHSO₄ (pH = 5-6). The desired product was then extracted into EtOAc. The organic phase was concentrated to dryness to yield the title compound.

### Intermediate 31c):

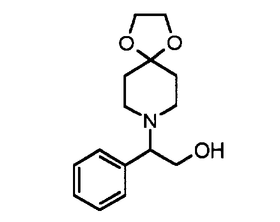

Intermediate 31b) (12.6 g) was dissolved in THF (160 ml). At 0°C BH₃-SMe₂ (8.65 ml) was slowly added. The reaction mixture was allowed to warm to room temperature and then stirred for 4 d, quenched with sat. NaHCO₃ (160 ml) at 0°C and diluted with Et₂O. The organic phase was dried over Na₂SO₄ and the solvent was evaporated under reduced pressure. The crude material was purified by column chromatography to yield the desired compound

### Intermediate 31d):

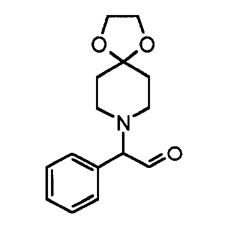

To DMSO (7.0 ml) in DCM (120 ml) at -78°C was added (COCl)₂ (4.15 ml) dropwise. The reaction mixture was allowed to stir at -78°C for 15 min and then intermediate 31c) (10.3 g) in DCM (24 ml) was added dropwise. The reaction mixture was stirred at -78°C for 1 h and then TEA (27.3 ml) was added followed by stirring for 3 h at -78°C. The reaction mixture was warmed up to room temperature and after stirring overnight it was quenched with sat. NH₄Cl. The aqueous phase was extracted with EtOAc. The combined organic phases were dried over Na₂SO₄ and concentrated in vacuo. The crude product was purified by column chromatography to yield the title compound.

### Intermediate 31e):

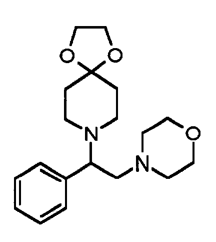

To intermediate 31 d) (6.4 g) in DCM (80 ml) was added morpholine (2.1 ml) and sodium triacetoxyborohydnde (7.3 g). The reaction was stirred overnight and then quenched with 5 M NaOH (89 ml) and diluted with EtOAc. The organic phase was dried over Na₂SO₄ and concentrated. The crude material was purified by column chromatography to yield the desired product.

### Intermediate 31f):

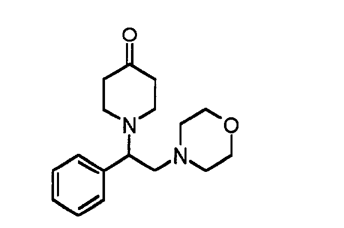

To the protected ketone from 31e) (4.0 g) was added 5% HCl in THF (100 ml) and stirred overnight. The volatiles were removed under reduced pressure and the residue was dissolved in EtOAc. The solution was extracted with water and saturated sodium bicarbonate solution, dried over sodium sulfate and the solvent was evaporated to yield the title compound.

### Intermediate 31g):

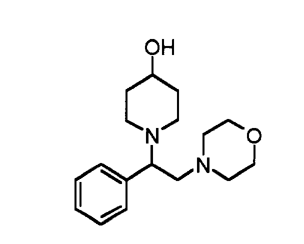

The ketone from 31f) (320 mg) was added portionwise to a solution of sodium borohydride (17 mg) in isopropanol (10 ml). The reaction mixture was stirred overnight and then quenched with 1 M HCl. Volatiles were removed under reduced pressure and the residue was taken up in EtOAc and extracted with 1 M NaOH, water and brine. The organic layer was dried over Na₂SO₄ and the solvent was removed in vacuo to yield the desired product

### Intermediate 31h):

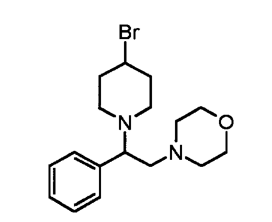

To a stirred solution of intermediate 31g) (261 mg) in DMF (1 ml) under argon at 0°C was added CBr₄ (289 mg). After 15 min PPh₃ (533 mg) was added in small portions over 10 min. The reaction was monitored by TLC. At the end of the reaction the mixture was poured in ice-water and carefully basified with saturated NaHCO₃. The reaction mixture was extracted three times with DCM. The combined organic layers were dried over Na₂SO₄ and the solvent was evaporated The title compound was obtained after column chromatography.

### Intermediate 31i):

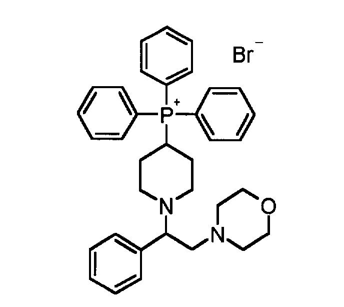

A solution of PPh₃ (499 mg) and intermediate 31h) (283 mg) in toluene (10 ml) was refluxed for 48 h. The precipitate was filtered off, washed with hot toluene and dried under reduced pressure.

### Intermediate 31j):

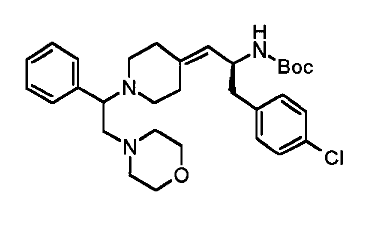

Sodium hydride (40 mg, 60% suspension in mineral oil) was washed three times with hexane and decanted. Dry DMSO (1 ml) was added and heated to 80°C until no more hydrogen was formed. The reaction mixture was cooled in an ice-bath and a solution of intermediate 31i) (616 mg) in dry DMSO (1 ml) was added. The solution was stirred for 10 min and Boc-4-chlorophenylalaninal (284 mg) was added. The reaction mixture was stirred until complete conversion was observed (monitoring by TLC) and poured in water (5 ml) and extracted three times with EtOAc. The combined organic layers were washed with water and brine and dried over Na₂SO₄. The solvent was removed under reduced pressure. The crude product was purified by column chromatography.

### Intermediate 31k):

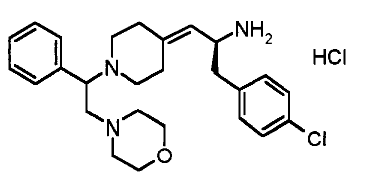

To the Boc-protected amine from 31j) (148 mg) in DCM (5 ml) was added TFA (1 ml) and it was stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and bnne, dried over Na₂SO₄ and concentrated to give a white solid.

For prolonged storage, the free-base was converted into the corresponding hydrochloride. The free-base was dissolved in DCM (5 ml) and app. 1 M HCl in ether (10 ml) was added. The precipitate was filtered and the residue was washed three times with ether and dned under reduced pressure to yield the desired compound.

### Intermediate 31l):

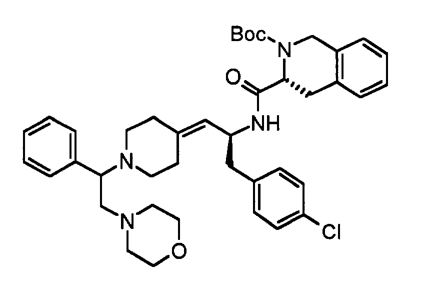

To (R)-Boc-1,2,3,4-tetrahydroisoquinoline-3-carboxylic acid (24 mg) in DCM (2 ml) was added intermediate 31k) (38 mg), N-methylmorpholine (14 µl) and HOBt (14 mg) and it was stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was purified by column chromatography.

The following examples can be prepared in a similar way:

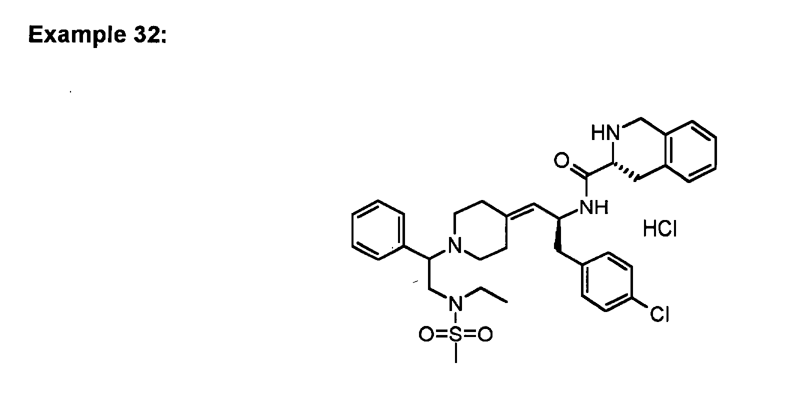

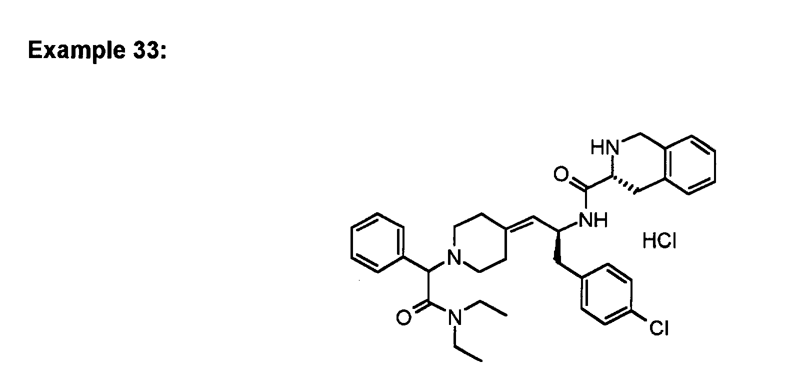

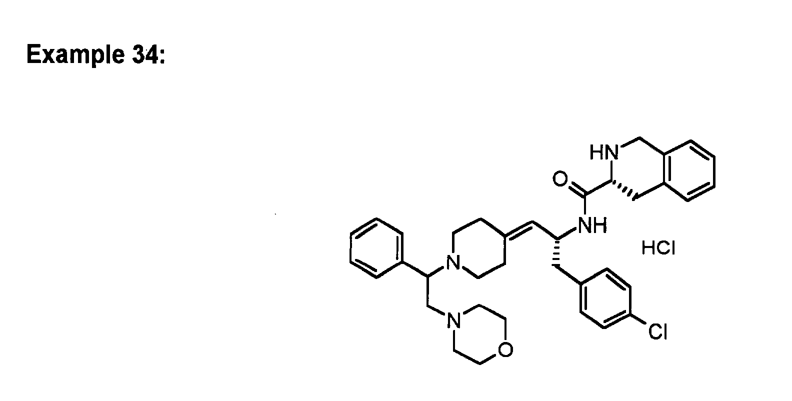

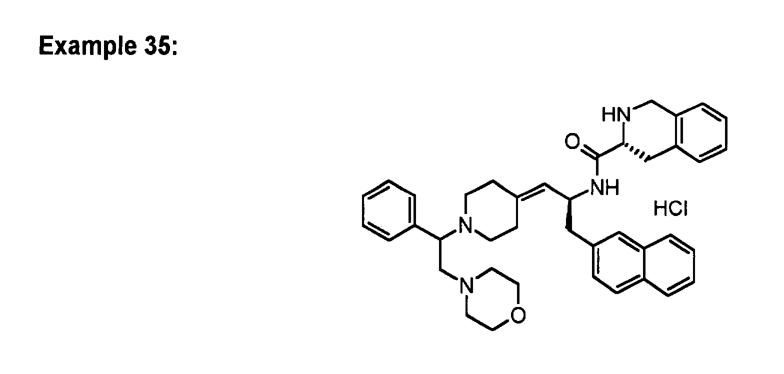

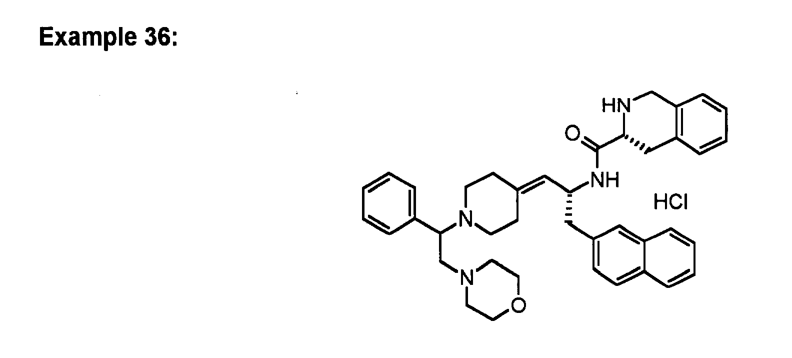

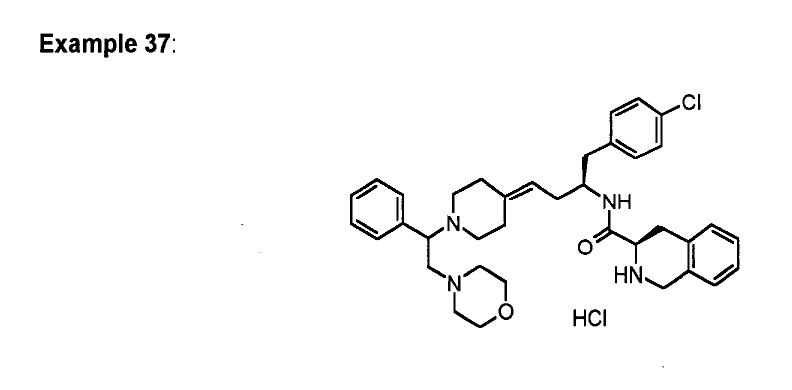

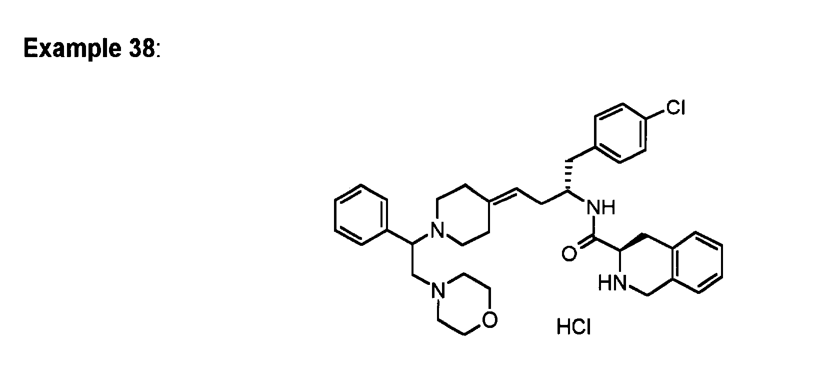

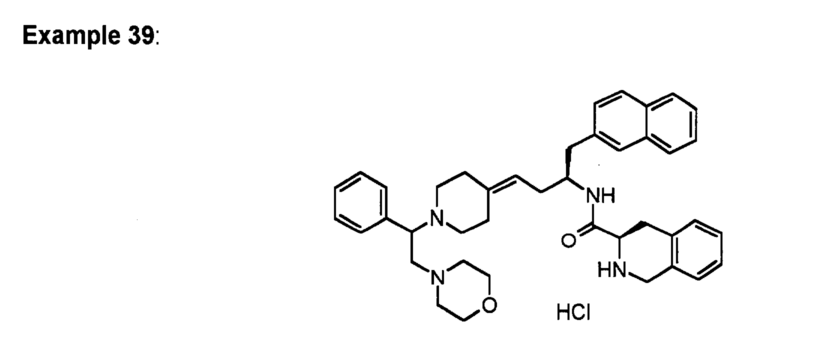

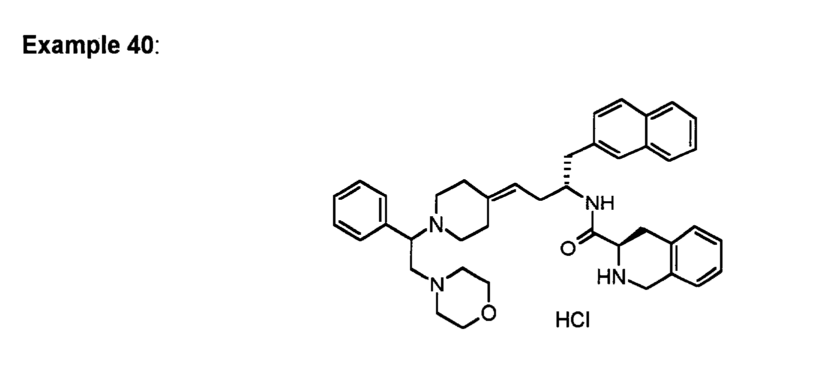

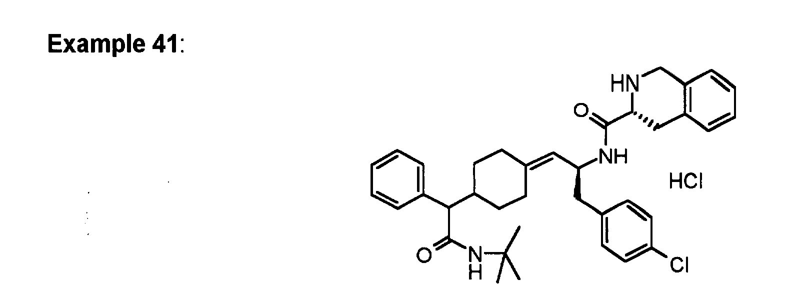

To Boc-protected intermediate 41k) (34 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.

### Intermediate 41a):

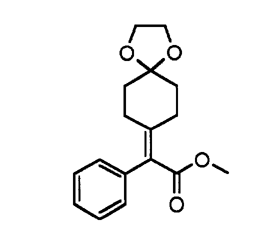

To a solution of methyl phenylacetate (21.9 ml) in ether (100 ml) was added 30 wt% sodium methoxide in methanol (27 ml) at 10 - 15°C and stirred for 30 min at room temperature. A solution of 1,4-dioxaspiro[4.5]decan-8-one (23.4 g) in ether (100 ml) was added dropwise at 10 - 15°C. The reaction mixture was stirred overnight and neutralized with glacial acetic acid and the solvent was evaporated. The residue was taken up in EtOAc and extracted with water and sat. NaHCO₃ and dried over Na₂SO₄ The solvent was evaporated and the crude material was purified by column chromatography to yield the desired compound.

### Intermediate 41b):

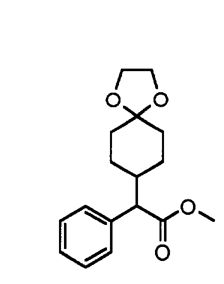

To a solution of intermediate 41a) (17.3 g) in methanol (250 ml) was added Pt₂O (136 mg). The solution was stirred under hydrogen (5 bar) overnight and filtered. The solvent was evaporated to yield the title compound.

### Intermediate 41c):

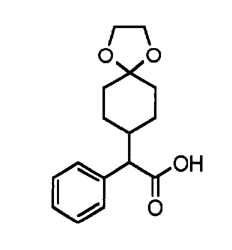

To intermediate 41b) (15.3 g) in EtOH (100 ml) was added 1 M NaOH (117 ml) and the reaction mixture stirred for 18 h and concentrated to dryness. The residue was taken up in water and acified slowly at 0°C with 10% NaHSO₄ (pH = 4-5). The desired product was then extracted into EtOAc. The organic phase was concentrated to dryness to yield the title compound. *Intermediate 41d):*

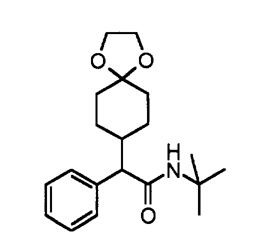

Intermediate 41c) (5.5 g) was dissolved in dry DCM (100 ml) and cooled to 0°C in an ice-water bath. Oxalyl chloride (1.9 ml) in DCM (10 ml) was added dropwise followed by the addition of 4 - 5 drops of DMF. This mixture was stirred at 0°C for 2.5 h and then concentrated with toluene. The residue was dissolved in dry DCM (100 ml) and cooled at 0°C in an ice-water bath. Tert-Butylamine (6.3 ml) was then added dropwise and the reaction mixture was stirred at 0°C for 2 h, warmed to room temperature and stirred at room temperature overnight. The resulting mixture was diluted with DCM, washed with bnne, dried over Na₂SO₄ and concentrated. The crude material was purified by column chromatography to yield the title compound.

### Intermediate 41e):

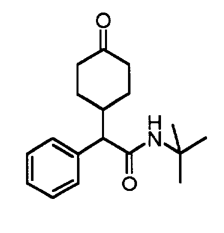

To the protected ketone from 41d) (3.9 g) was added 5% HCl in THF (100 ml) and stirred overnight. The volatiles were removed under reduced pressure and the residue was dissolved in EtOAc. The solution was extracted with water and saturated sodium bicarbonate solution, dried over sodium sulfate and the solvent was evaporated to yield the title compound. *Intermediate 41f)*:

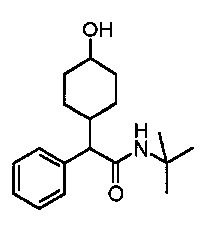

The ketone from 41 e) (319 mg) was added portionwise to a solution of sodium (17 mg) in isopropanol (10 ml). The reaction mixture was stirred overnight and then quenched with 1 M HCl. Volatiles were removed under reduced pressure and the residue was taken up in EtOAc and extracted with 1 M NaOH, water and brine. The organic layer was dried over Na₂SO₄ and the solvent was removed in vacuo to yield the desired product

### Intermediate 41g):

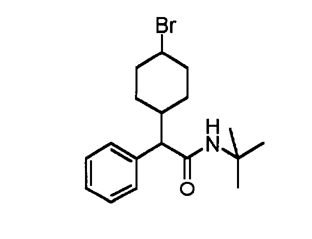

To a stirred solution of intermediate 41f) (260 mg) in DMF (1 ml) under argon at 0°C was added CBr₄ (289 mg). After 15 min PPh₃ (533 mg) was added in small portions over 10 min. The reaction was monitored by TLC. At the end of the reaction the mixture was poured into ice-water and carefully basified with saturated NaHCO₃. The reaction mixture was extracted three times with DCM. The combined organic layers were dried over Na₂SO₄ and the solvent was evaporated. The title compound was obtained after column chromatography.

### Intermediate 41h):

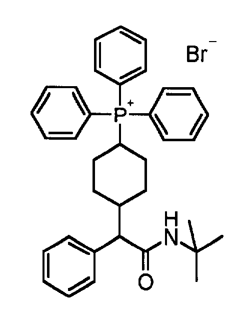

A solution of PPh₃ (499 mg) and intermediate 41g) (282 mg) in toluene (10 ml) was refluxed for 48 h. The precipitate was filtered off, washed with hot toluene and dried under reduced pressure.

### Intermediate 41i):

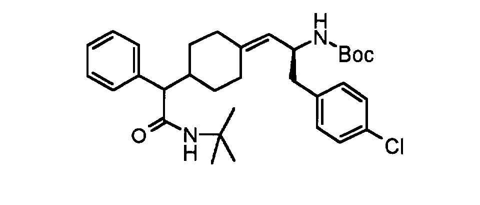

Sodium hydride (40 mg, 60% suspension in mineral oil) was washed three times with hexane and decanted. Dry DMSO (1 ml) was added and heated to 80°C until no more hydrogen was formed. The reaction mixture was cooled in an ice-bath and a solution of intermediate 41h) (615 mg) in dry DMSO (1 ml) was added. The solution was stirred for 10 min and Boc-4-chlorophenylalaninal (284 mg) was added. The reaction mixture was stirred until complete conversion was observed (monitoring by TLC), poured into water (5 ml) and extracted three times with EtOAc. The combined organic fractions were washed with water and brine and dried over Na₂SO₄. The solvent was removed under reduced pressure. The crude product was purified by column chromatography.

### Intermediate 41j):

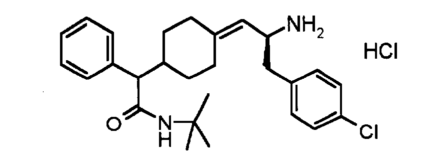

To the Boc-protected amine from 41i) (148 mg) in DCM (5 ml) was added TFA (1 ml) and then stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml) The organic layer was separated and the aqueous layer was further extracted three times with DCM. The combined organics were washed with water and brine, dned over Na₂SO₄ and concentrated to give a white solid.

For prolonged storage, the free-base was converted into the corresponding hydrochloride. The free-base was dissolved in DCM (5 ml) and app. 1 M HCl in ether (10 ml) was added. The precipitate was filtered and the residue was washed three times with ether and dried under reduced pressure to yield the desired compound.

### Intermediate 41k):

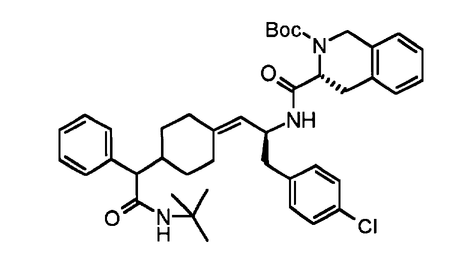

To (R)-Boc-1,2,3,4-tetrahydroisoquinoline-3-carboxylic acid (24 mg) in DCM (2 ml) was added intermediate 41j) (38 mg), N-methylmorpholine (14 µl) and HOBt (14 mg) and then stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was purified by column chromatography

The following examples can be prepared in a similar way:

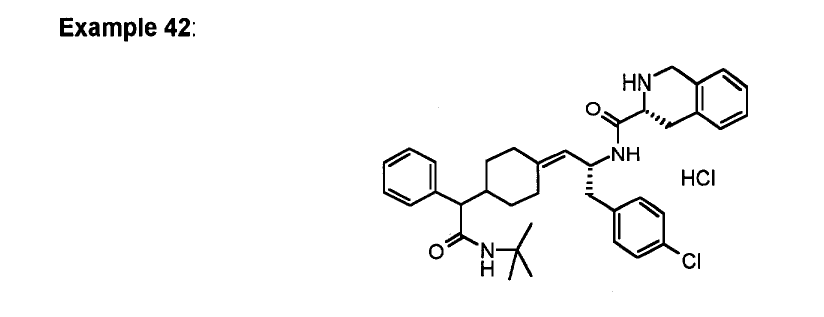

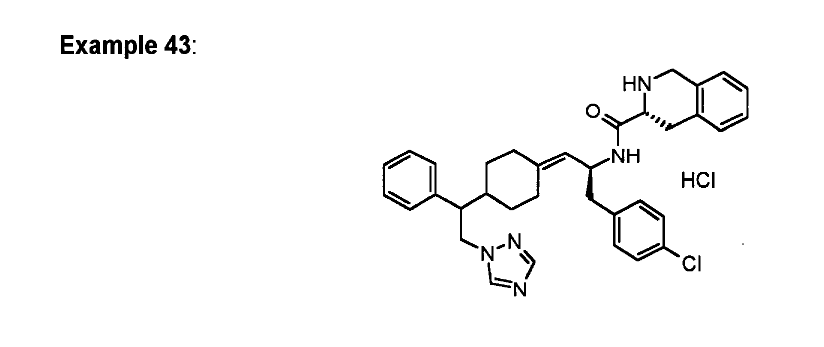

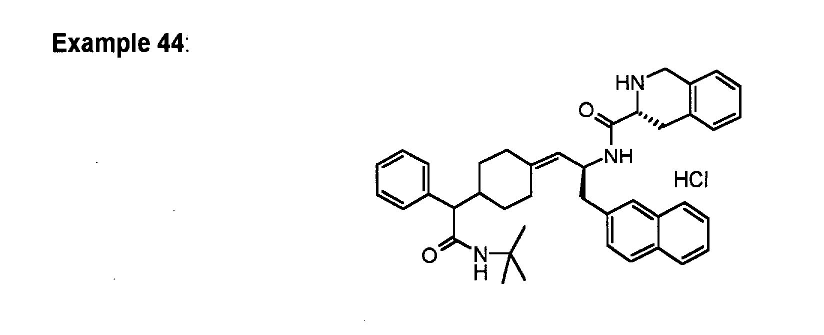

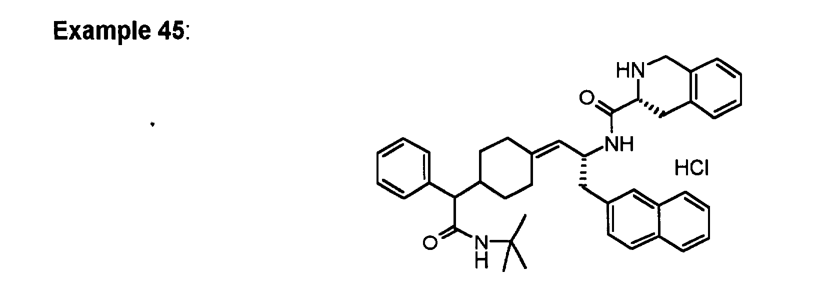

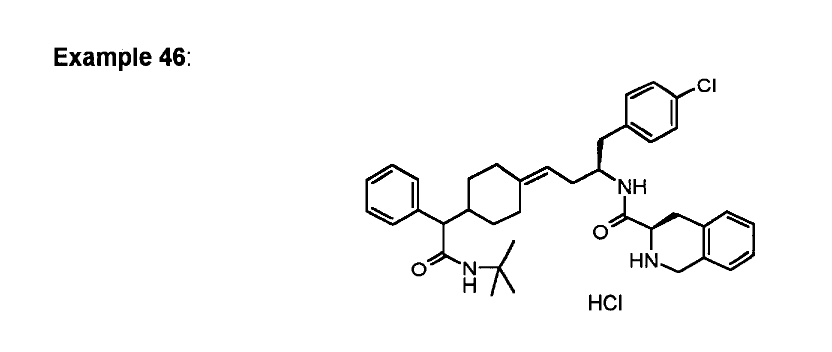

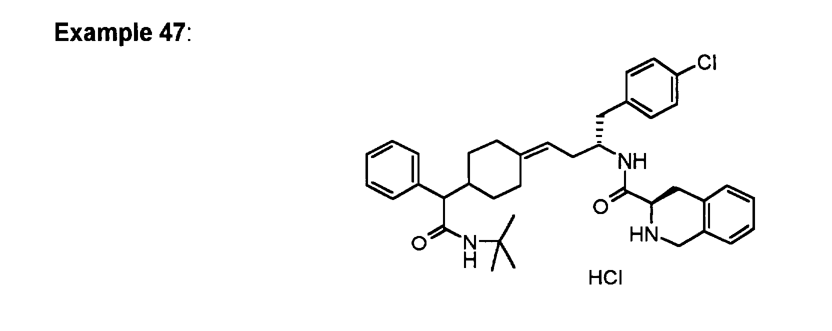

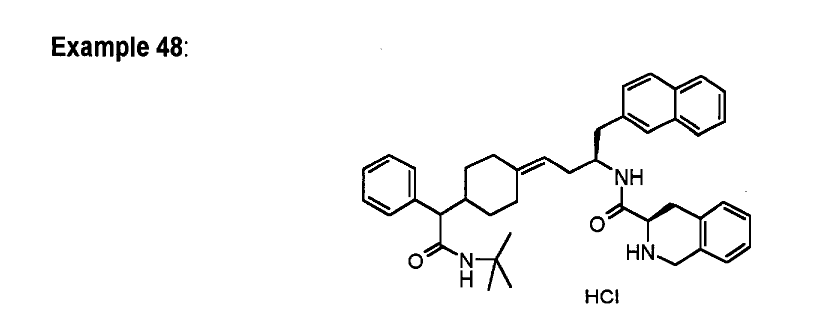

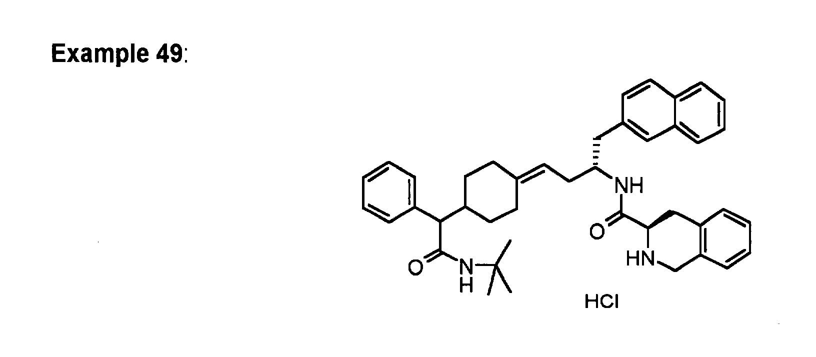

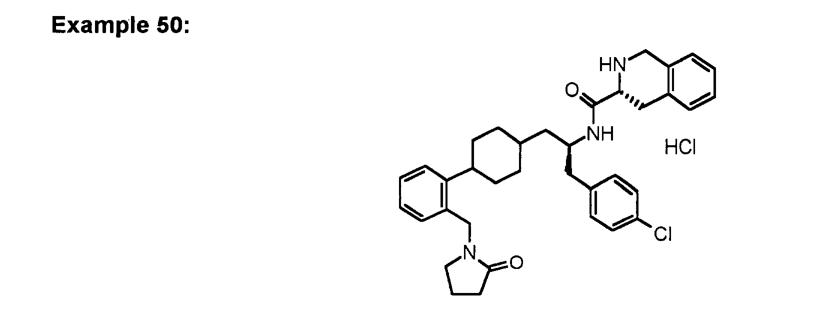

To a solution of example 1 (40 mg) in methanol (3 ml) was added Pt₂O (1 mg). The solution was stirred under hydrogen (1 bar) overnight and filtered. The solvent was evaporated to yield the title compound.

The following examples can be prepared in a similar way:

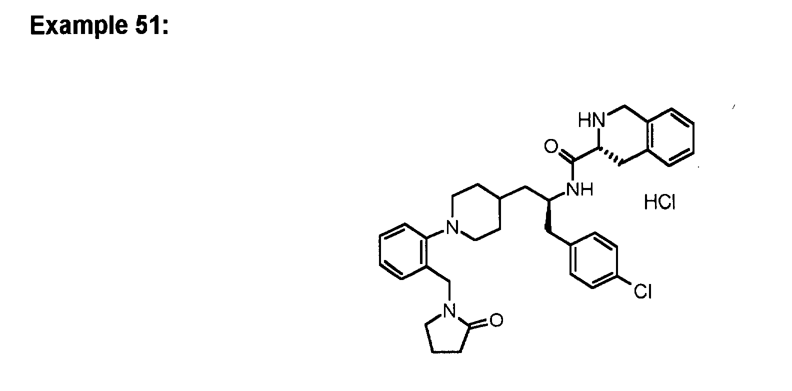

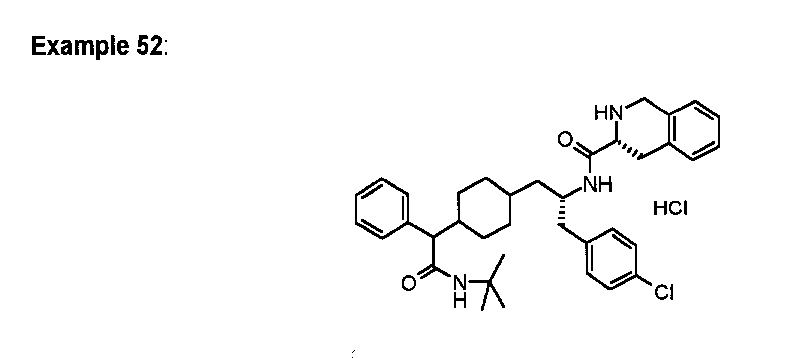

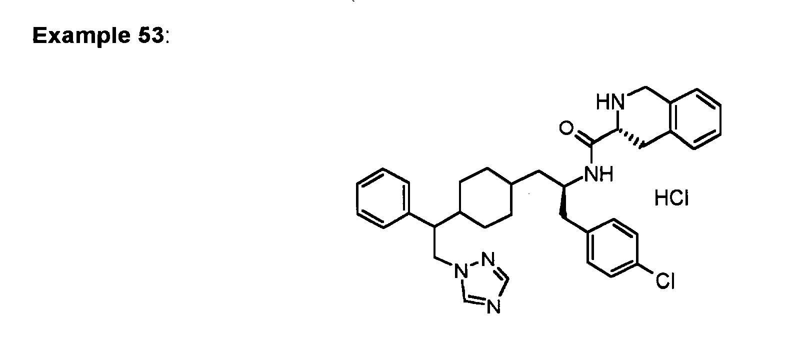

### Preparation of the carboxylic acids:

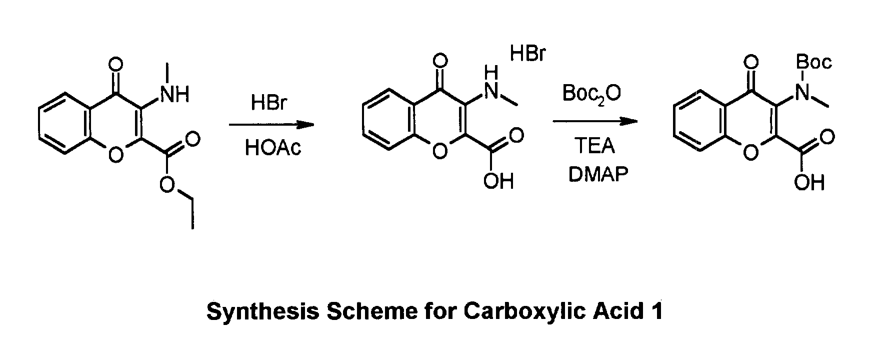

### Carboxylic Acid 1:

To a solution of intermediate CA1a) (0.4 g) in THF (10 ml) was added TEA (670 µl), DMAP (20 mg) and Boc₂O (384 mg). The reaction mixture was stirred overnight. The solvent was removed under reduced pressure and the residue was dissolved in ethyl acetate. The solution was extracted with water, 1 M HCl and sat. NaHCO₃ and dned over Na₂SO₄. The solvent was removed under reduced pressure to yield the title compound.

### Intermediate CA1a):

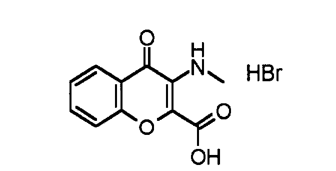

Ethyl 3-methylamino-4-oxochromene-2-carboxylate (0 8 g) was hydrolyzed by heating with hydrobromic acid (4 ml) and acetic acid (3 ml) for 4 h to give the desired compound after evaporation of the solvent

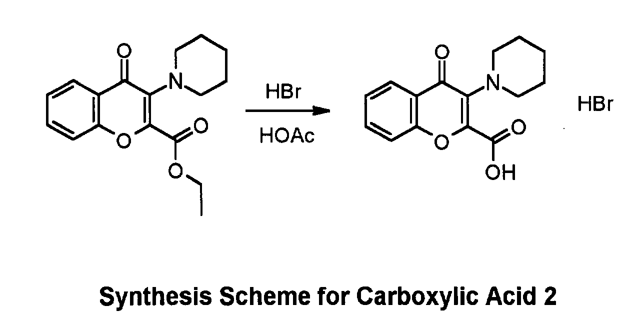

### Carboxylic Acid 2:

Ethyl 3-piperidino-4-oxochromene-2-carboxylate (0.8 g) was hydrolyzed by heating with hydrobromic acid (4 ml) and acetic acid (3 ml) for 4 h to give the desired compound after evaporation of the solvent.

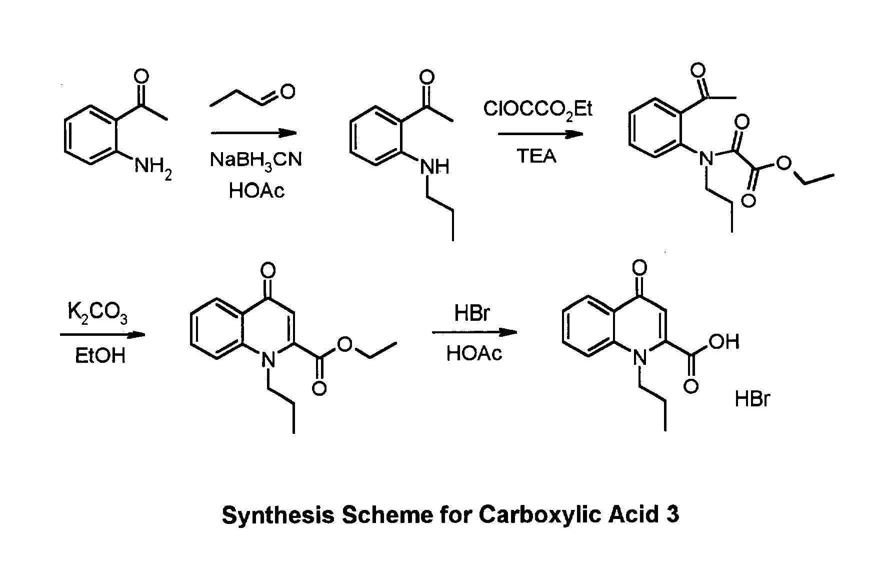

### Carboxylic Acid 3:

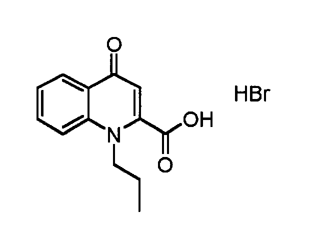

Intermediate CA3c) (0.1 g) was hydrolyzed by heating with hydrobromic acid (2 ml) and acetic acid (1.5 ml) for 3 h to give the desired compound after evaporation of the solvent.

### Intermediate CA3a):

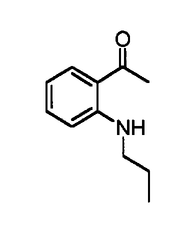

To a solution of 2'-aminoacetophenone (709 mg) in methanol (10 ml) was added propionaldehyde (580 µl) and TMOF (482 µl) and the solution was stirred overnight. The volatiles were removed under reduced pressure and the residue was redissolved in methanol (10 ml). Acetic acid (115 µl) and sodium cyanoborohydride (126 mg) were added and the reaction was stirred overnight. After basification with 1 M NaOH the volatiles were removed under reduced pressure. The residue was dissolved in ethyl acetate and washed with water and brine. The organic phase was dried over Na₂SO₄ and the solvent was removed under reduced pressure to yield the title compound.

### Intermediate CA3b):

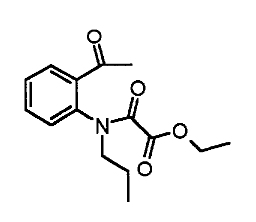

To a solution of intermediate CA3a) (681 mg) in dry THF (10 ml) was added TEA (670 µl) and the mixture was cooled to 0°C. At this temperature ethyl oxalyl chloride (470 µl) was added dropwise. The reaction mixture was stirred for 4 h at room temperature. The volatiles were removed under reduced pressure and the residue was dissolved in ethyl acetate. The solution was washed with water, sat. NaHCO₃ and brine and dried over Na₂SO₄ to yield the desired compound

### Intermediate CA3c):

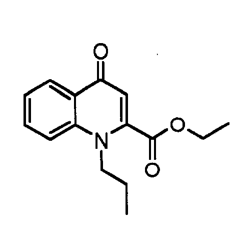

Intermediate CA3b) (555 mg) was dissolved in ethanol (10 ml). K₂CO₃ (276 mg) was added and the reaction mixture was stirred overnight. The reaction mixture was filtrated and the solvent was removed to yield the title compound.

### BIOLOGICAL ASSAYS

### A. Binding Assay

A membrane binding assay is used to identify competitive inhibitors of fluorescence labeled NDP-alpha-MSH binding to HEK293 cell membrane preparations expressing human melanocortin receptors.

The test compound, or unlabeled NDP-alpha-MSH, is dispensed at varying concentrations to a 384 well microtiter plate. Fluorescence labeled NDP-alpha-MSH is dispensed at a single concentration, followed by addition of membrane preparations. The plate is incubated for 60 to 90 min at room temperature.

The degree of fluorescence polarization is determined with a fluorescence polarization microplate reader

### B. Functional Assay

A functional cellular assay, based on competition between unlabeled cAMP and a fixed quantity of fluorescence labeled cAMP for a limited number of binding sites on a cAMP specific antibody, is used to discriminate melanocortin receptor agonists from antagonists by fluorescence polarization.

HEK293 cells, expressing one of the human melanocortin receptors, are grown in 384 well microtiter plates and are stimulated at different concentrations of the test compound to effect cAMP production. Cells are lysed and a fluorescence labeled cAMP conjugate is dispensed, followed by the addition of anti-cAMP antibody used to detect the produced cAMP. The plate is read on a fluorescence polarization microplate reader and the amount of cAMP produced as a response to a test compound is compared to the production of cAMP resulting from stimulation with NDP-alpha-MSH.

The ability of a compound to block CAMP production in response to NDP-alpha-MSH is measured to define antagonistic activity of a test compound. Percent inhibition is determined by comparing the amount of CAMP produced in the presence to that produced in the absence of test compound.

### C. In Vivo Food Intake Models

### 1. Spontaneous Feeding Paradigm

Food intake in rats is measured after i p. or p o. administration of the test compound (see e g. Chen, A.S. et al. Transgenic Res 2000 Apr;9(2):145-54).

### 2. Model of LPS and Tumor-Induced Cachexia

Prevention or amelioration of cachexia, induced by either lipopolysaccharide (LPS) administration or by tumor growth, is determined upon i.p. or p.o. administration of test compounds to rats (see e.g. Marks, D.L.; Ling, N and Cone, R.D. Cancer Res 2001 Feb 15;61(4):1432-8).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes approximately 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted, until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and/or vehicle evaluations are conducted to determine how, and if, an animal will respond. Some animals have a long duration until the first response while others are non-responders altogether During this baseline evaluation, latency to first response and number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired, 2 tailed t-tests to compared baseline and/or vehicle evaluations to drug treated evaluations for individual animals. Groups of a minimum of 4 animals are utilized to reduce variability

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

Representative compounds of the present invention were tested and found to bind to the melanocortin-4 receptor. These compounds were generally found to have IC50 values less than 2 µM. Representative compounds of the present invention were also tested in the functional assay and found generally to activate the melanocortin-4 receptor with EC50 values less than 1 µM.

### Examples of a Pharmaceutical Composition

As a specific embodiment of an oral composition of a compound of the present invention, 20 mg of Example 1 is formulated with sufficient finely divided lactose to provide a total amount of 580 to 590 mg to fill a size 0 hard gelatin capsule.

As another specific embodiment of an oral composition of a compound of the present invention, 50 mg of Example 22 is formulated with sufficient finely divided lactose to provide a total amount of 580 to 590 mg to fill a size 0 hard gelatin capsule.

As another specific embodiment of an oral composition of a compound of the present invention, 30 mg of Example 43 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions, can be made therein without departing from the spirit and scope of the invention. For example, effective dosages other than the preferred doses as set out above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of structural formula (I): 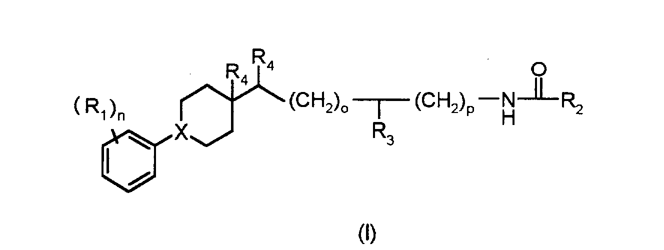 or a pharmaceutically acceptable salt or a solvate thereof, wherein
R₁ is independently:
hydrogen,
hydroxy,
cyano,
nitro,
halo,
alkyl,
alkoxy,
haloalkyl,
(D)-C(O)R₁₅,
(D)-C(O)OR₁₅,
(D)-C(O)SR₁₅,
(D)-C(O)-heteroaryl,
(D)-C(O)-heterocyclyl,
(D)-C(O)N(R₁₅)₂,
(D)-N(R₁₅)₂,
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅,
(D)-NR₁₅SO₂N(R₁₅)₂,
(D)-NR₁₅(D)-heterocyclyl,
(D)-NR₁₅(D)-heteroaryl,
(D)-OR₁₅,
OSO₂R₁₅,
(D)-[O]ᵥ(C₃ - C₇ cycloalkyl),
(D)-[O]ᵥ(D)aryl,
(D)-[O]ᵥ(D)-heteroary),
(D)-[O]ᵥ(D)-heterocyclyl (wherein heterocyclyl excludes a heterocyclyl containing a single nitrogen when v=1),
(D)-SR₁₅,
(D)-SOR₁₅,
(D)-SO₂R₁₅ or
(D)-SO₂N(R₁₅)₂,
wherein alkyl, alkoxy, cycloalkyl, aryl, heterocyclyl and heteroaryl are unsubstituted or substituted;
R₂ is: 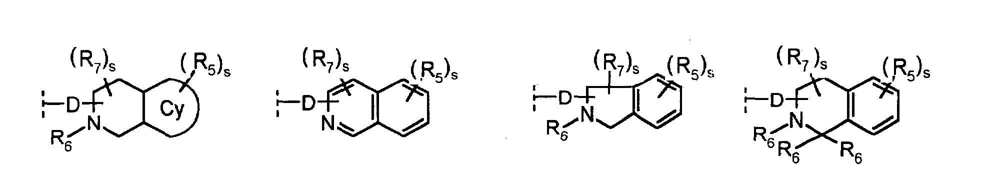 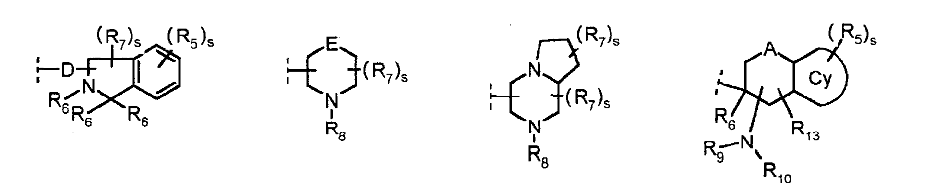 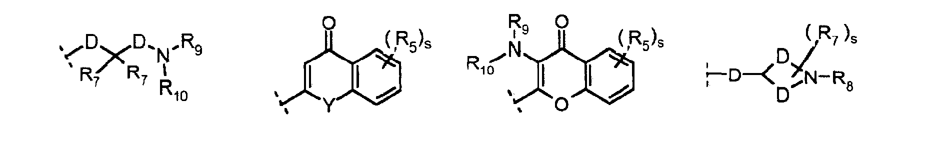
R₃ is independently:
(D)-aryl or
(D)-heteroaryl,
wherein aryl and heteroaryl are unsubstituted or substituted;
R₄ is H or a bond;
each R₅ is independently:
hydrogen,
halo,
alkyl,
haloalkyl,
(D)-cycloalkyl or
(D)-aryl (wherein aryl is phenyl or naphthyl),
(D)-heteroaryl or
(D)-heterocyclyl (wherein heterocyclyl excludes a heterocyclyl containing a single nitrogen), and
wherein aryl, heteroaryl, heterocyclyl, alkyl or cycloalkyl is unsubstituted or substituted
each R₆ is independently:
hydrogen,
alkyl,
C(O)-alkyl,
(D)-aryl or
cycloalkyl;
each R₇ is independently:
hydrogen,
alkyl,
(D)-aryl,
(D)-heteroaryl,
(D)-N(R₉)₂,
(D)-NR₉C(O) alkyl,
(D)-NR₉SO₂ alkyl,
(D)-SO₂N(R₉)₂,
(D)-(O)ᵣ alkyl,
(D)-(O)ᵣ(D)-NR₉COR₉,
(D)-(O)ᵣ(D)-NR₉SO₂R₉,
(D)-(O)ᵣ-heterocyclyl or
(D)-(O)ᵣ(alkyl)-heterocyclyl; and
each R₈ is independently:
hydrogen,
alkyl,
(D)-aryl,
C(O) alkyl,
C(O)-aryl,
SO₂-alkyl or
SO₂-aryl;
R₉ and R₁₀ are each independently:
hydrogen,
alkyl or
cycloalkyl, or
R₉ and R₁₀ together with the nitrogen to which they are attached form a 5-to 8-membered ring optionally containing an additional heteroatom selected from O, S and NR₆,
wherein alkyl and cycloalkyl are unsubstituted or substituted;
R₁₃ is:
hydrogen or
alkyl;
each R₁₅ is independently:
hydrogen,
alkyl,
haloalkyl,
(D)-cycloalkyl,
(D)-aryl (wherein aryl is phenyl or naphthyl),
(D)-heteroaryl or
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
Cy is:
aryl,
5- or 6-membered heteroaryl,
5- or 6-membered heterocyclyl or
5- or 7-membered carbocyclyl;
A is a bond, O, S(O)ᵤ, NR₈ or CH₂;
D is a bond or alkylene;
E is O, S or NR₈;
X is N or CH;
Y is O or NR₉;
n is 1 - 4;
o is 0 - 2;
p is 0 - 2;
r is 0 or 1;
s is 0 - 5;
u is 0 - 2;
v is 0 or 1.

2. The compound of claim 1, wherein
each R₁ is independently:
hydrogen,
hydroxy,
cyano,
nitro,
halo,
alkyl,
alkoxy,
haloalkyl,
(D)-N(R₁₅)₂,
(D)-NR₁₅COR₁₅,
(D)-NR₁₅CON(R₁₅)₂,
(D)-NR₁₅C(O)OR₁₅,
(D)-NR₁₅C(R₁₅)=N(R₁₅),
(D)-NR₁₅C(=NR₁₅)N(R₁₅)₂,
(D)-NR₁₅SO₂R₁₅,
(D)-NR₁₅SO₂N(R₁₅)₂ or
(D)-heterocyclyl;
R₂ is: 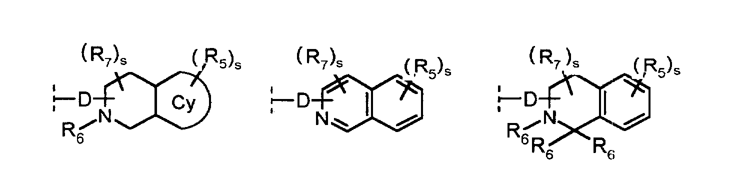 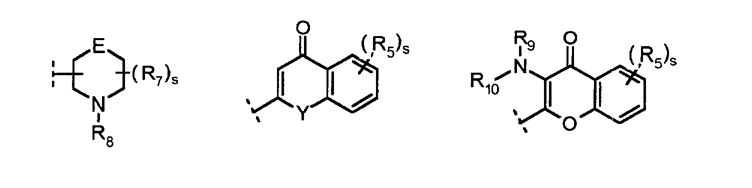
R₃ is (CH₂)-aryl, unsubstituted or substituted with one to three substituents selected from the group consisting of cyano, nitro, perfluoroalkoxy, halo, alkyl, (D)-cycloalkyl, alkoxy and haloalkyl;
each R₅ is independently:
hydrogen,
halo,
alkyl,
haloalkyl,
alkoxy or
(D)-cycloalkyl;
each R₆ is independently:
hydrogen or
alkyl;
each R₇ is independently:
alkyl,
hydrogen,
(D)-aryl,
(D)-heteroaryl,
(D)-N(R₉)₂,
(D)-NR₉C(O)alkyl or
(D)-NR₉SO₂alkyl;
each R₈ is independently:
hydrogen or
alkyl;
R₉ and R₁₀ are each independently.
hydrogen,
alkyl or
cycloalkyl, or
R₉ and R₁₀ together with the nitrogen to which they are attached form a 5-to 7-membered ring optionally containing an additional heteroatom selected from O, S and NR₆;
each R₁₁ is independently:
alkyl,
OR₁₂,
(D)-aryl,
(D)-cycloalkyl,
(D)-heteroaryl or
halo;
each R₁₂ is independently
hydrogen,
(D)-aryl or
alkyl;
each R₁₃ is independently:
hydrogen or
C₁ - C₄ alkyl;
R₁₄ is independently selected from the group consisting of:
hydrogen,
halo,
alkyl,
(D)-cycloalkyl,
alkoxy or
phenyl;
R₁₅ is independently:
hydrogen,
halo,
alkyl,
(D)-cycloalkyl,
alkoxy or
phenyl;
Cy is selected from aryl, 5- or 6-membered heteroaryl, 5- or 6-membered heterocyclyl or 5- to 7-membered carbocyclyl;
A is a bond or CH₂;
D is a bond or CH₂;
E is NR₈;
Y is NR₉;
n is 0, 1 or 2;
o is 0 or 1;
p is 0 or 1;
s is 0 - 3.

3. The compound of claim 1 or 2, wherein
each R₁ is independently:
cyano,
nitro,
halo,
alkyl,
(D)-heterocyclyl,
(D)-N(R₁₅)₂,
(D)-NR₁₅COR₁₅,
(D)-NR₁₅CON(R₁₅)₂,
(D)-NR₁₅C(O)OR₁₅ or
(D)-NR₁₅SO₂R₁₅;
R₂ is: 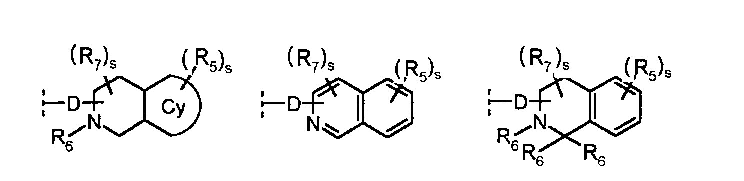 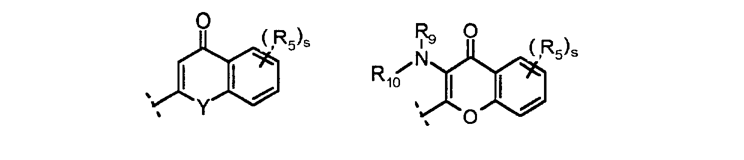
R₃ is (CH₂)-phenyl or (CH₂)-naphthyl, unsubstituted or substituted with one or two substituents selected from the group consisting of perfluoroalkoxy, halo, alkyl, alkoxy and haloalkyl;
each R₅ is independently:
hydrogen,
halo or
alkyl;
R₆ is hydrogen;
R₇ is hydrogen;
R₈ is hydrogen;
R₉ and R₁₀ are each independently:
hydrogen or
alkyl, or
Rg and R₁₀ together with the nitrogen to which they are attached form a 5-to 6-membered ring optionally containing an additional oxygen atom;
each R₁₃ is independently:
hydrogen,
methyl or
ethyl;
R₁₄ is independently:
hydrogen,
halo,
alkyl,
alkoxy or
phenyl;
R₁₅ is independently:
hydrogen,
halo,
alkyl,
alkoxy or
phenyl;
Cy is:
aryl or
heteroaryl;
D is a bond;
Y is N-alkyl;
n is 1 or 2;
s is 0 or 1.

4. The compound of claim 1, 2 or 3 wherein
R₁ is (D)-heterocyclyl;
R₂ is: 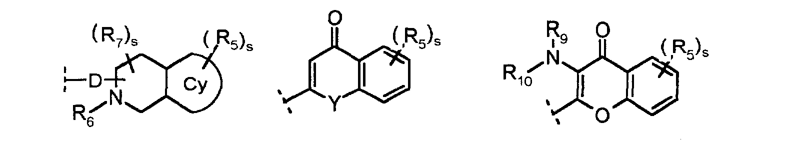
R₃ is (CH₂)-phenyl or (CH₂)-naphthyl, unsubstituted or substituted with one or two halo atoms;
each R₅ is independently:
hydrogen,
halo or
alkyl;
R₆ is hydrogen;
R₇ is hydrogen;
R₉ and R₁₀ are each independently:
hydrogen or
alkyl, or
R₉ and R₁₀ together with the nitrogen to which they are attached form a 5-to 6-membered ring optionally containing an additional oxygen atom;
Cy is benzene;
D is a bond;
Y is NR₉;
n is 1 or 2;
s is 0 or 1.

5. A compound of structural formula (II): 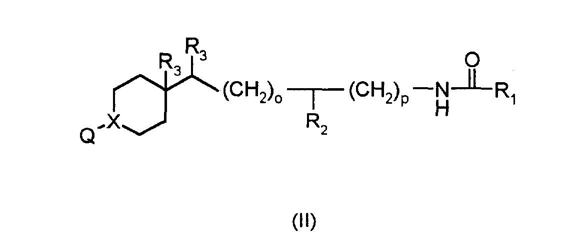 or a pharmaceutically acceptable salt or a solvate thereof, wherein
R₁ is: 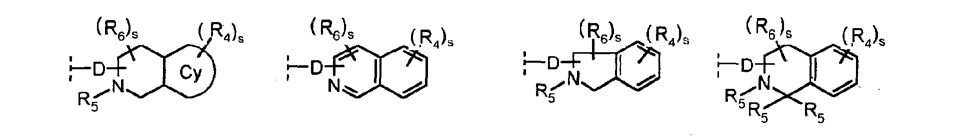 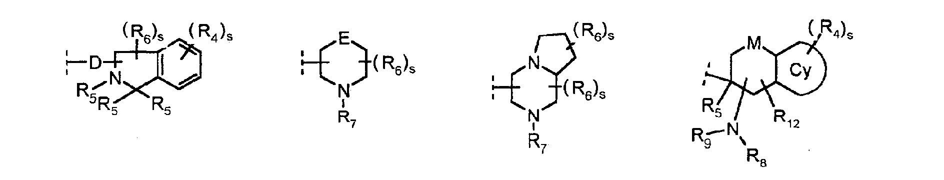 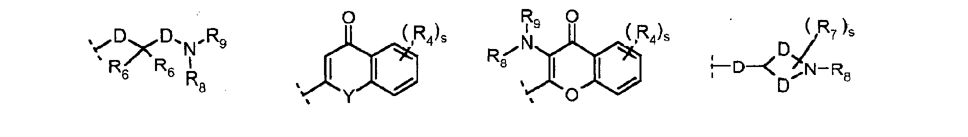
R₂ is independently:
(D)-aryl or
(D)-heteroaryl,
wherein aryl and heteroaryl are unsubstituted or substituted;
R₃ is H or a bond;
each R₄ is independently:
hydrogen,
halo,
alkyl,
haloalkyl,
(D)-cycloalkyl,
(D)-aryl (wherein aryl is phenyl or naphthyl),
(D)-heteroaryl or
(D)-heterocyclyl (wherein heterocyclyl excludes a heterocyclyl containing a single nitrogen), and
wherein aryl, heteroaryl, heterocyclyl, alkyl and cycloalkyl is unsubstituted or substituted;
each R₅ is independently:
hydrogen,
alkyl,
C(O)-alkyl,
(D)-aryl or
cycloalkyl;
each R₆ is independently:
hydrogen,
alkyl,
(D)-aryl,
(D)-heteroaryl,
(D)-N(R₈)₂,
(D)-NR₈C(O)-alkyl,
(D)-NR₈SO₂-alkyl,
(D)-SO₂N(R₈)₂,
(D)-(O)ᵣ-alky),
(D)-(O)ᵣ(D)-NR₈COR₈,
(D)-(O)ᵣ(D)-NR₈SO₂R₈,
(D)-(O)ᵣ-heterocyclyl or
(D)-(O)ᵣ(alkyl)-heterocyclyl; and
each R₇ is independently:
hydrogen,
alkyl,
(D)-aryl,
C(O)-alkyl,
C(O)-aryl,
SO₂-alkyl or
SO₂-aryl;
R₈ and R₉ are each independently:
hydrogen,
alkyl or
cycloalkyl, or
R₈ and R₉ together with the nitrogen to which they are attached form a 5-to 8-membered ring optionally containing an additional heteroatom selected from O, S and NR₅;
wherein alkyl and cycloalkyl are unsubstituted or substituted;
R₁₂ is:
hydrogen or
alkyl;
Cy is:
aryl,
5- or 6-membered heteroaryl,
5- or 6-membered heterocyclyl or
5- or 7-membered carbocyclyl;
Q is:
-C(Rₐ₁)(Rₐ₂)(Rₐ₃),
wherein Rₐ₁ is alkyl, alkenyl, alkynyl, alkoxy, (D)-cycloalkyl, (D)-heterocyclyl, (D)-aryl, 5- to 7- membered benzofused bicyclic ring or (D)-heteroaryl, and
wherein alkyl, alkenyl, alkynyl, (D)-cycloalkyl, (D)-heterocyclyl, (D)-aryl, 5-or 7-membered benzofused bicyclic ring and (D)-heteroaryl are each unsubstituted or substituted;
Rₐ₂ is:
alkyl,
alkenyl,
alkynyl,
(D)-cycloalkyl,
aryl,
(D)-N(R₁₃R₁₃),
(D)-NR₁₃C(O)-alkyl,
(D)-NR₁₃C(O)O-alkyl,
(D)-NR₁₃SO₂(alkyl),
(D)-O-alkyl,
(D)-OC(O)-alkyl or
CON(R₁₃R₁₃),
wherein for the group or subgroup -N(R₁₃R₁₃), each R₁₃ may combine with the other to form a 5-, 6- or 7-membered saturated or unsaturated, unsubstituted or substituted, nitrogen containing heterocycle;
Rₐ₃ is:
hydrogen,
methyl,
ethyl or
propyl;
each R₁₃ is independently:
hydrogen,
oxo,
alkyl,
alkenyl,
(D)-cycloalkyl,
aryl or
heteroaryl,
wherein alkyl, alkenyl, cycloalkyl, aryl and heteroaryl are unsubstituted or substituted;
M is a bond, O, S(O)ᵤ, NR₇ or CH₂;
D is a bond or alkylene;
E is O, S or NR₇;
X is N or CH;
Y is O or NR₈;
o is 0 - 2;
p is 0 - 2;
r is 0 or 1 ;
s is 0 - 5;
u is 0-2.

6. The compound according to claim 5, wherein
R₁ is: 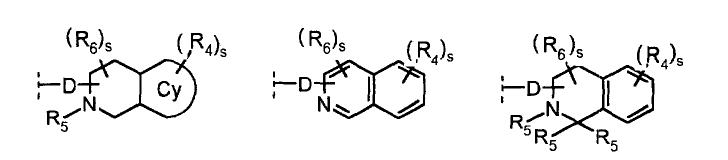 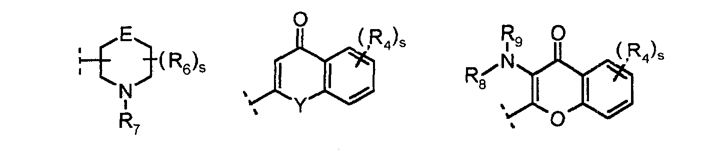
R₂ is unsubstituted or substituted (CH₂)-aryl, wherein (CH₂)-aryl is substituted with one to three substituents taken from the group consisting of cyano, nitro, perfluoroalkoxy, halo, alkyl, (D)-cycloalkyl, alkoxy or haloalkyl;
each R₄ is independently:
hydrogen,
halo,
alkyl,
haloalkyl,
alkoxy or
(D)-cycloalkyl;
each R₅ is independently:
hydrogen or
alkyl;
each R₆ is independently:
hydrogen,
(D)-aryl,
(D)-heteroaryl,
(D)-N(R₈)₂,
(D)-NR₈C(O)alkyl,
(D)-NR₈SO₂alkyl or
alkyl;
each R₇ is independently:
hydrogen or
alkyl;
R₈ and R₉ are each independently:
hydrogen,
alkyl and
cycloalkyl, or
R₈ and R₉ together with the nitrogen to which they are attached form a 5-to 7-membered ring optionally containing an additional heteroatom selected from O, S and NR₅,
wherein alkyl and cycloalkyl are unsubstituted or substituted;
R₁₀ is independently:
alkyl,
OR₁₁,
(D)-aryl,
(D)-cycloalkyl,
(D)-heteroaryl and
halo;
each R₁₁ is independently:
hydrogen,
(D)-aryl and
alkyl;
each R₁₂ is independently:
hydrogen or
C₁ - C₄ alkyl;
Cy is:
aryl,
5- to 6-membered heteroaryl,
5- to 6-membered heterocyclyl or
5- to 7-membered carbocyclyl;
Rₐ₁ is independently:
alkyl,
alkoxy,
(D)-cycloalkyl,
(D)-heteroaryl
(D)-heterocyclyl or
(D)-aryl,
wherein alkyl, cycloalkyl, heterocyclyl and aryl are unsubstituted or substituted;
Rₐ₂ is independently:
alkyl,
aryl,
(D)-cycloalkyl,
(D)-N(R₁₃R₁₃),
(D)-NR₁₃C(O)-alkyl,
(D)-NR₁₃SO₂(alkyl) or
CON(R₁₃R₁₃);
Rₐ₃ is hydrogen;
R₁₃ is independently:
hydrogen,
alkyl,
alkenyl,
(D)-cycloalkyl or
aryl;
M is a bond or CH₂;
D is a bond or CH₂;
E is NR₇;
Y is NR₈;
o is 0 or 1;
p is 0 or 1;
s is 0 - 3.

7. The compound according to claim 5 or 6, wherein
R₁ is: 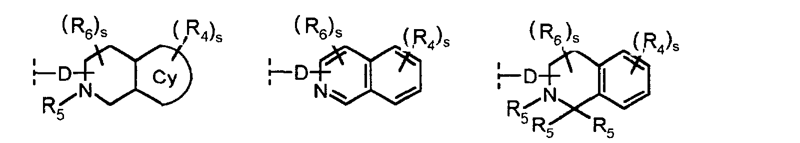 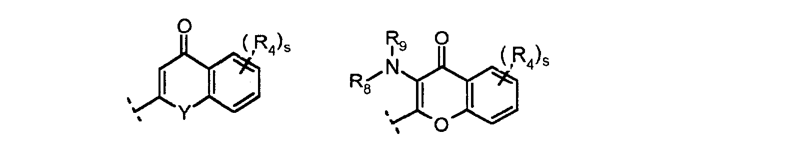
R₂ is (CH₂)-phenyl or (CH₂)-naphthyl, unsubstituted or substituted with one to three substituents selected from the group consisting of perfluoroalkoxy, halo, alkyl, alkoxy and haloalkyl;
each R₄ is independently:
hydrogen,
halo or
alkyl;
R₅ is hydrogen;
R₆ is hydrogen;
R₇ is hydrogen;
R₈ and R₉ are each independently:
hydrogen or
alkyl, or
R₈ and R₉ together with the nitrogen to which they are attached form a 5-to 6-membered ring optionally containing an additional oxygen atom;
each R₁₁ is independently:
hydrogen and
(D)-aryl;
each R₁₂ is independently;
hydrogen,
methyl or
ethyl;
Cy is
aryl or
heteroaryl;
Rₐ₁ is independently:
(D)-cycloalkyl,
(D)-heteroaryl,
(D)-heterocyclyl or
(D)-aryl;
Rₐ₂ is independently:
aryl,
(D)-cycloalkyl,
(D)-N(R₁₃R₁₃),
(D)-NR₁₃C(O)-alkyl,
(D)-NR₁₃SO₂(alkyl) or
CON(R₁₃R₁₃);
R₁₃ is independently:
hydrogen,
alkyl or
(D)-cycloalkyl;
D is a bond;
Y is N-alkyl;
s is 0 or 1.

8. The compound according to claims 5, 6 or 7, wherein
R₁ is: 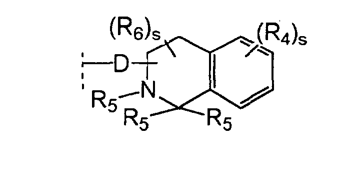
R₂ is (CH₂)-phenyl, unsubstituted or substituted with one to three halo atoms;
R₄ is hydrogen;
R₅ is hydrogen;
R₆ is hydrogen;
Rₐ₁ is independently:
(D)-heteroaryl or
(D)-heterocyclyl;
Rₐ₂ is independently:
aryl,
(D)-NR₁₃SO₂(alkyl) or
CON(R₁₃R₁₃);
R₁₃ is
hydrogen or
alkyl;
D is a bond;
s is 0 or 1.

9. An intermediate compound of structural formula (III) 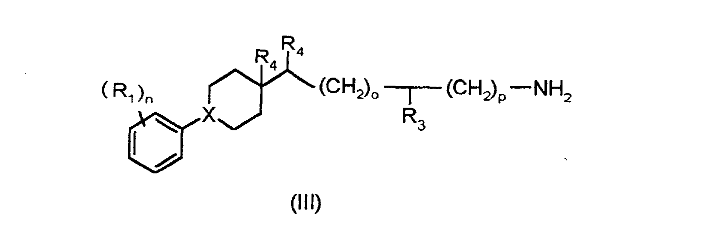 wherein X, R₁, R₃, R₄, n, o and p are as defined in claim 1.

10. An intermediate compound of structural formula (IV) 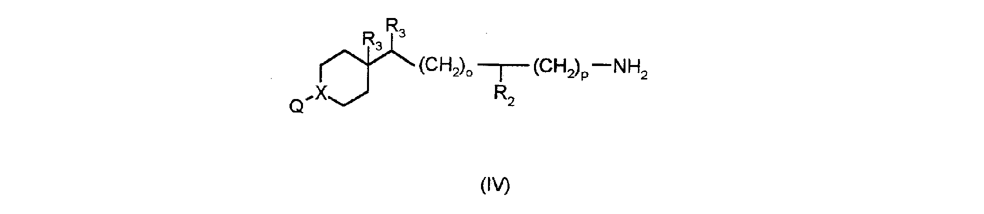 wherein Q, X, R₂, R₃, o and p are as defined in claim 5.

11. The compound of any of claims 1 to 10 for use as a medicament.

12. Use of the compound of any of claims 1 to 10 for the preparation of a medicament for the treatment or prevention of disorders, diseases or conditions responsive to the inactivation or activation of the melanocortin-4 receptor in a mammal.

13. Use according to claim 11 for the preparation of a medicament for the treatment or prevention of cancer cachexia.

14. Use according to claim 11 for the preparation of a medicament for the treatment or prevention of muscle wasting.

15. Use according to claim 11 for the preparation of a medicament for the treatment or prevention of anorexia.

16. Use according to claim 11 for the preparation of a medicament for the treatment or prevention of anxiety and/or depression.

17. Use according to claim 11 for the preparation of a medicament for the treatment or prevention of obesity.

18. Use according to claim 11 for the preparation of a medicament for the treatment or prevention of diabetes mellitus.

19. Use according to claim 11 for the preparation of a medicament for the treatment or prevention of male or female sexual dysfunction.

20. Use according to claim 11 for the preparation of a medicament for the treatment or prevention of erectile dysfunction.

21. A pharmaceutical composition which comprises a compound of any of claims 1 to 10 and a pharmaceutically acceptable carrier.
